# EUROPEAN PATENT APPLICATION

(11) **EP 3 552 608 A1**
(43) Date of publication of application: **16.10.2019**
(21) Application number: 18166400.4
(22) Date of filing: 09.04.2018
(51) Int. Cl.: A61K 31/711, A61K 35/768, A61P 35/00

(54) **INCREASED ACTIVITY OF ONCOLOYTIC NEWCASTLE DISEASE VIRUS**

(71) Applicant: Rapo Yerape B.H. Ltd, 9043300 St. Alon Shvut (IL); Noss, Gerhard Willi Michael, 88142 Wasserburg (DE); Thaller, Arno, 91801 Markt Berolzheim (DE)
(72) Inventor: NOSS, Gerhard Willi Michael, 88142 Wasserburg (DE); DE LEEUW, Olav, 1336 TT Almere (NL); PEETERS, Ben., 8241 AB Lelystad (NL); THALLER, Arno., 91801 Markt Berolzheim (DE)
(74) Representative: Mertzlufft-Paufler, Cornelius

(57) **Abstract**

The invention relates to Newcastle Disease Virus (NDV), an avian paramyxovirus, which has been demonstrated to possess significant oncolytic activity against mammalian cancers. The invention provides the elucidation of the mechanisms of NDV-mediated oncolysis as well as the development of novel oncolytic viruses through the use of genetic engineering. The invention also provides a nucleic acid encoding a reverse genetically engineered (rg-)NDV having a mutation in the HN gene, said mutation allowing replication of said rgNDV in a cancer cell to a higher level than replication of an otherwise identical rgNDV not having said mutation in the HN gene.

SEQUENCE LISTING

<110> Praxisklinik Arno Thaller

<120> Increased activity of oncolytic Newcastle Disease Virus

<130> E 18 0151 K

<160> 20

<170> BiSSAP 1.3.6

<210> 1
<211> 15186
<212> DNA
<213> Newcastle disease virus

<220>
<223> >NDV MutHu final sequence.dna (15,186 bp)

<400> 1
accaaacaga gaatccgtga gctacgataa aaggcgaagg agcaatcgaa gtcgtacggg
60

tagaaggtgt gaatctcgag tgcgagaccg aagcttaaac tcgagagagc cttctgccaa
120

catgtcttcc gtatttgacg agtatgaaca gctcctcgcg gctcagactc gccctaatgg
180

agctcacgga ggaggagaga aggggagcac cttaaaagtt gaagtcccgg tattcactct
240

taacagtgac gacccagaag atagatggaa ctttgcagta ttctgtctta ggattgctgt
300

tagcgaagat gccaacaaac cactcagaca aggtgctctc atatctctct tatgctccca
360

ttctcaagtg atgaggaacc atgtcgccct tgcagggaaa cagaatgagg ccacactggc
420

tgttcttgag atcgatggtt tcaccaacag cgtgcctcaa tttaacaaca ggagtggagt
480

gtctgaggag agagcacaga gattcttgat gatagcaggg tccctccctc gggcatgcag
540

caacggtacc ccgttcatca cagctggggt tgaagatgat gcaccagaag acatcactga
600

tactctggaa agaatcatct ctatccaggc tcaagtatgg gtcacggtag caaaggccat
660

gactgcatat gagacagcgg atgagtcgga aacaagaaga atcaataagt atatgcagca
720

aggcagagtc caaaagaagt acatcctcca ccccgtatgt aggagcgcaa ttcaactcac
780

gattagacaa tctctggcag tccgcatttt cttggttagc gagcttaaga gaggccgcaa
840

cacggcaggt ggaagttcca cttattatag cttggtaggg gacatagact catacatcag
900

gaacaccggg cttactgcat tcttcctgac actcaggtat ggaattaaca ccaagacatc
960

agcccttgca ctcagcagcc tcgcaggcga tatccaaaaa atgaagcagc tcatgcgtct
1020

atatcggatg aaaggagata atgcgccgta catgacactg ctcggtgaca gtgaccagat
1080

gagctttgca ccggctgagt atgcacaact ctactctttt gcaatgggta tggcatccgt
1140

cctagacaaa ggaactggca agtaccagtt cgccagagac tttatgagca catcattctg
1200

gagacttgga gtagagtatg ctcgggctca gggaagcagc atcaatgagg atatggctgc
1260

cgagctaaag ctgaccccag cggcaaggag gggcctggca gctgctgccc aacgagcatc
1320

cgaggagacc ggcagcatgg acattcccac tcaacaagct ggggtcctca cagggctcag
1380

cgacggaggc cctcaagccc cacaaggtgg actgaacagg tcgcaagggc aaccggacgc
1440

cggagatggg gagacccaat tttagatttt gatgagagca gtggcaaata gtatgagaga
1500

agcgccaaac tctgtgcaga acaccactca gcaggagcct ccatctactc ctgggccatc
1560

tcaagacaac gacaccgact ggggatactg accgacaaca ctcagcctgc ctccatcgaa
1620

ttatctcgat tcttctgcct gtaacctaac ccctggtcca cagtctcaca cggccaaacc
1680

cgcaaacgag cccccccctc tctccccccc cctccagcca cacggctcca accgcccaaa
1740

acaacacggg cacaactcga ctcactcata atctacacag agccgaagat cttagaaaaa
1800

aatacgggta gaagagagac attcagagat caggacaaat catcagggtc tccgctctcc
1860

cctctaccca gcagaccaga gtgaagatgg ccaccttcac ggatgcagag atcgacgatc
1920

tatttgaaac cagtggaact gtcattgaca gcataattac ggcccaaggc aacccagtag
1980

agactgttgg aaggagcgca atcccacaag gcaaaactaa agcattgagt gcagcatggg
2040

agaaacatgg aagcacccaa ccgccggccg gccaagacac ccctgatcga atggacagat
2100

cagacaaaca accatcccca cccgaacagg caaccacaca caacagcccg ccagccacat
2160

ccaccgacca gccccccacc caggccgcag gcgaagccgg tgacacacag ctcaagactg
2220

gagcaagcaa ctccctcctg tcgatgctcg acaagcttag caataaatcg tccaatgcta
2280

aaaagggccc acggtcgagt ccccaggaag ggcaccacca acctccgacc cagcagcagg
2340

ggagtcaaca gagccgcgga aacagtcagg agagatcgca aaaccaggtc aaggccgcct
2400

ctggagacca gggcacagac gcgaacacag catatcatgg acaatggaag gagtcacaac
2460

catcagctgg tgcaatccct catgctctcc ggtcagggca gagccaagcc aatactcctg
2520

catctgtgga tcatgtccag ctacctgtcg actttgtgca ggcgatgatg tctatgatgg
2580

aggcgatatc acagaaggta aataaagtcg actatcagct agaccttgtc tcaaagcaga
2640

catcctccat ccccatgatg cgatctgaaa tccaacagct gaaaacatcc gttgcggtca
2700

tggaagccaa cctgggcatg atgaagattc tggaccctgg ttgtgctaac gtttcatctt
2760

taagtgacct acgggcggtc gcccgctctc acccagtttt agtttcaggc cccggagacc
2820

catctcctta tgtgacacaa gggggtgaat tgatactcaa taaactctcg caaccagtgc
2880

aacatccttc tgagttgatt aaacctgcca cgataagcgg gcctgatata ggagtggaga
2940

aggacactgt ccgtgcattg atcacctcac gcccaatgca tccgagctct tcagctaagc
3000

tcctgagtaa gctggatgca gccgggtcga tagaagaaat taggaaaatc aaacgccttg
3060

cgctgaatgg ctgatcacta ctacaacccg caacgggttc ccgtttatct attgtcacaa
3120

ggaacccgcc ccgagccctc ctctataaac ccaagattca aggctccaag cgataacctt
3180

ctcccgcctc ttccatccca ctgaatgatc gcgcagctgc aattaatcta gcaacattaa
3240

agattaagaa aaaatacggg tagaatcgga gtgcaccgat tgtgccaaga tggactcatc
3300

taggacaatt gggttatact ttgattctgc ccttccttct agcaacctgt tagcattccc
3360

gattgtctta cgagatgtag gagatggaaa gaagcaaatt actccgcaat ataggatccg
3420

gcgtcttgac tcgtggacag acagtaaaga agactcggta ttcatcacta cctacggatt
3480

catcttccag gttgggaatg aagaagtcac tgtcggcatg atcaatgata atcccaagcg
3540

agagttactc tccgctgcga tgctctgcct aggaagcgtc ccgaatgttg gagatcctgt
3600

tgagctggca agggcctgcc tcactatggt ggtaacatgc aagaagagtg caactaatac
3660

tgagagaatg gtcttctcag tagtgcaggc accccgggtg ctgcaaagct gtagggtcgt
3720

ggcagacaaa tactcgtcag tgaatgcagt taagcacgtg aaagcaccag agaagatccc
3780

ttggagtgaa accctagagt acaaggtgaa ttttgtctct ttgactgtgg tgccgaagaa
3840

ggatgtctac aagatcccaa ccgcagtatt gaaggtatcc ggctcgagcc tgtataatct
3900

tgcgctcaat gtcactattg atgtggaggt agacccaaag agcccgttag tcaagtccct
3960

ttctaggtcc gacagtggat actatgctaa tctcttctta catatcggac ttatgtccac
4020

tgtagataag aggggaaaga aagtgacatt tgaccagctg gagaggaaga taaggagact
4080

tgatctatct gtcgggctca gtgatgtgct cggaccttct gtgcttgtga aggcgagagg
4140

tgcacggact aggctgctgg cacctttctt ctctaacagc gggacagcct gctaccctat
4200

agcaaatgcc tctcctcagg tagctaagat actctggagt cagaccgcgt gcctgcggag
4260

tgtaaaaatc attattcaag cgggcaccca acgcgctgtc gcagtgactg ctgaccatga
4320

ggttacctct actaagatag agaagaggca taccatcgct aaatacaatc ccttcaagaa
4380

ataggccgta tccctgagac tgcaattcac ccgccttccc aaaacaccat gacaccagat
4440

aatgatctgt cttgattact tacagttagt ttccctgtct atcaaattag aaaaaacacg
4500

ggtagaagag ttcggatccc ggccggcgca ccaaaagcgc aagatgggcc ccagatcttc
4560

taccaggatc ccagtacctc taatgctgac catacggatc acgctggcac tgagttatgt
4620

ccgtctgaca agttctcttg atggcaggcc tcttgcagcc gcagggatcg tggtaacagg
4680

ggataaagca gttaacatat acacctcatc ccagacaggg tcaatcatag tcaagttact
4740

cccaaatatg cccaaggaca aagaggcatg tgcaaaagcc ccattggagg cttacaacag
4800

gacactgact actttgctta ccccccttgg tgattctatc cgcaggatac aagagtctgt
4860

gactacatcc ggaggaagga gacagagacg ctttataggt gccattattg gcagtgtagc
4920

tctaggggtt gcaacagctg cacagataac ggcagcctcg gctctgatac aagccaacca
4980

gaatgctgct aacatcctcc ggcttaagga gagcattgct gcaaccaatg aagctgtaca
5040

cgaggtcact ggcggattgt cacagttagc agtggcagtt ggaaagatgc agcaatttgt
5100

taatgaccag tttaataaca cagctcaaga gttggactgt ataaaaatta cacagcaggt
5160

tggtgtagaa ctcaacttat acttaactga attgactaca gtgttcgggc cacaaatcac
5220

ttcccctgcc ttaactcagt tgactgttca ggctctttac aatctggctg gtggtaatgt
5280

agattacttg ttgactaagt taggtgtagg gaacaaccag ctcagctcat tgattggtag
5340

cggcttgatc accggtaacc ctatttttta cgactcacag actcaactct tgggcataca
5400

ggtgacttta ccctcagtcg ggaacctaaa taatatgcgt gccacctact tggagacctt
5460

gtctgtaagc acaacaaagg gatttgcctc agcacttgtc ccaaaagtag tgacacaggt
5520

cggttctgtg atagaagagc ttgacacctc atactgtata gaagctgatt tggatttata
5580

ttgtacaaga atagtgacat tccctatgtc ccctggtatt tattcctgtt tgagcggcaa
5640

tacatcggct tgcatgtatt caaggactga aggcgcactt actacaccat acatgactct
5700

caaaggctca gttgttgcca attgccagat gacaacatgt agatgtgcag accccccggg
5760

tatcatatca caaaattatg gagaggctgt gtctctaata gataagcact catgcaatgt
5820

cgtatcctta gacgggataa ctttgaggct cagtggggaa tttgatgcaa cttatcaaaa
5880

gaatatctca atattagatt ctcaagtact agtgacaggc aatctcgata tctcaactga
5940

gcttgggaat gtcaaccact caataagtaa tgctttggat aagttagagg aaagcaacag
6000

caaactagac aaagtcaatg tcaaactgac cagcacatct gctctcatta cctatattgt
6060

tttaactgtc atatctcttg ttcttggtat gcttagcctg gttctagcat gctatctgat
6120

gtacaagcaa aaagcgcaac gaaagacctt gttgtggctt ggaaataata ccctagatca
6180

gatgagagct actacaaaaa tgtgaatgca gatgagaggc agaggtatcc ccaatagcaa
6240

tctgtgtgtc aattctggca gcctgttaat cagaagaatt aagaaaaaac taccggatgt
6300

aggtgaacaa aagggaatat acgggtagaa cggcctgaga ggccacccct caatcgggag
6360

ccaggcccca ctacgtccgc tctaccgcat caccaacagc agtcttcagt catggacagc
6420

gcagttagcc aagttgcgct agagaatgat agaagagaag cgaataatac atggcgcttg
6480

gttttccgga tcgcagcctt acttttactg gtaataacct tagccgtctc tgcagtcgcc
6540

ctggcatata gtatggaggc tagcacacct ggcgaccttg taagcatacc gactgcgatc
6600

tctagggcag aggaaaggat tacatctgca ctcggttcca atcaagatgt agtagatagg
6660

atatacaagc aagtggccct tgagtctcca ttggcactgc taaataccga atctataatt
6720

atgaatgcaa taacgtctct ctcttatcaa atcaatggag ccacaaataa tagcgggtgt
6780

ggggcacctg ttcatgaccc agattacatc ggggggatag gtcaagaact tattgtagat
6840

gatacgagtg acgtcacatc attttatccc tctgcattcc aagaacacct gaattttatc
6900

ccggcgccca ctacaggatc aggctgcact cggataccct cattcgacat gagtgctacc
6960

cattactgtt acactcacaa tgtgatattg tctggctgca gagatcactc acactcacat
7020

cagtatttag cacttggtgt gcttcggaca tctgcaacag ggagggtatt cttttctact
7080

ctgcgttcca tcaacttgga tgacgcccaa aatcggaagt cttgcagtgt gagtgcaact
7140

cctttaggtt gtgatatgct gtgctctaaa atcacagaga ctgaggaaga ggattataaa
7200

tcagttatcc ccacatcgat ggtacatgga aggttagggc ttgacggcca ataccatgag
7260

aaggacctag acgtcacaac actatttagg gactgggtgg caaattaccc aggagtagga
7320

ggtgggtctt ttattaacaa tcgcgtatgg ttcccggtct acggagggct aaaacccagc
7380

tcgcctagtg acactgcaca agaagggaga tacgtaatat acaagcgata caatgacaca
7440

tgcccggatg agcaagatta ccagattcgg atggctaagt catcgtataa gcctaggcgg
7500

tttggtggaa aacgcgtaca gcaggccatc ctatccatca aggtgtcaac atccttgggt
7560

gaggacccgg tgttgactgt accgcctaat acggtcgcac tcatgggggc cgaaggcaga
7620

gttctcacag tggggacatc tcatttctta tatcagcgag ggtcatcata cttctctccc
7680

gctttgttat accctatgac agtcaacaac aaaacagcca ctcttcataa tccttataca
7740

ttcaatgctt tcactcggcc aggtagtgtc ccttgccagg cttcagcaag atgccccaac
7800

tcatgtgtta ccggagtcta tactgatcca tatcccttag tcttccatag gaaccacacc
7860

ttgcgagggg tattcggaac aatgcttgat gacgaacaag caagactcaa ccctgtatct
7920

gcagtatttg ataacatatc ccgcagtcgc ataactcggg taagttcaag cagtaccagg
7980

gcagcataca cgacatcaac gtgttttaaa gttgtcaaga ccaataaaac ctattgcctc
8040

agcattgcag aaatatccaa taccctcttc ggggaattca gaattgtccc cttactagtt
8100

gagattctca aggatggtgg ggtttaagaa gctaggaatg gtcggttgag tcaaccgtga
8160

gaaggcgggg aagatgatat tgcatcatct atcttctgta acaccaagaa tcaaatcgaa
8220

taccagtgcg agctcgaatc ctacgctgcc agtcagccat aatcggctgg tgctaatgtg
8280

gttagcctga atcttgtcga tagtcacttg attaagaaaa aatgtggacg gtagtgaaat
8340

ataaggcaaa gcagctcaca gaggacagca cgggtaggac atggcgggct ccggacccga
8400

aagggcagag catcagatta tcctaccaga gtcacatctg tcctcaccat tggtcaagca
8460

taaattgctt tattactgga aattaactgg gctaccgctt cctgacgaat gtgacttcga
8520

ccatcttatt atcagccgac aatggaagaa ggtacttgaa tcggccaccc ctgacattga
8580

gagaatgata aaactagggc gggcagtaca ccagactctc aaccacaatt ccaggataac
8640

cggagtactc catccccggt gtttagagga actggctagt attgaggttc ctgattcaac
8700

caacaaattt cggaagatcg aaaaaaagat ccagattcac aacacaaggt atggagaact
8760

gttcacaaga ttatgcacgc atgtagaaaa gaaactattg gggtcatctt ggtctaacaa
8820

tgtcccacgg tcagaggaat tcaacagcat ccgtacagat ccggcatttt ggtttcactc
8880

aaaatggtcc acagccaaat ttgcatggct ccatataaaa caggtccaga ggcatctgat
8940

tgtagcagca agaacaaggt ccgcagtcaa caaattagtg acgctgaccc ataaggtagg
9000

ccaaatcttt gttactcctg agcttgtcat tgtgacacat acagatgaga acaagttcac
9060

gtgtcttacc caggaacttg tgttgatgta tgcagatatg atggagggca gagatatggt
9120

cagcataata tcatccacgg cggcacatct taggagctta tcagagaaaa ttgatgatat
9180

tctgcggtta gtagatgctc tggcaagaga tttgggcaat caagtctacg atgttgtagc
9240

actaatggag ggattcgcat acggcgctgt tcagctgctt gaaccgtcag gtacatttgc
9300

gggggatttc ttcgcattca acctgcagga gctcaaagat actctaaccg gactcctccc
9360

caaggatatc gcagaatctg tgactcacgc aatcgcaacc atattctctg gcttagaaca
9420

aaatcaagca gctgagatgt tgtgcctgtt gcgtctgtgg ggtcacccac tactcgagtc
9480

ccgtattgca gcaaaagcag ttaggagtca aatgtgcgca ccaaaaatgg tagactttga
9540

tatgatcctc caggtattat ctttctttaa aggaacaatc atcaatggat atagaaagaa
9600

gaatgcaggc gtgtggccac gtgtcaagat agatacgata tacgggaagg tcatagggca
9660

gctacacgca gattctgcag agatttcaca tgatgtcatg ttgagggaat acaagagttt
9720

atctgcactt gaattcgagc catgtataga gtatgaccct gtcaccaatc tgagcatgtt
9780

tttaaaagac aaggcaatcg cacacccgaa agacaactgg ctcgcttcgt ttaggcgaaa
9840

ccttctctct gaggaccaga agaaacatgt aagagaggca acctcaacta accgcctctt
9900

gatagagttc ttagagtcaa atgattttga tccatataaa gagatggaat atctgacgac
9960

ccttgagtac ctaagagatg ataatgtggc agtatcatac tcactcaaag aaaaagaggt
10020

gaaagttaat gggcggattt tcgcaaagct aacaaataga ttaaggaatt gtcaggtaat
10080

ggcagaaggg atcctagctg accagattgc acctttcttc cagggaaatg gggtcattca
10140

ggatagcata tctttgacta aaagtatgct agcgatgagt caactgtctt tcaacagcaa
10200

caagaaacgt attactgact gcaaagaaag agtatcctca aaccgcaatc atgatccgaa
10260

gagcaagaat cgtcggagag ttgccacttt tgtaactacc gacctgcaaa agtattgtct
10320

caattggaga tatcagacag tcaagctgtt cgctcatgcc atcaatcagc tgatgggctt
10380

acctcacttc ttcgagtgga ttcatcttag actaatggat actacgatgt ttgtagggga
10440

ccctttcaat cctccaagtg accctacgga ctgtgatcta tcaagagtcc caaatgatga
10500

catatatatt gtcagtgcta ggggaggcat tgagggatta tgccagaagc tatggacaat
10560

gatctcaatt gctgcaatcc agcttgctgc agcaagatca cattgtcgcg ttgcctgtat
10620

ggtacaaggc gacaatcaag taatagctgt aacgagagag gtaagatcag atgactcccc
10680

agagatggtg ttaacacaat tgcatcaagc cagtgataat ttcttcaagg aattgattca
10740

tgtcaatcat ttgatcggcc ataatttgaa ggatcgtgaa accatcaggt cagacacgtt
10800

cttcatatac agcaaacgaa tattcaaaga tggagcaata ctcagtcagg tcctcaaaaa
10860

ctcatctaaa ttagtgctaa tatcaggcga ccttagtgaa aacactgtaa tgtcttgtgc
10920

caacattgca tctactgtag cacggttatg cgagaacggg cttcctaagg atttctgtta
10980

ttacttaaac tacttaatga gttgcgtgca gacatacttc gattccgaat tttccatcac
11040

caacaactcg caactcgatt ctaaccagtc gtggatagag gacatttctt ttgtgcactc
11100

atatgtcctg acccctgctc aactgggggg actgagtaac cttcaatact caaggctcta
11160

cacaaggaac atcggcgacc cgggaaccac tgctttcgca gagatcaaga gattagaggc
11220

agtggggtta ctgagtccta gcattatgac taacatctta actaggccgc ctggaaatgg
11280

agattgggcc agtctgtgca acgatccata ctcctttaat tttgagactg tcgcaagccc
11340

aaatattgtc cttaagaaac atacacaaag agtcctattt gaaacttgct cgaatccctt
11400

attatctgga gtgcacacag aggataatga ggcagaagag aaggcattgg ctgaattctt
11460

gctcaatcaa gaaatgattc atccacgtgt cgcgcatgct atcatggaag caagctctgt
11520

aggtaggaga aagcaaattc aggggcttgt tgacacaaca aacaccgtga ttaagattgc
11580

actgactagg aggccactcg gcatcaagag gctgatgcgg atagtcaatt actcgagcat
11640

gcatgcaatg ctatttagag atgatgtttt ctcgtctaat agatccaacc atcccttagt
11700

ctcttctgat atgtgttctc tgacactggc agactatgca cggaacagaa gctggtcacc
11760

tttgacaggg ggtagaaaaa tactgggtgt atctaatcct gataccatag aacttgtaga
11820

gggtgagatc cttagtgtca gtggagggtg cactagatgt gacagtgggg atgaacagtt
11880

tacttggttc catcttccaa gcaatataga gctaaacgat gacaccagca agaatcctcc
11940

aatgagagtg ccatatctcg ggtcaaagac tcaagagagg agagccgcct cgcttgcgaa
12000

aatagctcat atgtcaccac acgtgaaggc ggctctaagg gcgtcatctg tgttaatctg
12060

ggcttatggg gacaacgaaa taaactggac tgctgccctt aagattgcaa ggtctcggtg
12120

caacataagc tcagagtatc ttcgactatt gtcacccttg cctacagctg ggaatctcca
12180

acatagattg gacgacggca taactcagat gacattcacc cctgcatctc tatacagggt
12240

gtcaccttac attcatatat ctaatgattc tcaaaggcta tttactgaag aaggagtcaa
12300

agaggggaat gtggtttatc agcaaatcat gctcttgggt ttgtccttaa ttgagtcact
12360

cttcccaatg acaacaacca agacatatga tgaaatcaca ttgcacctcc acagtaaatt
12420

tagctgctgt atcagggaag cacctgttgc agttcctttc gagctactcg ggtggcacc
12480

ggaactaagg gcagtaacct caaataagtt tatgtatgat cctagccctg tatcagaggg
12540

agactttgcg agacttgact tagctatctt taagagttat gaacttaatt tagagtcata
12600

ttccacaata gagctaatga acgttctttc aatatctagt gggaagttga ttggccagtc
12660

tgtggtttct tatgatgaag atacctccat aaagaatgac gctataatag tgtatgacaa
12720

cacacggaat tggatcagcg aagctcagaa ttcagatgtg gtccgcctat tcgagtatgc
12780

ggcactcgaa gtgctcctcg actgttctta tcaactctac tatctgagag taagaggcct
12840

aaacaatatc gtcctgtaca tgagtgattt atacaagaat atgccaggaa ttctactctc
12900

taatattgca gccacaatat ctcaccctgt catccattca aggttgaatg cagtaggtct
12960

ggtcaaccat gacgggtcac accaacttgc agacacagat ttcattgaaa tgtctgcaaa
13020

gctgctagtc ttttgcactc gacgcgtggt ctcaggttta catgcaggga ataagtatga
13080

cctgctgttt ccatctgtct tggatgataa cctaagtgag aagatgcttc agttgatatc
13140

ccaattatgc tgtctgtata cggtgctctt tgctacaaca agagaaatcc cgaaaataag
13200

aggcttatct gcagaagaga aatgttcagt acttactgag tacctactgt cagatgctgt
13260

gaaaccgtta cttgggtccg agcaagtgag ctctatcatg tctcccaaca tagttacgtt
13320

cccagccaat ctgtattaca tgtctaggaa gagccttaat ttgatcaggg agagagagga
13380

cagggatact atcttggcat tgttgttccc tcaagaaccg ctgctcgagt ttcctctggt
13440

acgagatatt ggtgctcgtg taaaagatcc atttacccga caacctgcgg cgtttttaca
13500

agagttagat ttgagtgctc cggcaaggca tgacgcattc acaatcagtc aggcgcattc
13560

tgaacacata ttgccaaacc cagaggaaga tcacttagta cgatacttgt tcagaggaat
13620

agggactgcg tcctcctctt ggtataaggc atcccatctt ctttctgtac ccgaggtcag
13680

atgtgcaagg catgggaact ccttatattt agcagaagga agtggagcca tcatgagtct
13740

tctcgaattg catataccac acgaaactat ctattacaat acacttttct cgaacgagat
13800

gaacccccca cagcgacatt tcggaccaac tccaacacag tttctgaatt cggttgtttt
13860

taggaattta caggcggaag taccatgcaa ggatggattt gtccaggagt tccgtccgtt
13920

atggagagag aatacagaag aaagcgatct gacctcagat aaagcagtgg gatatatcac
13980

atctgtagtg ccgtacaggt ctgtatcatt gctgcattgt gacattgaaa tccctccagg
14040

atctaatcaa agcttactag atcagctggc taccaatctg tctctgattg ccatgcattc
14100

tgtgaaggag ggcggggtcg tgattatcaa agtactgtat gcaatgggat attacttcca
14160

tctactcatg aacttgttta ctccatgttc cacgaaagga tatattctct ctaatggcta
14220

tgcctgtaga ggggatatgg agtgttacct gatatttgtc atgggctacc taggcgggcc
14280

tacatttgta catgaggtgg tgaggatggc aaaaacgcta gtacagcggc atggcacact
14340

tttgtctaag tcagatgaga ttacactgac taggttattt acttcacagc aacagcgtgt
14400

aacagacatc ctatccagcc ctttaccgag actaatgaag tacttgagag agaatattga
14460

tactgcactg attgaagccg ggggacagcc tgtccgtcca ttctgtgcag agagtttagt
14520

gagcacacta acagacatga ctcagacaac ccagatcatc gccagccaca ttgatacagt
14580

cattcgatct gtgatctata tggaagcaga gggtgatctt gctgacacag tattcttatt
14640

taccccttac aacctctcta tggacgggaa aaagagaaca tcacttaaac agtgcacaag
14700

acagatctta gaggtcacaa tactgggtct cagagtcaaa aatctcaata aagtaggtga
14760

tgtaatcagc ctagtactca gaggtatgat ttctctggag gaccttctcc cactgagaac
14820

ctacttgaag tgtagtacct gccctaagta tttgaaggct gtcctaggta ttaccaaact
14880

caaagaaatg ttcacagacg cctctttatt atacttgact cgtgctcaac aaaaattcta
14940

catgaaaact ataggcaatg cagtcaaggg atactatagt aactgtgact cttaaaggca
15000

accacatatc aataggccct ctttctagcc gatcgtattc ttgttgactt cattatacca
15060

tattagaaaa aaattgaatt ccgacccttt aagactcgta ttcggattca aataattatc
15120

tcagaaaaaa agtgcacgta gttgttcttg attatagtcc cgtcattcac caagtctttg
15180

tttggt
15186

<210> 2
<211> 15612
<212> DNA
<213> Newcastle disease virus

<220>
<223> >rgMutHu-Apoptin final sequence.dna (15,612 bp)

<400> 2
accaaacaga gaatccgtga gctacgataa aaggcgaagg agcaatcgaa gtcgtacggg
60

tagaaggtgt gaatctcgag tgcgagaccg aagcttaaac tcgagagagc cttctgccaa
120

catgtcttcc gtatttgacg agtatgaaca gctcctcgcg gctcagactc gccctaatgg
180

agctcacgga ggaggagaga aggggagcac cttaaaagtt gaagtcccgg tattcactct
240

taacagtgac gacccagaag atagatggaa ctttgcagta ttctgtctta ggattgctgt
300

tagcgaagat gccaacaaac cactcagaca aggtgctctc atatctctct tatgctccca
360

ttctcaagtg atgaggaacc atgtcgccct tgcagggaaa cagaatgagg ccacactggc
420

tgttcttgag atcgatggtt tcaccaacag cgtgcctcaa tttaacaaca ggagtggagt
480

gtctgaggag agagcacaga gattcttgat gatagcaggg tccctccctc gggcatgcag
540

caacggtacc ccgttcatca cagctggggt tgaagatgat gcaccagaag acatcactga
600

tactctggaa agaatcatct ctatccaggc tcaagtatgg gtcacggtag caaaggccat
660

gactgcatat gagacagcgg atgagtcgga aacaagaaga atcaataagt atatgcagca
720

aggcagagtc caaaagaagt acatcctcca ccccgtatgt aggagcgcaa ttcaactcac
780

gattagacaa tctctggcag tccgcatttt cttggttagc gagcttaaga gaggccgcaa
840

cacggcaggt ggaagttcca cttattatag cttggtaggg gacatagact catacatcag
900

gaacaccggg cttactgcat tcttcctgac actcaggtat ggaattaaca ccaagacatc
960

agcccttgca ctcagcagcc tcgcaggcga tatccaaaaa atgaagcagc tcatgcgtct
1020

atatcggatg aaaggagata atgcgccgta catgacactg ctcggtgaca gtgaccagat
1080

gagctttgca ccggctgagt atgcacaact ctactctttt gcaatgggta tggcatccgt
1140

cctagacaaa ggaactggca agtaccagtt cgccagagac tttatgagca catcattctg
1200

gagacttgga gtagagtatg ctcgggctca gggaagcagc atcaatgagg atatggctgc
1260

cgagctaaag ctgaccccag cggcaaggag gggcctggca gctgctgccc aacgagcatc
1320

cgaggagacc ggcagcatgg acattcccac tcaacaagct ggggtcctca cagggctcag
1380

cgacggaggc cctcaagccc cacaaggtgg actgaacagg tcgcaagggc aaccggacgc
1440

cggagatggg gagacccaat ttttagattt gatgagagca gtggcaaata gtatgagaga
1500

agcgccaaac tctgtgcaga acaccactca gcaggagcct ccatctactc ctgggccatc
1560

tcaagacaac gacaccgact ggggatactg accgacaaca ctcagcctgc ctccatcgaa
1620

ttatctcgat tcttctgcct gtaacctaac ccctggtcca cagtctcaca cggccaaacc
1680

cgcaaacgag cccccccctc tctccccccc cctccagcca cacggctcca accgcccaaa
1740

acaacacggg cacaactcga ctcactcata atctacacag agccgaagat cttagaaaaa
1800

aatacgggta gaagagagac attcagagat caggacaaat catcagggtc tccgctctcc
1860

cctctaccca gcagaccaga gtgaagatgg ccaccttcac ggatgcagag atcgacgatc
1920

tatttgaaac cagtggaact gtcattgaca gcataattac ggcccaaggc aacccagtag
1980

agactgttgg aaggagcgca atcccacaag gcaaaactaa agcattgagt gcagcatggg
2040

agaaacatgg aagcacccaa ccgccggccg gccaagacac ccctgatcga atggacagat
2100

cagacaaaca accatcccca cccgaacagg caaccacaca caacagcccg ccagccacat
2160

ccaccgacca gccccccacc caggccgcag gcgaagccgg tgacacacag ctcaagactg
2220

gagcaagcaa ctccctcctg tcgatgctcg acaagcttag caataaatcg tccaatgcta
2280

aaaagggccc acggtcgagt ccccaggaag ggcaccacca acctccgacc cagcagcagg
2340

ggagtcaaca gagccgcgga aacagtcagg agagatcgca aaaccaggtc aaggccgcct
2400

ctggagacca gggcacagac gcgaacacag catatcatgg acaatggaag gagtcacaac
2460

catcagctgg tgcaatccct catgctctcc ggtcagggca gagccaagcc aatactcctg
2520

catctgtgga tcatgtccag ctacctgtcg actttgtgca ggcgatgatg tctatgatgg
2580

aggcgatatc acagaaggta aataaagtcg actatcagct agaccttgtc tcaaagcaga
2640

catcctccat ccccatgatg cgatctgaaa tccaacagct gaaaacatcc gttgcggtca
2700

tggaagccaa cctgggcatg atgaagattc tggaccctgg ttgtgctaac gtttcatctt
2760

taagtgacct acgggcggtc gcccgctctc acccagtttt agtttcaggc cccggagacc
2820

catctcctta tgtgacacaa gggggtgaat tgatactcaa taaactctcg caaccagtgc
2880

aacatccttc tgagttgatt aaacctgcca cgataagcgg gcctgatata ggagtggaga
2940

aggacactgt ccgtgcattg atcacctcac gcccaatgca tccgagctct tcagctaagc
3000

tcctgagtaa gctggatgca gccgggtcga tagaagaaat taggaaaatc aaacgccttg
3060

cgctgaatgg ctgatcacta ctacaacccg caacgggttc ccgtttatct attgtcacaa
3120

ggaacccgcc ccgaagctta agaaaaaata cgggtagaag cagtttgaga gctccgttcg
3180

ccaccatgaa cgcactccag gaggacacac cacctggacc ctctaccgtg ttccggccac
3240

ccaccagcag ccggcccctg gagacacccc actgccggga gatcagaatc ggcatcgccg
3300

gcatcacaat caccctgagc ctgtgcggat gtgcaaatgc cagggccccc acactgcgct
3360

ccgccaccgc cgacaactct gagagcaccg gctttaagaa tgtgcctgac ctgcggacag
3420

atcagccaaa gcctccatcc aagaagaggt cttgtgatcc tagcgagtac cgcgtgtccg
3480

agctgaagga gtctctgatc accacaaccc ccagcaggcc tcgcaccgca aaaaggcgta
3540

taagactgta agctgaccga gccctcctct ataaacccaa gattcaaggc tccaagcgat
3600

aaccttctcc cgcctcttcc atcccactga atgatcgcgc agctgcaatt aatctagcaa
3660

cattaaagat taagaaaaaa tacgggtaga atcggagtgc accgattgtg ccaagatgga
3720

ctcatctagg acaattgggt tatactttga ttctgccctt ccttctagca acctgttagc
3780

attcccgatt gtcttacgag atgtaggaga tggaaagaag caaattactc cgcaatatag
3840

gatccggcgt cttgactcgt ggacagacag taaagaagac tcggtattca tcactaccta
3900

cggattcatc ttccaggttg ggaatgaaga agtcactgtc ggcatgatca atgataatcc
3960

caagcgagag ttactctccg ctgcgatgct ctgcctagga agcgtcccga atgttggaga
4020

tcctgttgag ctggcaaggg cctgcctcac tatggtggta acatgcaaga agagtgcaac
4080

taatactgag agaatggtct tctcagtagt gcaggcaccc cgggtgctgc aaagctgtag
4140

ggtcgtggca gacaaatact cgtcagtgaa tgcagttaag cacgtgaaag caccagagaa
4200

gatcccttgg agtgaaaccc tagagtacaa ggtgaatttt gtctctttga ctgtggtgcc
4260

gaagaaggat gtctacaaga tcccaaccgc agtattgaag gtatccggct cgagcctgta
4320

taatcttgcg ctcaatgtca ctattgatgt ggaggtagac ccaaagagcc cgttagtcaa
4380

gtccctttct aggtccgaca gtggatacta tgctaatctc ttcttacata tcggacttat
4440

gtccactgta gataagaggg gaaagaaagt gacatttgac cagctggaga ggaagataag
4500

gagacttgat ctatctgtcg ggctcagtga tgtgctcgga ccttctgtgc ttgtgaaggc
4560

gagaggtgca cggactaggc tgctggcacc tttcttctct aacagcggga cagcctgcta
4620

ccctatagca aatgcctctc ctcaggtagc taagatactc tggagtcaga ccgcgtgcct
4680

gcggagtgta aaaatcatta ttcaagcggg cacccaacgc gctgtcgcag tgactgctga
4740

ccatgaggtt acctctacta agatagagaa gaggcatacc atcgctaaat acaatccctt
4800

caagaaatag gccgtatccc tgagactgca attcacccgc cttcccaaaa caccatgaca
4860

ccagataatg atctgtcttg attacttaca gttagtttcc ctgtctatca aattagaaaa
4920

aacacgggta gaagagttcg gatcccggcc ggcgcaccaa aagcgcaaga tgggccccag
4980

atcttctacc aggatcccag tacctctaat gctgaccata cggatcacgc tggcactgag
5040

ttatgtccgt ctgacaagtt ctcttgatgg caggcctctt gcagccgcag ggatcgtggt
5100

aacaggggat aaagcagtta acatatacac ctcatcccag acagggtcaa tcatagtcaa
5160

gttactccca aatatgccca aggacaaaga ggcatgtgca aaagccccat tggaggctta
5220

caacaggaca ctgactactt tgcttacccc ccttggtgat tctatccgca ggatacaaga
5280

gtctgtgact acatccggag gaaggagaca gagacgcttt ataggtgcca ttattggcag
5340

tgtagctcta ggggttgcaa cagctgcaca gataacggca gcctcggctc tgatacaagc
5400

caaccagaat gctgctaaca tcctccggct taaggagagc attgctgcaa ccaatgaagc
5460

tgtacacgag gtcactggcg gattgtcaca gttagcagtg gcagttggaa agatgcagca
5520

atttgttaat gaccagttta ataacacagc tcaagagttg gactgtataa aaattacaca
5580

gcaggttggt gtagaactca acttatactt aactgaattg actacagtgt tcgggccaca
5640

aatcacttcc cctgccttaa ctcagttgac tgttcaggct ctttacaatc tggctggtgg
5700

taatgtagat tacttgttga ctaagttagg tgtagggaac aaccagctca gctcattgat
5760

tggtagcggc ttgatcaccg gtaaccctat tttttacgac tcacagactc aactcttggg
5820

catacaggtg actttaccct cagtcgggaa cctaaataat atgcgtgcca cctacttgga
5880

gaccttgtct gtaagcacaa caaagggatt tgcctcagca cttgtcccaa aagtagtgac
5940

acaggtcggt tctgtgatag aagagcttga cacctcatac tgtatagaag ctgatttgga
6000

tttatattgt acaagaatag tgacattccc tatgtcccct ggtatttatt cctgtttgag
6060

cggcaataca tcggcttgca tgtattcaag gactgaaggc gcacttacta caccatacat
6120

gactctcaaa ggctcagttg ttgccaattg ccagatgaca acatgtagat gtgcagaccc
6180

cccgggtatc atatcacaaa attatggaga ggctgtgtct ctaatagata agcactcatg
6240

caatgtcgta tccttagacg ggataacttt gaggctcagt ggggaatttg atgcaactta
6300

tcaaaagaat atctcaatat tagattctca agtactagtg acaggcaatc tcgatatctc
6360

aactgagctt gggaatgtca accactcaat aagtaatgct ttggataagt tagaggaaag
6420

caacagcaaa ctagacaaag tcaatgtcaa actgaccagc acatctgctc tcattaccta
6480

tattgtttta actgtcatat ctcttgttct tggtatgctt agcctggttc tagcatgcta
6540

tctgatgtac aagcaaaaag cgcaacgaaa gaccttgttg tggcttggaa ataataccct
6600

agatcagatg agagctacta caaaaatgtg aatgcagatg agaggcagag gtatccccaa
6660

tagcaatctg tgtgtcaatt ctggcagcct gttaatcaga agaattaaga aaaaactacc
6720

ggatgtaggt gaacaaaagg gaatatacgg gtagaacggc ctgagaggcc acccctcaat
6780

cgggagccag gccccactac gtccgctcta ccgcatcacc aacagcagtc ttcagtcatg
6840

gacagcgcag ttagccaagt tgcgctagag aatgatagaa gagaagcgaa taatacatgg
6900

cgcttggttt tccggatcgc agccttactt ttactggtaa taaccttagc cgtctctgca
6960

gtcgccctgg catatagtat ggaggctagc acacctggcg accttgtaag cataccgact
7020

gcgatctcta gggcagagga aaggattaca tctgcactcg gttccaatca agatgtagta
7080

gataggatat acaagcaagt ggcccttgag tctccattgg cactgctaaa taccgaatct
7140

ataattatga atgcaataac gtctctctct tatcaaatca atggagccac aaataatagc
7200

gggtgtgggg cacctgttca tgacccagat tacatcgggg ggataggtca agaacttatt
7260

gtagatgata cgagtgacgt cacatcattt tatccctctg cattccaaga acacctgaat
7320

tttatcccgg cgcccactac aggatcaggc tgcactcgga taccctcatt cgacatgagt
7380

gctacccatt actgttacac tcacaatgtg atattgtctg gctgcagaga tcactcacac
7440

tcacatcagt atttagcact tggtgtgctt cggacatctg caacagggag ggtattcttt
7500

tctactctgc gttccatcaa cttggatgac gcccaaaatc ggaagtcttg cagtgtgagt
7560

gcaactcctt taggttgtga tatgctgtgc tctaaaatca cagagactga ggaagaggat
7620

tataaatcag ttatccccac atcgatggta catggaaggt tagggcttga cggccaatac
7680

catgagaagg acctagacgt cacaacacta tttagggact gggtggcaaa ttacccagga
7740

gtaggaggtg ggtcttttat taacaatcgc gtatggttcc cggtctacgg agggctaaaa
7800

cccagctcgc ctagtgacac tgcacaagaa gggagatacg taatatacaa gcgatacaat
7860

gacacatgcc cggatgagca agattaccag attcggatgg ctaagtcatc gtataagcct
7920

aggcggtttg gtggaaaacg cgtacagcag gccatcctat ccatcaaggt gtcaacatcc
7980

ttgggtgagg acccggtgtt gactgtaccg cctaatacgg tcgcactcat gggggccgaa
8040

ggcagagttc tcacagtggg gacatctcat ttcttatatc agcgagggtc atcatacttc
8100

tctcccgctt tgttataccc tatgacagtc aacaacaaaa cagccactct tcataatcct
8160

tatacattca atgctttcac tcggccaggt agtgtccctt gccaggcttc agcaagatgc
8220

cccaactcat gtgttaccgg agtctatact gatccatatc ccttagtctt ccataggaac
8280

cacaccttgc gaggggtatt cggaacaatg cttgatgacg aacaagcaag actcaaccct
8340

gtatctgcag tatttgataa catatcccgc agtcgcataa ctcgggtaag ttcaagcagt
8400

accagggcag catacacgac atcaacgtgt tttaaagttg tcaagaccaa taaaacctat
8460

tgcctcagca ttgcagaaat atccaatacc ctcttcgggg aattcagaat tgtcccctta
8520

ctagttgaga ttctcaagga tggtggggtt taagaagcta ggaatggtcg gttgagtcaa
8580

ccgtgagaag gcggggaaga tgatattgca tcatctatct tctgtaacac caagaatcaa
8640

atcgaatacc agtgcgagct cgaatcctac gctgccagtc agccataatc ggctggtgct
8700

aatgtggtta gcctgaatct tgtcgatagt cacttgatta agaaaaaatg tggacggtag
8760

tgaaatataa ggcaaagcag ctcacagagg acagcacggg taggacatgg cgggctccgg
8820

acccgaaagg gcagagcatc agattatcct accagagtca catctgtcct caccattggt
8880

caagcataaa ttgctttatt actggaaatt aactgggcta ccgcttcctg acgaatgtga
8940

cttcgaccat cttattatca gccgacaatg gaagaaggta cttgaatcgg ccacccctga
9000

cattgagaga atgataaaac tagggcgggc agtacaccag actctcaacc acaattccag
9060

gataaccgga gtactccatc cccggtgttt agaggaactg gctagtattg aggttcctga
9120

ttcaaccaac aaatttcgga agatcgaaaa aaagatccag attcacaaca caaggtatgg
9180

agaactgttc acaagattat gcacgcatgt agaaaagaaa ctattggggt catcttggtc
9240

taacaatgtc ccacggtcag aggaattcaa cagcatccgt acagatccgg cattttggtt
9300

tcactcaaaa tggtccacag ccaaatttgc atggctccat ataaaacagg tccagaggca
9360

tctgattgta gcagcaagaa caaggtccgc agtcaacaaa ttagtgacgc tgacccataa
9420

ggtaggccaa atctttgtta ctcctgagct tgtcattgtg acacatacag atgagaacaa
9480

gttcacgtgt cttacccagg aacttgtgtt gatgtatgca gatatgatgg agggcagaga
9540

tatggtcagc ataatatcat ccacggcggc acatcttagg agcttatcag agaaaattga
9600

tgatattctg cggttagtag atgctctggc aagagatttg ggcaatcaag tctacgatgt
9660

tgtagcacta atggagggat tcgcatacgg cgctgttcag ctgcttgaac cgtcaggtac
9720

atttgcgggg gatttcttcg cattcaacct gcaggagctc aaagatactc taaccggact
9780

cctccccaag gatatcgcag aatctgtgac tcacgcaatc gcaaccatat tctctggctt
9840

agaacaaaat caagcagctg agatgttgtg cctgttgcgt ctgtggggtc acccactact
9900

cgagtcccgt attgcagcaa aagcagttag gagtcaaatg tgcgcaccaa aaatggtaga
9960

ctttgatatg atcctccagg tattatcttt ctttaaagga acaatcatca atggatatag
10020

aaagaagaat gcaggcgtgt ggccacgtgt caagatagat acgatatacg ggaaggtcat
10080

agggcagcta cacgcagatt ctgcagagat ttcacatgat gtcatgttga gggaatacaa
10140

gagtttatct gcacttgaat tcgagccatg tatagagtat gaccctgtca ccaatctgag
10200

catgttttta aaagacaagg caatcgcaca cccgaaagac aactggctcg cttcgtttag
10260

gcgaaacctt ctctctgagg accagaagaa acatgtaaga gaggcaacct caactaaccg
10320

cctcttgata gagttcttag agtcaaatga ttttgatcca tataaagaga tggaatatct
10380

gacgaccctt gagtacctaa gagatgataa tgtggcagta tcatactcac tcaaagaaaa
10440

agaggtgaaa gttaatgggc ggattttcgc aaagctaaca aatagattaa ggaattgtca
10500

ggtaatggca gaagggatcc tagctgacca gattgcacct ttcttccagg gaaatggggt
10560

cattcaggat agcatatctt tgactaaaag tatgctagcg atgagtcaac tgtctttcaa
10620

cagcaacaag aaacgtatta ctgactgcaa agaaagagta tcctcaaacc gcaatcatga
10680

tccgaagagc aagaatcgtc ggagagttgc cacttttgta actaccgacc tgcaaaagta
10740

ttgtctcaat tggagatatc agacagtcaa gctgttcgct catgccatca atcagctgat
10800

gggcttacct cacttcttcg agtggattca tcttagacta atggatacta cgatgtttgt
10860

aggggaccct ttcaatcctc caagtgaccc tacggactgt gatctatcaa gagtcccaaa
10920

tgatgacata tatattgtca gtgctagggg aggcattgag ggattatgcc agaagctatg
10980

gacaatgatc tcaattgctg caatccagct tgctgcagca agatcacatt gtcgcgttgc
11040

ctgtatggta caaggcgaca atcaagtaat agctgtaacg agagaggtaa gatcagatga
11100

ctccccagag atggtgttaa cacaattgca tcaagccagt gataatttct tcaaggaatt
11160

gattcatgtc aatcatttga tcggccataa tttgaaggat cgtgaaacca tcaggtcaga
11220

cacgttcttc atatacagca aacgaatatt caaagatgga gcaatactca gtcaggtcct
11280

caaaaactca tctaaattag tgctaatatc aggcgacctt agtgaaaaca ctgtaatgtc
11340

ttgtgccaac attgcatcta ctgtagcacg gttatgcgag aacgggcttc ctaaggattt
11400

ctgttattac ttaaactact taatgagttg cgtgcagaca tacttcgatt ccgaattttc
11460

catcaccaac aactcgcaac tcgattctaa ccagtcgtgg atagaggaca tttcttttgt
11520

gcactcatat gtcctgaccc ctgctcaact ggggggactg agtaaccttc aatactcaag
11580

gctctacaca aggaacatcg gcgacccggg aaccactgct ttcgcagaga tcaagagatt
11640

agaggcagtg gggttactga gtcctagcat tatgactaac atcttaacta ggccgcctgg
11700

aaatggagat tgggccagtc tgtgcaacga tccatactcc tttaattttg agactgtcgc
11760

aagcccaaat attgtcctta agaaacatac acaaagagtc ctatttgaaa cttgctcgaa
11820

tcccttatta tctggagtgc acacagagga taatgaggca gaagagaagg cattggctga
11880

attcttgctc aatcaagaaa tgattcatcc acgtgtcgcg catgctatca tggaagcaag
11940

ctctgtaggt aggagaaagc aaattcaggg gcttgttgac acaacaaaca ccgtgattaa
12000

gattgcactg actaggaggc cactcggcat caagaggctg atgcggatag tcaattactc
12060

gagcatgcat gcaatgctat ttagagatga tgttttctcg tctaatagat ccaaccatcc
12120

cttagtctct tctgatatgt gttctctgac actggcagac tatgcacgga acagaagctg
12180

gtcacctttg acagggggta gaaaaatact gggtgtatct aatcctgata ccatagaact
12240

tgtagagggt gagatcctta gtgtcagtgg agggtgcact agatgtgaca gtggggatga
12300

acagtttact tggttccatc ttccaagcaa tatagagcta aacgatgaca ccagcaagaa
12360

tcctccaatg agagtgccat atctcgggtc aaagactcaa gagaggagag ccgcctcgct
12420

tgcgaaaata gctcatatgt caccacacgt gaaggcggct ctaagggcgt catctgtgtt
12480

aatctgggct tatggggaca acgaaataaa ctggactgct gcccttaaga ttgcaaggtc
12540

tcggtgcaac ataagctcag agtatcttcg actattgtca cccttgccta cagctgggaa
12600

tctccaacat agattggacg acggcataac tcagatgaca ttcacccctg catctctata
12660

cagggtgtca ccttacattc atatatctaa tgattctcaa aggctattta ctgaagaagg
12720

agtcaaagag gggaatgtgg tttatcagca aatcatgctc ttgggtttgt ccttaattga
12780

gtcactcttc ccaatgacaa caaccaagac atatgatgaa atcacattgc acctccacag
12840

taaatttagc tgctgtatca gggaagcacc tgttgcagtt cctttcgagc tactcggggt
12900

ggcaccggaa ctaagggcag taacctcaaa taagtttatg tatgatccta gccctgtatc
12960

agagggagac tttgcgagac ttgacttagc tatctttaag agttatgaac ttaatttaga
13020

gtcatattcc acaatagagc taatgaacgt tctttcaata tctagtggga agttgattgg
13080

ccagtctgtg gtttcttatg atgaagatac ctccataaag aatgacgcta taatagtgta
13140

tgacaacaca cggaattgga tcagcgaagc tcagaattca gatgtggtcc gcctattcga
13200

gtatgcggca ctcgaagtgc tcctcgactg ttcttatcaa ctctactatc tgagagtaag
13260

aggcctaaac aatatcgtcc tgtacatgag tgatttatac aagaatatgc caggaattct
13320

actctctaat attgcagcca caatatctca ccctgtcatc cattcaaggt tgaatgcagt
13380

aggtctggtc aaccatgacg ggtcacacca acttgcagac acagatttca ttgaaatgtc
13440

tgcaaagctg ctagtctttt gcactcgacg cgtggtctca ggtttacatg cagggaataa
13500

gtatgacctg ctgtttccat ctgtcttgga tgataaccta agtgagaaga tgcttcagtt
13560

gatatcccaa ttatgctgtc tgtatacggt gctctttgct acaacaagag aaatcccgaa
13620

aataagaggc ttatctgcag aagagaaatg ttcagtactt actgagtacc tactgtcaga
13680

tgctgtgaaa ccgttacttg ggtccgagca agtgagctct atcatgtctc ccaacatagt
13740

tacgttccca gccaatctgt attacatgtc taggaagagc cttaatttga tcagggagag
13800

agaggacagg gatactatct tggcattgtt gttccctcaa gaaccgctgc tcgagtttcc
13860

tctggtacga gatattggtg ctcgtgtaaa agatccattt acccgacaac ctgcggcgtt
13920

tttacaagag ttagatttga gtgctccggc aaggcatgac gcattcacaa tcagtcaggc
13980

gcattctgaa cacatattgc caaacccaga ggaagatcac ttagtacgat acttgttcag
14040

aggaataggg actgcgtcct cctcttggta taaggcatcc catcttcttt ctgtacccga
14100

ggtcagatgt gcaaggcatg ggaactcctt atatttagca gaaggaagtg gagccatcat
14160

gagtcttctc gaattgcata taccacacga aactatctat tacaatacac ttttctcgaa
14220

cgagatgaac cccccacagc gacatttcgg accaactcca acacagtttc tgaattcggt
14280

tgtttttagg aatttacagg cggaagtacc atgcaaggat ggatttgtcc aggagttccg
14340

tccgttatgg agagagaata cagaagaaag cgatctgacc tcagataaag cagtgggata
14400

tatcacatct gtagtgccgt acaggtctgt atcattgctg cattgtgaca ttgaaatccc
14460

tccaggatct aatcaaagct tactagatca gctggctacc aatctgtctc tgattgccat
14520

gcattctgtg aaggagggcg gggtcgtgat tatcaaagta ctgtatgcaa tgggatatta
14580

cttccatcta ctcatgaact tgtttactcc atgttccacg aaaggatata ttctctctaa
14640

tggctatgcc tgtagagggg atatggagtg ttacctgata tttgtcatgg gctacctagg
14700

cgggcctaca tttgtacatg aggtggtgag gatggcaaaa acgctagtac agcggcatgg
14760

cacacttttg tctaagtcag atgagattac actgactagg ttatttactt cacagcaaca
14820

gcgtgtaaca gacatcctat ccagcccttt accgagacta atgaagtact tgagagagaa
14880

tattgatact gcactgattg aagccggggg acagcctgtc cgtccattct gtgcagagag
14940

tttagtgagc acactaacag acatgactca gacaacccag atcatcgcca gccacattga
15000

tacagtcatt cgatctgtga tctatatgga agcagagggt gatcttgctg acacagtatt
15060

cttatttacc ccttacaacc tctctatgga cgggaaaaag agaacatcac ttaaacagtg
15120

cacaagacag atcttagagg tcacaatact gggtctcaga gtcaaaaatc tcaataaagt
15180

aggtgatgta atcagcctag tactcagagg tatgatttct ctggaggacc ttctcccact
15240

gagaacctac ttgaagtgta gtacctgccc taagtatttg aaggctgtcc taggtattac
15300

caaactcaaa gaaatgttca cagacgcctc tttattatac ttgactcgtg ctcaacaaaa
15360

attctacatg aaaactatag gcaatgcagt caagggatac tatagtaact gtgactctta
15420

aaggcaacca catatcaata ggccctcttt ctagccgatc gtattcttgt tgacttcatt
15480

ataccatatt agaaaaaaat tgaattccga ccctttaaga ctcgtattcg gattcaaata
15540

attatctcag aaaaaaagtg cacgtagttg ttcttgatta tagtcccgtc attcaccaag
15600

tctttgtttg gt
15612

<210> 3
<211> 16302
<212> DNA
<213> Newcastle disease virus

<220>
<223> >rgMutHu-B18R final sequence.dna (16,302 bp)

<400> 3
accaaacaga gaatccgtga gctacgataa aaggcgaagg agcaatcgaa gtcgtacggg
60

tagaaggtgt gaatctcgag tgcgagaccg aagcttaaac tcgagagagc cttctgccaa
120

catgtcttcc gtatttgacg agtatgaaca gctcctcgcg gctcagactc gccctaatgg
180

agctcacgga ggaggagaga aggggagcac cttaaaagtt gaagtcccgg tattcactct
240

taacagtgac gacccagaag atagatggaa ctttgcagta ttctgtctta ggattgctgt
300

tagcgaagat gccaacaaac cactcagaca aggtgctctc atatctctct tatgctccca
360

ttctcaagtg atgaggaacc atgtcgccct tgcagggaaa cagaatgagg ccacactggc
420

tgttcttgag atcgatggtt tcaccaacag cgtgcctcaa tttaacaaca ggagtggagt
480

gtctgaggag agagcacaga gattcttgat gatagcaggg tccctccctc gggcatgcag
540

caacggtacc ccgttcatca cagctggggt tgaagatgat gcaccagaag acatcactga
600

tactctggaa agaatcatct ctatccaggc tcaagtatgg gtcacggtag caaaggccat
660

gactgcatat gagacagcgg atgagtcgga aacaagaaga atcaataagt atatgcagca
720

aggcagagtc caaaagaagt acatcctcca ccccgtatgt aggagcgcaa ttcaactcac
780

gattagacaa tctctggcag tccgcatttt cttggttagc gagcttaaga gaggccgcaa
840

cacggcaggt ggaagttcca cttattatag cttggtaggg gacatagact catacatcag
900

gaacaccggg cttactgcat tcttcctgac actcaggtat ggaattaaca ccaagacatc
960

agcccttgca ctcagcagcc tcgcaggcga tatccaaaaa atgaagcagc tcatgcgtct
1020

atatcggatg aaaggagata atgcgccgta catgacactg ctcggtgaca gtgaccagat
1080

gagctttgca ccggctgagt atgcacaact ctactctttt gcaatgggta tggcatccgt
1140

cctagacaaa ggaactggca agtaccagtt cgccagagac tttatgagca catcattctg
1200

gagacttgga gtagagtatg ctcgggctca gggaagcagc atcaatgagg atatggctgc
1260

cgagctaaag ctgaccccag cggcaaggag gggcctggca gctgctgccc aacgagcatc
1320

cgaggagacc ggcagcatgg acattcccac tcaacaagct ggggtcctca caggctcag
1380

cgacggaggc cctcaagccc cacaaggtgg actgaacagg tcgcaagggc aaccggacgc
1440

cggagatggg gagacccaat ttttagattt gatgagagca gtggcaaata gtatgagaga
1500

agcgccaaac tctgtgcaga acaccactca gcaggagcct ccatctactc ctgggccatc
1560

tcaagacaac gacaccgact ggggatactg accgacaaca ctcagcctgc ctccatcgaa
1620

ttatctcgat tcttctgcct gtaacctaac ccctggtcca cagtctcaca cggccaaacc
1680

cgcaaacgag cccccccctc tctccccccc cctccagcca cacggctcca accgcccaaa
1740

acaacacggg cacaactcga ctcactcata atctacacag agccgaagat cttagaaaaa
1800

aatacgggta gaagagagac attcagagat caggacaaat catcagggtc tccgctctcc
1860

cctctaccca gcagaccaga gtgaagatgg ccaccttcac ggatgcagag atcgacgatc
1920

tatttgaaac cagtggaact gtcattgaca gcataattac ggcccaaggc aacccagtag
1980

agactgttgg aaggagcgca atcccacaag gcaaaactaa agcattgagt gcagcatggg
2040

agaaacatgg aagcacccaa ccgccggccg gccaagacac ccctgatcga atggacagat
2100

cagacaaaca accatcccca cccgaacagg caaccacaca caacagcccg ccagccacat
2160

ccaccgacca gccccccacc caggccgcag gcgaagccgg tgacacacag ctcaagactg
2220

gagcaagcaa ctccctcctg tcgatgctcg acaagcttag caataaatcg tccaatgcta
2280

aaaagggccc acggtcgagt ccccaggaag ggcaccacca acctccgacc cagcagcagg
2340

ggagtcaaca gagccgcgga aacagtcagg agagatcgca aaaccaggtc aaggccgcct
2400

ctggagacca gggcacagac gcgaacacag catatcatgg acaatggaag gagtcacaac
2460

catcagctgg tgcaatccct catgctctcc ggtcagggca gagccaagcc aatactcctg
2520

catctgtgga tcatgtccag ctacctgtcg actttgtgca ggcgatgatg tctatgatgg
2580

aggcgatatc acagaaggta aataaagtcg actatcagct agaccttgtc tcaaagcaga
2640

catcctccat ccccatgatg cgatctgaaa tccaacagct gaaaacatcc gttgcggtca
2700

tggaagccaa cctgggcatg atgaagattc tggaccctgg ttgtgctaac gtttcatctt
2760

taagtgacct acgggcggtc gcccgctctc acccagtttt agtttcaggc cccggagacc
2820

catctcctta tgtgacacaa gggggtgaat tgatactcaa taaactctcg caaccagtgc
2880

aacatccttc tgagttgatt aaacctgcca cgataagcgg gcctgatata ggagtggaga
2940

aggacactgt ccgtgcattg atcacctcac gcccaatgca tccgagctct tcagctaagc
3000

tcctgagtaa gctggatgca gccgggtcga tagaagaaat taggaaaatc aaacgccttg
3060

cgctgaatgg ctgatcacta ctacaacccg caacgggttc ccgtttatct attgtcacaa
3120

ggaacccgcc ccgaagctta agaaaaaata cgggtagaat acaattaacg atctttatag
3180

ccaccatgac gatgaaaatg atggtacata tatatttcgt atcattattg ttattgctat
3240

tccacagtta cgccatagac atcgaaaatg aaatcacaga attcttcaat aaaatgagag
3300

atactctacc agctaaagac tctaaatggt tgaatccagc atgtatgttc ggaggcacaa
3360

tgaatgatat agccgctcta ggagagccat tcagcgcaaa gtgtcctcct attgaagaca
3420

gtcttttatc gcacagatat aaagactatg tggttaaatg ggaaaggcta gaaaaaaata
3480

gacggcgaca ggtttctaat aaacgtgtta aacatggtga tttatggata gccaactata
3540

catctaaatt cagtaaccgt aggtatttgt gcaccgtaac tacaaagaat ggtgactgtg
3600

ttcagggtat agttagatct catattagaa aacctccttc atgcattcca aaaacatatg
3660

aactaggtac tcatgataag tatggcatag acttatactg tggaattctt tacgcaaaac
3720

attataataa tataacttgg tataaagata ataaggaaat taatatcgac gacattaagt
3780

attcacaaac gggaaaggaa ttaattattc ataatccaga gttagaagat agcggaagat
3840

acgactgtta cgttcattac gacgacgtta gaatcaagaa tgatatcgta gtatcaagat
3900

gtaaaatact tacggttata ccgtcacaag accacaggtt taaactaata ctagatccaa
3960

aaatcaacgt aacgatagga gaacctgcca atataacatg cactgctgtg tcaacgtcat
4020

tattgattga cgatgtactg attgaatggg aaaatccatc cggatggctt ataggattcg
4080

attttgatgt atactctgtt ttaactagta gaggcggtat taccgaggcg accttgtact
4140

ttgaaaatgt tactgaagaa tatataggta atacatataa atgtcgtgga cacaactatt
4200

attttgaaaa aacccttaca actacagtag tattggagta agctgaccga gccctcctct
4260

ataaacccaa gattcaaggc tccaagcgat aaccttctcc cgcctcttcc atcccactga
4320

atgatcgcgc agctgcaatt aatctagcaa cattaaagat taagaaaaaa tacgggtaga
4380

atcggagtgc accgattgtg ccaagatgga ctcatctagg acaattgggt tatactttga
4440

ttctgccctt ccttctagca acctgttagc attcccgatt gtcttacgag atgtaggaga
4500

tggaaagaag caaattactc cgcaatatag gatccggcgt cttgactcgt ggacagacag
4560

taaagaagac tcggtattca tcactaccta cggattcatc ttccaggttg ggaatgaaga
4620

agtcactgtc ggcatgatca atgataatcc caagcgagag ttactctccg ctgcgatgct
4680

ctgcctagga agcgtcccga atgttggaga tcctgttgag ctggcaaggg cctgcctcac
4740

tatggtggta acatgcaaga agagtgcaac taatactgag agaatggtct tctcagtagt
4800

gcaggcaccc cgggtgctgc aaagctgtag ggtcgtggca gacaaatact cgtcagtgaa
4860

tgcagttaag cacgtgaaag caccagagaa gatcccttgg agtgaaaccc tagagtacaa
4920

ggtgaatttt gtctctttga ctgtggtgcc gaagaaggat gtctacaaga tcccaaccgc
4980

agtattgaag gtatccggct cgagcctgta taatcttgcg ctcaatgtca ctattgatgt
5040

ggaggtagac ccaaagagcc cgttagtcaa gtccctttct aggtccgaca gtggatacta
5100

tgctaatctc ttcttacata tcggacttat gtccactgta gataagaggg gaaagaaagt
5160

gacatttgac cagctggaga ggaagataag gagacttgat ctatctgtcg ggctcagtga
5220

tgtgctcgga ccttctgtgc ttgtgaaggc gagaggtgca cggactaggc tgctggcacc
5280

tttcttctct aacagcggga cagcctgcta ccctatagca aatgcctctc ctcaggtagc
5340

taagatactc tggagtcaga ccgcgtgcct gcggagtgta aaaatcatta ttcaagcggg
5400

cacccaacgc gctgtcgcag tgactgctga ccatgaggtt acctctacta agatagagaa
5460

gaggcatacc atcgctaaat acaatccctt caagaaatag gccgtatccc tgagactgca
5520

attcacccgc cttcccaaaa caccatgaca ccagataatg atctgtcttg attacttaca
5580

gttagtttcc ctgtctatca aattagaaaa aacacgggta gaagagttcg gatcccggcc
5640

ggcgcaccaa aagcgcaaga tgggccccag atcttctacc aggatcccag tacctctaat
5700

gctgaccata cggatcacgc tggcactgag ttatgtccgt ctgacaagtt ctcttgatgg
5760

caggcctctt gcagccgcag ggatcgtggt aacaggggat aaagcagtta acatatacac
5820

ctcatcccag acagggtcaa tcatagtcaa gttactccca aatatgccca aggacaaaga
5880

ggcatgtgca aaagccccat tggaggctta caacaggaca ctgactactt tgcttacccc
5940

ccttggtgat tctatccgca ggatacaaga gtctgtgact acatccggag gaaggagaca
6000

gagacgcttt ataggtgcca ttattggcag tgtagctcta ggggttgcaa cagctgcaca
6060

gataacggca gcctcggctc tgatacaagc caaccagaat gctgctaaca tcctccggct
6120

taaggagagc attgctgcaa ccaatgaagc tgtacacgag gtcactggcg gattgtcaca
6180

gttagcagtg gcagttggaa agatgcagca atttgttaat gaccagttta ataacacagc
6240

tcaagagttg gactgtataa aaattacaca gcaggttggt gtagaactca acttatactt
6300

aactgaattg actacagtgt tcgggccaca aatcacttcc cctgccttaa ctcagttgac
6360

tgttcaggct ctttacaatc tggctggtgg taatgtagat tacttgttga ctaagttagg
6420

tgtagggaac aaccagctca gctcattgat tggtagcggc ttgatcaccg gtaaccctat
6480

tttttacgac tcacagactc aactcttggg catacaggtg actttaccct cagtcgggaa
6540

cctaaataat atgcgtgcca cctacttgga gaccttgtct gtaagcacaa caaaggatt
6600

tgcctcagca cttgtcccaa aagtagtgac acaggtcggt tctgtgatag aagagcttga
6660

cacctcatac tgtatagaag ctgatttgga tttatattgt acaagaatag tgacattccc
6720

tatgtcccct ggtatttatt cctgtttgag cggcaataca tcggcttgca tgtattcaag
6780

gactgaaggc gcacttacta caccatacat gactctcaaa ggctcagttg ttgccaattg
6840

ccagatgaca acatgtagat gtgcagaccc cccgggtatc atatcacaaa attatggaga
6900

ggctgtgtct ctaatagata agcactcatg caatgtcgta tccttagacg ggataacttt
6960

gaggctcagt ggggaatttg atgcaactta tcaaaagaat atctcaatat tagattctca
7020

agtactagtg acaggcaatc tcgatatctc aactgagctt gggaatgtca accactcaat
7080

aagtaatgct ttggataagt tagaggaaag caacagcaaa ctagacaaag tcaatgtcaa
7140

actgaccagc acatctgctc tcattaccta tattgtttta actgtcatat ctcttgttct
7200

tggtatgctt agcctggttc tagcatgcta tctgatgtac aagcaaaaag cgcaacgaaa
7260

gaccttgttg tggcttggaa ataataccct agatcagatg agagctacta caaaaatgtg
7320

aatgcagatg agaggcagag gtatccccaa tagcaatctg tgtgtcaatt ctggcagcct
7380

gttaatcaga agaattaaga aaaaactacc ggatgtaggt gaacaaaagg gaatatacgg
7440

gtagaacggc ctgagaggcc acccctcaat cgggagccag gccccactac gtccgctcta
7500

ccgcatcacc aacagcagtc ttcagtcatg gacagcgcag ttagccaagt tgcgctagag
7560

aatgatagaa gagaagcgaa taatacatgg cgcttggttt tccggatcgc agccttactt
7620

ttactggtaa taaccttagc cgtctctgca gtcgccctgg catatagtat ggaggctagc
7680

acacctggcg accttgtaag cataccgact gcgatctcta gggcagagga aaggattaca
7740

tctgcactcg gttccaatca agatgtagta gataggatat acaagcaagt ggcccttgag
7800

tctccattgg cactgctaaa taccgaatct ataattatga atgcaataac gtctctctct
7860

tatcaaatca atggagccac aaataatagc gggtgtgggg cacctgttca tgacccagat
7920

tacatcgggg ggataggtca agaacttatt gtagatgata cgagtgacgt cacatcattt
7980

tatccctctg cattccaaga acacctgaat tttatcccgg cgcccactac aggatcaggc
8040

tgcactcgga taccctcatt cgacatgagt gctacccatt actgttacac tcacaatgtg
8100

atattgtctg gctgcagaga tcactcacac tcacatcagt atttagcact tggtgtgctt
8160

cggacatctg caacagggag ggtattcttt tctactctgc gttccatcaa cttggatgac
8220

gcccaaaatc ggaagtcttg cagtgtgagt gcaactcctt taggttgtga tatgctgtgc
8280

tctaaaatca cagagactga ggaagaggat tataaatcag ttatccccac atcgatggta
8340

catggaaggt tagggcttga cggccaatac catgagaagg acctagacgt cacaacacta
8400

tttagggact gggtggcaaa ttacccagga gtaggaggtg ggtcttttat taacaatcgc
8460

gtatggttcc cggtctacgg agggctaaaa cccagctcgc ctagtgacac tgcacaagaa
8520

gggagatacg taatatacaa gcgatacaat gacacatgcc cggatgagca agattaccag
8580

attcggatgg ctaagtcatc gtataagcct aggcggtttg gtggaaaacg cgtacagcag
8640

gccatcctat ccatcaaggt gtcaacatcc ttgggtgagg acccggtgtt gactgtaccg
8700

cctaatacgg tcgcactcat gggggccgaa ggcagagttc tcacagtggg gacatctcat
8760

ttcttatatc agcgagggtc atcatacttc tctcccgctt tgttataccc tatgacagtc
8820

aacaacaaaa cagccactct tcataatcct tatacattca atgctttcac tcggccaggt
8880

agtgtccctt gccaggcttc agcaagatgc cccaactcat gtgttaccgg agtctatact
8940

gatccatatc ccttagtctt ccataggaac cacaccttgc gaggggtatt cggaacaatg
9000

cttgatgacg aacaagcaag actcaaccct gtatctgcag tatttgataa catatcccgc
9060

agtcgcataa ctcgggtaag ttcaagcagt accagggcag catacacgac atcaacgtgt
9120

tttaaagttg tcaagaccaa taaaacctat tgcctcagca ttgcagaaat atccaatacc
9180

ctcttcgggg aattcagaat tgtcccctta ctagttgaga ttctcaagga tggtggggtt
9240

taagaagcta ggaatggtcg gttgagtcaa ccgtgagaag gcggggaaga tgatattgca
9300

tcatctatct tctgtaacac caagaatcaa atcgaatacc agtgcgagct cgaatcctac
9360

gctgccagtc agccataatc ggctggtgct aatgtggtta gcctgaatct tgtcgatagt
9420

cacttgatta agaaaaaatg tggacggtag tgaaatataa ggcaaagcag ctcacagagg
9480

acagcacggg taggacatgg cgggctccgg acccgaaagg gcagagcatc agattatcct
9540

accagagtca catctgtcct caccattggt caagcataaa ttgctttatt actggaaatt
9600

aactgggcta ccgcttcctg acgaatgtga cttcgaccat cttattatca gccgacaatg
9660

gaagaaggta cttgaatcgg ccacccctga cattgagaga atgataaaac tagggcgggc
9720

agtacaccag actctcaacc acaattccag gataaccgga gtactccatc cccggtgttt
9780

agaggaactg gctagtattg aggttcctga ttcaaccaac aaatttcgga agatcgaaaa
9840

aaagatccag attcacaaca caaggtatgg agaactgttc acaagattat gcacgcatgt
9900

agaaaagaaa ctattggggt catcttggtc taacaatgtc ccacggtcag aggaattcaa
9960

cagcatccgt acagatccgg cattttggtt tcactcaaaa tggtccacag ccaaatttgc
10020

atggctccat ataaaacagg tccagaggca tctgattgta gcagcaagaa caaggtccgc
10080

agtcaacaaa ttagtgacgc tgacccataa ggtaggccaa atctttgtta ctcctgagct
10140

tgtcattgtg acacatacag atgagaacaa gttcacgtgt cttacccagg aacttgtgtt
10200

gatgtatgca gatatgatgg agggcagaga tatggtcagc ataatatcat ccacggcggc
10260

acatcttagg agcttatcag agaaaattga tgatattctg cggttagtag atgctctggc
10320

aagagatttg ggcaatcaag tctacgatgt tgtagcacta atggagggat tcgcatacgg
10380

cgctgttcag ctgcttgaac cgtcaggtac atttgcgggg gatttcttcg cattcaacct
10440

gcaggagctc aaagatactc taaccggact cctccccaag gatatcgcag aatctgtgac
10500

tcacgcaatc gcaaccatat tctctggctt agaacaaaat caagcagctg agatgttgtg
10560

cctgttgcgt ctgtggggtc acccactact cgagtcccgt attgcagcaa aagcagttag
10620

gagtcaaatg tgcgcaccaa aaatggtaga ctttgatatg atcctccagg tattatcttt
10680

ctttaaagga acaatcatca atggatatag aaagaagaat gcaggcgtgt ggccacgtgt
10740

caagatagat acgatatacg ggaaggtcat agggcagcta cacgcagatt ctgcagagat
10800

ttcacatgat gtcatgttga gggaatacaa gagtttatct gcacttgaat tcgagccatg
10860

tatagagtat gaccctgtca ccaatctgag catgttttta aaagacaagg caatcgcaca
10920

cccgaaagac aactggctcg cttcgtttag gcgaaacctt ctctctgagg accagaagaa
10980

acatgtaaga gaggcaacct caactaaccg cctcttgata gagttcttag agtcaaatga
11040

ttttgatcca tataaagaga tggaatatct gacgaccctt gagtacctaa gagatgataa
11100

tgtggcagta tcatactcac tcaaagaaaa agaggtgaaa gttaatgggc ggattttcgc
11160

aaagctaaca aatagattaa ggaattgtca ggtaatggca gaagggatcc tagctgacca
11220

gattgcacct ttcttccagg gaaatggggt cattcaggat agcatatctt tgactaaaag
11280

tatgctagcg atgagtcaac tgtctttcaa cagcaacaag aaacgtatta ctgactgcaa
11340

agaaagagta tcctcaaacc gcaatcatga tccgaagagc aagaatcgtc ggagagttgc
11400

cacttttgta actaccgacc tgcaaaagta ttgtctcaat tggagatatc agacagtcaa
11460

gctgttcgct catgccatca atcagctgat gggcttacct cacttcttcg agtggattca
11520

tcttagacta atggatacta cgatgtttgt aggggaccct ttcaatcctc caagtgaccc
11580

tacggactgt gatctatcaa gagtcccaaa tgatgacata tatattgtca gtgctagggg
11640

aggcattgag ggattatgcc agaagctatg gacaatgatc tcaattgctg caatccagct
11700

tgctgcagca agatcacatt gtcgcgttgc ctgtatggta caaggcgaca atcaagtaat
11760

agctgtaacg agagaggtaa gatcagatga ctccccagag atggtgttaa cacaattgca
11820

tcaagccagt gataatttct tcaaggaatt gattcatgtc aatcatttga tcggccataa
11880

tttgaaggat cgtgaaacca tcaggtcaga cacgttcttc atatacagca aacgaatatt
11940

caaagatgga gcaatactca gtcaggtcct caaaaactca tctaaattag tgctaatatc
12000

aggcgacctt agtgaaaaca ctgtaatgtc ttgtgccaac attgcatcta ctgtagcacg
12060

gttatgcgag aacgggcttc ctaaggattt ctgttattac ttaaactact taatgagttg
12120

cgtgcagaca tacttcgatt ccgaattttc catcaccaac aactcgcaac tcgattctaa
12180

ccagtcgtgg atagaggaca tttcttttgt gcactcatat gtcctgaccc ctgctcaact
12240

ggggggactg agtaaccttc aatactcaag gctctacaca aggaacatcg gcgacccggg
12300

aaccactgct ttcgcagaga tcaagagatt agaggcagtg gggttactga gtcctagcat
12360

tatgactaac atcttaacta ggccgcctgg aaatggagat tgggccagtc tgtgcaacga
12420

tccatactcc tttaattttg agactgtcgc aagcccaaat attgtcctta agaaacatac
12480

acaaagagtc ctatttgaaa cttgctcgaa tcccttatta tctggagtgc acacagagga
12540

taatgaggca gaagagaagg cattggctga attcttgctc aatcaagaaa tgattcatcc
12600

acgtgtcgcg catgctatca tggaagcaag ctctgtaggt aggagaaagc aaattcaggg
12660

gcttgttgac acaacaaaca ccgtgattaa gattgcactg actaggaggc cactcggcat
12720

caagaggctg atgcggatag tcaattactc gagcatgcat gcaatgctat ttagagatga
12780

tgttttctcg tctaatagat ccaaccatcc cttagtctct tctgatatgt gttctctgac
12840

actggcagac tatgcacgga acagaagctg gtcacctttg acagggggta gaaaaatact
12900

gggtgtatct aatcctgata ccatagaact tgtagagggt gagatcctta gtgtcagtgg
12960

agggtgcact agatgtgaca gtggggatga acagtttact tggttccatc ttccaagcaa
13020

tatagagcta aacgatgaca ccagcaagaa tcctccaatg agagtgccat atctcgggtc
13080

aaagactcaa gagaggagag ccgcctcgct tgcgaaaata gctcatatgt caccacacgt
13140

gaaggcggct ctaagggcgt catctgtgtt aatctgggct tatggggaca acgaaataaa
13200

ctggactgct gcccttaaga ttgcaaggtc tcggtgcaac ataagctcag agtatcttcg
13260

actattgtca cccttgccta cagctgggaa tctccaacat agattggacg acggcataac
13320

tcagatgaca ttcacccctg catctctata cagggtgtca ccttacattc atatatctaa
13380

tgattctcaa aggctattta ctgaagaagg agtcaaagag gggaatgtgg tttatcagca
13440

aatcatgctc ttgggtttgt ccttaattga gtcactcttc ccaatgacaa caaccaagac
13500

atatgatgaa atcacattgc acctccacag taaatttagc tgctgtatca gggaagcacc
13560

tgttgcagtt cctttcgagc tactcggggt ggcaccggaa ctaagggcag taacctcaaa
13620

taagtttatg tatgatccta gccctgtatc agagggagac tttgcgagac ttgacttagc
13680

tatctttaag agttatgaac ttaatttaga gtcatattcc acaatagagc taatgaacgt
13740

tctttcaata tctagtggga agttgattgg ccagtctgtg gtttcttatg atgaagatac
13800

ctccataaag aatgacgcta taatagtgta tgacaacaca cggaattgga tcagcgaagc
13860

tcagaattca gatgtggtcc gcctattcga gtatgcggca ctcgaagtgc tcctcgactg
13920

ttcttatcaa ctctactatc tgagagtaag aggcctaaac aatatcgtcc tgtacatgag
13980

tgatttatac aagaatatgc caggaattct actctctaat attgcagcca caatatctca
14040

ccctgtcatc cattcaaggt tgaatgcagt aggtctggtc aaccatgacg ggtcacacca
14100

acttgcagac acagatttca ttgaaatgtc tgcaaagctg ctagtctttt gcactcgacg
14160

cgtggtctca ggtttacatg cagggaataa gtatgacctg ctgtttccat ctgtcttgga
14220

tgataaccta agtgagaaga tgcttcagtt gatatcccaa ttatgctgtc tgtatacggt
14280

gctctttgct acaacaagag aaatcccgaa aataagaggc ttatctgcag aagagaaatg
14340

ttcagtactt actgagtacc tactgtcaga tgctgtgaaa ccgttacttg ggtccgagca
14400

agtgagctct atcatgtctc ccaacatagt tacgttccca gccaatctgt attacatgtc
14460

taggaagagc cttaatttga tcagggagag agaggacagg gatactatct tggcattgtt
14520

gttccctcaa gaaccgctgc tcgagtttcc tctggtacga gatattggtg ctcgtgtaaa
14580

agatccattt acccgacaac ctgcggcgtt tttacaagag ttagatttga gtgctccggc
14640

aaggcatgac gcattcacaa tcagtcaggc gcattctgaa cacatattgc caaacccaga
14700

ggaagatcac ttagtacgat acttgttcag aggaataggg actgcgtcct cctcttggta
14760

taaggcatcc catcttcttt ctgtacccga ggtcagatgt gcaaggcatg ggaactcctt
14820

atatttagca gaaggaagtg gagccatcat gagtcttctc gaattgcata taccacacga
14880

aactatctat tacaatacac ttttctcgaa cgagatgaac cccccacagc gacatttcgg
14940

accaactcca acacagtttc tgaattcggt tgtttttagg aatttacagg cggaagtacc
15000

atgcaaggat ggatttgtcc aggagttccg tccgttatgg agagagaata cagaagaaag
15060

cgatctgacc tcagataaag cagtgggata tatcacatct gtagtgccgt acaggtctgt
15120

atcattgctg cattgtgaca ttgaaatccc tccaggatct aatcaaagct tactagatca
15180

gctggctacc aatctgtctc tgattgccat gcattctgtg aaggagggcg gggtcgtgat
15240

tatcaaagta ctgtatgcaa tgggatatta cttccatcta ctcatgaact tgtttactcc
15300

atgttccacg aaaggatata ttctctctaa tggctatgcc tgtagagggg atatggagtg
15360

ttacctgata tttgtcatgg gctacctagg cgggcctaca tttgtacatg aggtggtgag
15420

gatggcaaaa acgctagtac agcggcatgg cacacttttg tctaagtcag atgagattac
15480

actgactagg ttatttactt cacagcaaca gcgtgtaaca gacatcctat ccagcccttt
15540

accgagacta atgaagtact tgagagagaa tattgatact gcactgattg aagccggggg
15600

acagcctgtc cgtccattct gtgcagagag tttagtgagc acactaacag acatgactca
15660

gacaacccag atcatcgcca gccacattga tacagtcatt cgatctgtga tctatatgga
15720

agcagagggt gatcttgctg acacagtatt cttatttacc ccttacaacc tctctatgga
15780

cgggaaaaag agaacatcac ttaaacagtg cacaagacag atcttagagg tcacaatact
15840

gggtctcaga gtcaaaaatc tcaataaagt aggtgatgta atcagcctag tactcagagg
15900

tatgatttct ctggaggacc ttctcccact gagaacctac ttgaagtgta gtacctgccc
15960

taagtatttg aaggctgtcc taggtattac caaactcaaa gaaatgttca cagacgcctc
16020

tttattatac ttgactcgtg ctcaacaaaa attctacatg aaaactatag gcaatgcagt
16080

caagggatac tatagtaact gtgactctta aaggcaacca catatcaata ggccctcttt
16140

ctagccgatc gtattcttgt tgacttcatt ataccatatt agaaaaaaat tgaattccga
16200

ccctttaaga ctcgtattcg gattcaaata attatctcag aaaaaaagtg cacgtagttg
16260

ttcttgatta tagtcccgtc attcaccaag tctttgtttg gt
16302

<210> 4
<211> 17412
<212> DNA
<213> Newcastle disease virus

<220>
<223> >rgMutHu-Nivolumab final sequence.dna (17,412 bp)

<400> 4
accaaacaga gaatccgtga gctacgataa aaggcgaagg agcaatcgaa gtcgtacggg
60

tagaaggtgt gaatctcgag tgcgagaccg aagcttaaac tcgagagagc cttctgccaa
120

catgtcttcc gtatttgacg agtatgaaca gctcctcgcg gctcagactc gccctaatgg
180

agctcacgga ggaggagaga aggggagcac cttaaaagtt gaagtcccgg tattcactct
240

taacagtgac gacccagaag atagatggaa ctttgcagta ttctgtctta ggattgctgt
300

tagcgaagat gccaacaaac cactcagaca aggtgctctc atatctctct tatgctccca
360

ttctcaagtg atgaggaacc atgtcgccct tgcagggaaa cagaatgagg ccacactggc
420

tgttcttgag atcgatggtt tcaccaacag cgtgcctcaa tttaacaaca ggagtggagt
480

gtctgaggag agagcacaga gattcttgat gatagcaggg tccctccctc gggcatgcag
540

caacggtacc ccgttcatca cagctggggt tgaagatgat gcaccagaag acatcactga
600

tactctggaa agaatcatct ctatccaggc tcaagtatgg gtcacggtag caaaggccat
660

gactgcatat gagacagcgg atgagtcgga aacaagaaga atcaataagt atatgcagca
720

aggcagagtc caaaagaagt acatcctcca ccccgtatgt aggagcgcaa ttcaactcac
780

gattagacaa tctctggcag tccgcatttt cttggttagc gagcttaaga gaggccgcaa
840

cacggcaggt ggaagttcca cttattatag cttggtaggg gacatagact catacatcag
900

gaacaccggg cttactgcat tcttcctgac actcaggtat ggaattaaca ccaagacatc
960

agcccttgca ctcagcagcc tcgcaggcga tatccaaaaa atgaagcagc tcatgcgtct
1020

atatcggatg aaaggagata atgcgccgta catgacactg ctcggtgaca gtgaccagat
1080

gagctttgca ccggctgagt atgcacaact ctactctttt gcaatgggta tggcatccgt
1140

cctagacaaa ggaactggca agtaccagtt cgccagagac tttatgagca catcattctg
1200

gagacttgga gtagagtatg ctcgggctca gggaagcagc atcaatgagg atatggctgc
1260

cgagctaaag ctgaccccag cggcaaggag gggcctggca gctgctgccc aacgagcatc
1320

cgaggagacc ggcagcatgg acattcccac tcaacaagct ggggtcctca cagggctcag
1380

cgacggaggc cctcaagccc cacaaggtgg actgaacagg tcgcaagggc aaccggacgc
1440

cggagatggg gagacccaat ttttagattt gatgagagca gtggcaaata gtatgagaga
1500

agcgccaaac tctgtgcaga acaccactca gcaggagcct ccatctactc ctgggccatc
1560

tcaagacaac gacaccgact ggggatactg accgacaaca ctcagcctgc ctccatcgaa
1620

ttatctcgat tcttctgcct gtaacctaac ccctggtcca cagtctcaca cggccaaacc
1680

cgcaaacgag cccccccctc tctccccccc cctccagcca cacggctcca accgcccaaa
1740

acaacacggg cacaactcga ctcactcata atctacacag agccgaagat cttagaaaaa
1800

aatacgggta gaagagagac attcagagat caggacaaat catcagggtc tccgctctcc
1860

cctctaccca gcagaccaga gtgaagatgg ccaccttcac ggatgcagag atcgacgatc
1920

tatttgaaac cagtggaact gtcattgaca gcataattac ggcccaaggc aacccagtag
1980

agactgttgg aaggagcgca atcccacaag gcaaaactaa agcattgagt gcagcatggg
2040

agaaacatgg aagcacccaa ccgccggccg gccaagacac ccctgatcga atggacagat
2100

cagacaaaca accatcccca cccgaacagg caaccacaca caacagcccg ccagccacat
2160

ccaccgacca gccccccacc caggccgcag gcgaagccgg tgacacacag ctcaagactg
2220

gagcaagcaa ctccctcctg tcgatgctcg acaagcttag caataaatcg tccaatgcta
2280

aaaagggccc acggtcgagt ccccaggaag ggcaccacca acctccgacc cagcagcagg
2340

ggagtcaaca gagccgcgga aacagtcagg agagatcgca aaaccaggtc aaggccgcct
2400

ctggagacca gggcacagac gcgaacacag catatcatgg acaatggaag gagtcacaac
2460

catcagctgg tgcaatccct catgctctcc ggtcagggca gagccaagcc aatactcctg
2520

catctgtgga tcatgtccag ctacctgtcg actttgtgca ggcgatgatg tctatgatgg
2580

aggcgatatc acagaaggta aataaagtcg actatcagct agaccttgtc tcaaagcaga
2640

catcctccat ccccatgatg cgatctgaaa tccaacagct gaaaacatcc gttgcggtca
2700

tggaagccaa cctgggcatg atgaagattc tggaccctgg ttgtgctaac gtttcatctt
2760

taagtgacct acgggcggtc gcccgctctc acccagtttt agtttcaggc cccggagacc
2820

catctcctta tgtgacacaa gggggtgaat tgatactcaa taaactctcg caaccagtgc
2880

aacatccttc tgagttgatt aaacctgcca cgataagcgg gcctgatata ggagtggaga
2940

aggacactgt ccgtgcattg atcacctcac gcccaatgca tccgagctct tcagctaagc
3000

tcctgagtaa gctggatgca gccgggtcga tagaagaaat taggaaaatc aaacgccttg
3060

cgctgaatgg ctgatcacta ctacaacccg caacgggttc ccgtttatct attgtcacaa
3120

ggaacccgcc ccgaagctta agaaaaaata cgggtagaag cagtttgaga gctccgttcg
3180

ccaccatgga gttcggcctg tcctgggtgt tcctggtggc cctgtttaga ggcgtgcagt
3240

gccaggtgca gctggtggag agcggaggag gagtggtgca gccaggccgg agcctgcgcc
3300

tggactgtaa ggccagcggc atcaccttta gcaactccgg aatgcactgg gtgaggcagg
3360

caccaggcaa gggcctggag tgggtggccg tgatctggta cgacggctct aagcggtact
3420

atgccgatag cgtgaagggc cggttcacaa tctccagaga caactctaag aataccctgt
3480

tcctccagat gaacagcctg agggccgagg atacagccgt gtactattgc gccaccaatg
3540

acgattactg gggccagggc acactggtga ccgtgagcag cgccagcacc aagggaccaa
3600

gcgtgttccc actggcacct tgctctcgca gcacatccga gtctaccgcc gccctgggat
3660

gtctggtgaa ggattatttc cccgagcctg tgaccgtgag ctggaacagc ggcgccctga
3720

catccggagt gcacaccttt cccgccgtgc tccagagcag cggcctgtac agcctgtcct
3780

ctgtggtgac agtgcccagc tcctctctgg gcaccaagac atatacctgc aacgtggacc
3840

acaagcctag caataccaag gtggataagc gggtggagtc caagtacgga ccaccttgcc
3900

caccatgtcc agcaccagag ttcctgggag gaccaagcgt gttcctgttt cctccaaagc
3960

ccaaggacac cctgatgatc tccagaacac cagaggtgac ctgcgtggtg gtggacgtgt
4020

ctcaggagga ccccgaggtg cagttcaact ggtatgtgga tggcgtggag gtgcacaatg
4080

ccaagaccaa gcccagggag gagcagttta actccacata ccgcgtggtg tctgtgctga
4140

ccgtgctgca ccaggactgg ctgaacggca aggagtataa gtgcaaggtg tccaataagg
4200

gcctgcctag ctccatcgag aagacaatca gcaaggcaaa gggacagcca agggagccac
4260

aggtgtacac cctgccccct tctcaggagg agatgacaaa gaaccaggtg agcctgacct
4320

gtctggtgaa gggcttctat ccttccgaca tcgccgtgga gtgggagtct aatggccagc
4380

cagagaacaa ttacaagacc acaccacccg tgctggactc tgatggcagc ttctttctgt
4440

attccaggct gacagtggat aagtctcgct ggcaggaggg caacgtgttt agctgctccg
4500

tgatgcacga ggccctgcac aatcactaca cccagaagtc tctgagcctg tccctgggca
4560

agcggagaaa gaggggcagc ggagagggaa ggggctccct gctgacatgt ggcgacgtgg
4620

aggagaatcc tggcccaatg aagtatctgc tgcccaccgc cgccgccggc ctgctgctgc
4680

tggcagcaca gcctgctatg gccgagatcg tgctgacaca gtccccagcc accctgtctc
4740

tgagccctgg agagagggcc accctgagct gtagagcatc ccagagcgtg agcagctacc
4800

tggcctggta tcagcagaag cctggacagg caccaaggct gctgatctac gatgcaagca
4860

acagggcaac aggcatcccc gcaagattca gcggctccgg ctctggcacc gactttacac
4920

tgaccatctc ctctctggag cctgaggatt tcgccgtgta ctattgccag cagagcagca
4980

attggccacg gacatttggc cagggcacca aggtggagat caagagaaca gtggccgccc
5040

ctagcgtgtt catctttcct ccatccgacg agcagctgaa gagcggcacc gcatccgtgg
5100

tgtgcctgct gaacaatttc tacccacggg aggccaaggt gcagtggaag gtggataacg
5160

ccctccagtc cggcaattct caggagagcg tgacagagca ggactccaag gattctacct
5220

atagcctgtc tagcacactg accctgtcca aggccgacta cgagaagcac aaggtgtatg
5280

cctgcgaggt gacacaccag ggcctgtcct ctcccgtgac caagagcttt aacagaggcg
5340

agtgctgata agctgaccga gccctcctct ataaacccaa gattcaaggc tccaagcgat
5400

aaccttctcc cgcctcttcc atcccactga atgatcgcgc agctgcaatt aatctagcaa
5460

cattaaagat taagaaaaaa tacgggtaga atcggagtgc accgattgtg ccaagatgga
5520

ctcatctagg acaattgggt tatactttga ttctgccctt ccttctagca acctgttagc
5580

attcccgatt gtcttacgag atgtaggaga tggaaagaag caaattactc cgcaatatag
5640

gatccggcgt cttgactcgt ggacagacag taaagaagac tcggtattca tcactaccta
5700

cggattcatc ttccaggttg ggaatgaaga agtcactgtc ggcatgatca atgataatcc
5760

caagcgagag ttactctccg ctgcgatgct ctgcctagga agcgtcccga atgttggaga
5820

tcctgttgag ctggcaaggg cctgcctcac tatggtggta acatgcaaga agagtgcaac
5880

taatactgag agaatggtct tctcagtagt gcaggcaccc cgggtgctgc aaagctgtag
5940

ggtcgtggca gacaaatact cgtcagtgaa tgcagttaag cacgtgaaag caccagagaa
6000

gatcccttgg agtgaaaccc tagagtacaa ggtgaatttt gtctctttga ctgtggtgcc
6060

gaagaaggat gtctacaaga tcccaaccgc agtattgaag gtatccggct cgagcctgta
6120

taatcttgcg ctcaatgtca ctattgatgt ggaggtagac ccaaagagcc cgttagtcaa
6180

gtccctttct aggtccgaca gtggatacta tgctaatctc ttcttacata tcggacttat
6240

gtccactgta gataagaggg gaaagaaagt gacatttgac cagctggaga ggaagataag
6300

gagacttgat ctatctgtcg ggctcagtga tgtgctcgga ccttctgtgc ttgtgaaggc
6360

gagaggtgca cggactaggc tgctggcacc tttcttctct aacagcggga cagcctgcta
6420

ccctatagca aatgcctctc ctcaggtagc taagatactc tggagtcaga ccgcgtgcct
6480

gcggagtgta aaaatcatta ttcaagcggg cacccaacgc gctgtcgcag tgactgctga
6540

ccatgaggtt acctctacta agatagagaa gaggcatacc atcgctaaat acaatccctt
6600

caagaaatag gccgtatccc tgagactgca attcacccgc cttcccaaaa caccatgaca
6660

ccagataatg atctgtcttg attacttaca gttagtttcc ctgtctatca aattagaaaa
6720

aacacgggta gaagagttcg gatcccggcc ggcgcaccaa aagcgcaaga tgggccccag
6780

atcttctacc aggatcccag tacctctaat gctgaccata cggatcacgc tggcactgag
6840

ttatgtccgt ctgacaagtt ctcttgatgg caggcctctt gcagccgcag ggatcgtggt
6900

aacaggggat aaagcagtta acatatacac ctcatcccag acagggtcaa tcatagtcaa
6960

gttactccca aatatgccca aggacaaaga ggcatgtgca aaagccccat tggaggctta
7020

caacaggaca ctgactactt tgcttacccc ccttggtgat tctatccgca ggatacaaga
7080

gtctgtgact acatccggag gaaggagaca gagacgcttt ataggtgcca ttattggcag
7140

tgtagctcta ggggttgcaa cagctgcaca gataacggca gcctcggctc tgatacaagc
7200

caaccagaat gctgctaaca tcctccggct taaggagagc attgctgcaa ccaatgaagc
7260

tgtacacgag gtcactggcg gattgtcaca gttagcagtg gcagttggaa agatgcagca
7320

atttgttaat gaccagttta ataacacagc tcaagagttg gactgtataa aaattacaca
7380

gcaggttggt gtagaactca acttatactt aactgaattg actacagtgt tcgggccaca
7440

aatcacttcc cctgccttaa ctcagttgac tgttcaggct ctttacaatc tggctggtgg
7500

taatgtagat tacttgttga ctaagttagg tgtagggaac aaccagctca gctcattgat
7560

tggtagcggc ttgatcaccg gtaaccctat tttttacgac tcacagactc aactcttggg
7620

catacaggtg actttaccct cagtcgggaa cctaaataat atgcgtgcca cctacttgga
7680

gaccttgtct gtaagcacaa caaagggatt tgcctcagca cttgtcccaa aagtagtgac
7740

acaggtcggt tctgtgatag aagagcttga cacctcatac tgtatagaag ctgatttgga
7800

tttatattgt acaagaatag tgacattccc tatgtcccct ggtatttatt cctgtttgag
7860

cggcaataca tcggcttgca tgtattcaag gactgaaggc gcacttacta caccatacat
7920

gactctcaaa ggctcagttg ttgccaattg ccagatgaca acatgtagat gtgcagaccc
7980

cccgggtatc atatcacaaa attatggaga ggctgtgtct ctaatagata agcactcatg
8040

caatgtcgta tccttagacg ggataacttt gaggctcagt ggggaatttg atgcaactta
8100

tcaaaagaat atctcaatat tagattctca agtactagtg acaggcaatc tcgatatctc
8160

aactgagctt gggaatgtca accactcaat aagtaatgct ttggataagt tagaggaaag
8220

caacagcaaa ctagacaaag tcaatgtcaa actgaccagc acatctgctc tcattaccta
8280

tattgtttta actgtcatat ctcttgttct tggtatgctt agcctggttc tagcatgcta
8340

tctgatgtac aagcaaaaag cgcaacgaaa gaccttgttg tggcttggaa ataataccct
8400

agatcagatg agagctacta caaaaatgtg aatgcagatg agaggcagag gtatccccaa
8460

tagcaatctg tgtgtcaatt ctggcagcct gttaatcaga agaattaaga aaaaactacc
8520

ggatgtaggt gaacaaaagg gaatatacgg gtagaacggc ctgagaggcc acccctcaat
8580

cgggagccag gccccactac gtccgctcta ccgcatcacc aacagcagtc ttcagtcatg
8640

gacagcgcag ttagccaagt tgcgctagag aatgatagaa gagaagcgaa taatacatgg
8700

cgcttggttt tccggatcgc agccttactt ttactggtaa taaccttagc cgtctctgca
8760

gtcgccctgg catatagtat ggaggctagc acacctggcg accttgtaag cataccgact
8820

gcgatctcta gggcagagga aaggattaca tctgcactcg gttccaatca agatgtagta
8880

gataggatat acaagcaagt ggcccttgag tctccattgg cactgctaaa taccgaatct
8940

ataattatga atgcaataac gtctctctct tatcaaatca atggagccac aaataatagc
9000

gggtgtgggg cacctgttca tgacccagat tacatcgggg ggataggtca agaacttatt
9060

gtagatgata cgagtgacgt cacatcattt tatccctctg cattccaaga acacctgaat
9120

tttatcccgg cgcccactac aggatcaggc tgcactcgga taccctcatt cgacatgagt
9180

gctacccatt actgttacac tcacaatgtg atattgtctg gctgcagaga tcactcacac
9240

tcacatcagt atttagcact tggtgtgctt cggacatctg caacagggag ggtattcttt
9300

tctactctgc gttccatcaa cttggatgac gcccaaaatc ggaagtcttg cagtgtgagt
9360

gcaactcctt taggttgtga tatgctgtgc tctaaaatca cagagactga ggaagaggat
9420

tataaatcag ttatccccac atcgatggta catggaaggt tagggcttga cggccaatac
9480

catgagaagg acctagacgt cacaacacta tttaggactt gggtggcaaa ttacccagga
9540

gtaggaggtg ggtcttttat taacaatcgc gtatggttcc cggtctacgg agggctaaaa
9600

cccagctcgc ctagtgacac tgcacaagaa gggagatacg taatatacaa gcgatacaat
9660

gacacatgcc cggatgagca agattaccag attcggatgg ctaagtcatc gtataagcct
9720

aggcggtttg gtggaaaacg cgtacagcag gccatcctat ccatcaaggt gtcaacatcc
9780

ttgggtgagg acccggtgtt gactgtaccg cctaatacgg tcgcactcat gggggccgaa
9840

ggcagagttc tcacagtggg gacatctcat ttcttatatc agcgagggtc atcatacttc
9900

tctcccgctt tgttataccc tatgacagtc aacaacaaaa cagccactct tcataatcct
9960

tatacattca atgctttcac tcggccaggt agtgtccctt gccaggcttc agcaagatgc
10020

cccaactcat gtgttaccgg agtctatact gatccatatc ccttagtctt ccataggaac
10080

cacaccttgc gaggggtatt cggaacaatg cttgatgacg aacaagcaag actcaaccct
10140

gtatctgcag tatttgataa catatcccgc agtcgcataa ctcgggtaag ttcaagcagt
10200

accagggcag catacacgac atcaacgtgt tttaaagttg tcaagaccaa taaaacctat
10260

tgcctcagca ttgcagaaat atccaatacc ctcttcgggg aattcagaat tgtcccctta
10320

ctagttgaga ttctcaagga tggtggggtt taagaagcta ggaatggtcg gttgagtcaa
10380

ccgtgagaag gcggggaaga tgatattgca tcatctatct tctgtaacac caagaatcaa
10440

atcgaatacc agtgcgagct cgaatcctac gctgccagtc agccataatc ggctggtgct
10500

aatgtggtta gcctgaatct tgtcgatagt cacttgatta agaaaaaatg tggacggtag
10560

tgaaatataa ggcaaagcag ctcacagagg acagcacggg taggacatgg cgggctccgg
10620

acccgaaagg gcagagcatc agattatcct accagagtca catctgtcct caccattggt
10680

caagcataaa ttgctttatt actggaaatt aactgggcta ccgcttcctg acgaatgtga
10740

cttcgaccat cttattatca gccgacaatg gaagaaggta cttgaatcgg ccacccctga
10800

cattgagaga atgataaaac tagggcgggc agtacaccag actctcaacc acaattccag
10860

gataaccgga gtactccatc cccggtgttt agaggaactg gctagtattg aggttcctga
10920

ttcaaccaac aaatttcgga agatcgaaaa aaagatccag attcacaaca caaggtatgg
10980

agaactgttc acaagattat gcacgcatgt agaaaagaaa ctattggggt catcttggtc
11040

taacaatgtc ccacggtcag aggaattcaa cagcatccgt acagatccgg cattttggtt
11100

tcactcaaaa tggtccacag ccaaatttgc atggctccat ataaaacagg tccagaggca
11160

tctgattgta gcagcaagaa caaggtccgc agtcaacaaa ttagtgacgc tgacccataa
11220

ggtaggccaa atctttgtta ctcctgagct tgtcattgtg acacatacag atgagaacaa
11280

gttcacgtgt cttacccagg aacttgtgtt gatgtatgca gatatgatgg agggcagaga
11340

tatggtcagc ataatatcat ccacggcggc acatcttagg agcttatcag agaaaattga
11400

tgatattctg cggttagtag atgctctggc aagagatttg ggcaatcaag tctacgatgt
11460

tgtagcacta atggagggat tcgcatacgg cgctgttcag ctgcttgaac cgtcaggtac
11520

atttgcgggg gatttcttcg cattcaacct gcaggagctc aaagatactc taaccggact
11580

cctccccaag gatatcgcag aatctgtgac tcacgcaatc gcaaccatat tctctggctt
11640

agaacaaaat caagcagctg agatgttgtg cctgttgcgt ctgtggggtc acccactact
11700

cgagtcccgt attgcagcaa aagcagttag gagtcaaatg tgcgcaccaa aaatggtaga
11760

ctttgatatg atcctccagg tattatcttt ctttaaagga acaatcatca atggatatag
11820

aaagaagaat gcaggcgtgt ggccacgtgt caagatagat acgatatacg ggaaggtcat
11880

agggcagcta cacgcagatt ctgcagagat ttcacatgat gtcatgttga gggaatacaa
11940

gagtttatct gcacttgaat tcgagccatg tatagagtat gaccctgtca ccaatctgag
12000

catgttttta aaagacaagg caatcgcaca cccgaaagac aactggctcg cttcgtttag
12060

gcgaaacctt ctctctgagg accagaagaa acatgtaaga gaggcaacct caactaaccg
12120

cctcttgata gagttcttag agtcaaatga ttttgatcca tataaagaga tggaatatct
12180

gacgaccctt gagtacctaa gagatgataa tgtggcagta tcatactcac tcaaagaaaa
12240

agaggtgaaa gttaatgggc ggattttcgc aaagctaaca aatagattaa ggaattgtca
12300

ggtaatggca gaagggatcc tagctgacca gattgcacct ttcttccagg gaaatggggt
12360

cattcaggat agcatatctt tgactaaaag tatgctagcg atgagtcaac tgtctttcaa
12420

cagcaacaag aaacgtatta ctgactgcaa agaaagagta tcctcaaacc gcaatcatga
12480

tccgaagagc aagaatcgtc ggagagttgc cacttttgta actaccgacc tgcaaaagta
12540

ttgtctcaat tggagatatc agacagtcaa gctgttcgct catgccatca atcagctgat
12600

gggcttacct cacttcttcg agtggattca tcttagacta atggatacta cgatgtttgt
12660

aggggaccct ttcaatcctc caagtgaccc tacggactgt gatctatcaa gagtcccaaa
12720

tgatgacata tatattgtca gtgctagggg aggcattgag ggattatgcc agaagctatg
12780

gacaatgatc tcaattgctg caatccagct tgctgcagca agatcacatt gtcgcgttgc
12840

ctgtatggta caaggcgaca atcaagtaat agctgtaacg agagaggtaa gatcagatga
12900

ctccccagag atggtgttaa cacaattgca tcaagccagt gataatttct tcaaggaatt
12960

gattcatgtc aatcatttga tcggccataa tttgaaggat cgtgaaacca tcaggtcaga
13020

cacgttcttc atatacagca aacgaatatt caaagatgga gcaatactca gtcaggtcct
13080

caaaaactca tctaaattag tgctaatatc aggcgacctt agtgaaaaca ctgtaatgtc
13140

ttgtgccaac attgcatcta ctgtagcacg gttatgcgag aacgggcttc ctaaggattt
13200

ctgttattac ttaaactact taatgagttg cgtgcagaca tacttcgatt ccgaattttc
13260

catcaccaac aactcgcaac tcgattctaa ccagtcgtgg atagaggaca tttcttttgt
13320

gcactcatat gtcctgaccc ctgctcaact ggggggactg agtaaccttc aatactcaag
13380

gctctacaca aggaacatcg gcgacccggg aaccactgct ttcgcagaga tcaagagatt
13440

agaggcagtg gggttactga gtcctagcat tatgactaac atcttaacta ggccgcctgg
13500

aaatggagat tgggccagtc tgtgcaacga tccatactcc tttaattttg agactgtcgc
13560

aagcccaaat attgtcctta agaaacatac acaaagagtc ctatttgaaa cttgctcgaa
13620

tcccttatta tctggagtgc acacagagga taatgaggca gaagagaagg cattggctga
13680

attcttgctc aatcaagaaa tgattcatcc acgtgtcgcg catgctatca tggaagcaag
13740

ctctgtaggt aggagaaagc aaattcaggg gcttgttgac acaacaaaca ccgtgattaa
13800

gattgcactg actaggaggc cactcggcat caagaggctg atgcggatag tcaattactc
13860

gagcatgcat gcaatgctat ttagagatga tgttttctcg tctaatagat ccaaccatcc
13920

cttagtctct tctgatatgt gttctctgac actggcagac tatgcacgga acagaagctg
13980

gtcacctttg acagggggta gaaaaatact gggtgtatct aatcctgata ccatagaact
14040

tgtagagggt gagatcctta gtgtcagtgg agggtgcact agatgtgaca gtggggatga
14100

acagtttact tggttccatc ttccaagcaa tatagagcta aacgatgaca ccagcaagaa
14160

tcctccaatg agagtgccat atctcgggtc aaagactcaa gagaggagag ccgcctcgct
14220

tgcgaaaata gctcatatgt caccacacgt gaaggcggct ctaagggcgt catctgtgtt
14280

aatctgggct tatggggaca acgaaataaa ctggactgct gcccttaaga ttgcaaggtc
14340

tcggtgcaac ataagctcag agtatcttcg actattgtca cccttgccta cagctgggaa
14400

tctccaacat agattggacg acggcataac tcagatgaca ttcacccctg catctctata
14460

cagggtgtca ccttacattc atatatctaa tgattctcaa aggctattta ctgaagaagg
14520

agtcaaagag gggaatgtgg tttatcagca aatcatgctc ttgggtttgt ccttaattga
14580

gtcactcttc ccaatgacaa caaccaagac atatgatgaa atcacattgc acctccacag
14640

taaatttagc tgctgtatca gggaagcacc tgttgcagtt cctttcgagc tactcggggt
14700

ggcaccggaa ctaagggcag taacctcaaa taagtttatg tatgatccta gccctgtatc
14760

agagggagac tttgcgagac ttgacttagc tatctttaag agttatgaac ttaatttaga
14820

gtcatattcc acaatagagc taatgaacgt tctttcaata tctagtggga agttgattgg
14880

ccagtctgtg gtttcttatg atgaagatac ctccataaag aatgacgcta taatagtgta
14940

tgacaacaca cggaattgga tcagcgaagc tcagaattca gatgtggtcc gcctattcga
15000

gtatgcggca ctcgaagtgc tcctcgactg ttcttatcaa ctctactatc tgagagtaag
15060

aggcctaaac aatatcgtcc tgtacatgag tgatttatac aagaatatgc caggaattct
15120

actctctaat attgcagcca caatatctca ccctgtcatc cattcaaggt tgaatgcagt
15180

aggtctggtc aaccatgacg ggtcacacca acttgcagac acagatttca ttgaaatgtc
15240

tgcaaagctg ctagtctttt gcactcgacg cgtggtctca ggtttacatg cagggaataa
15300

gtatgacctg ctgtttccat ctgtcttgga tgataaccta agtgagaaga tgcttcagtt
15360

gatatcccaa ttatgctgtc tgtatacggt gctctttgct acaacaagag aaatcccgaa
15420

aataagaggc ttatctgcag aagagaaatg ttcagtactt actgagtacc tactgtcaga
15480

tgctgtgaaa ccgttacttg ggtccgagca agtgagctct atcatgtctc ccaacatagt
15540

tacgttccca gccaatctgt attacatgtc taggaagagc cttaatttga tcagggagag
15600

agaggacagg gatactatct tggcattgtt gttccctcaa gaaccgctgc tcgagtttcc
15660

tctggtacga gatattggtg ctcgtgtaaa agatccattt acccgacaac ctgcggcgtt
15720

tttacaagag ttagatttga gtgctccggc aaggcatgac gcattcacaa tcagtcaggc
15780

gcattctgaa cacatattgc caaacccaga ggaagatcac ttagtacgat acttgttcag
15840

aggaataggg actgcgtcct cctcttggta taaggcatcc catcttcttt ctgtacccga
15900

ggtcagatgt gcaaggcatg ggaactcctt atatttagca gaaggaagtg gagccatcat
15960

gagtcttctc gaattgcata taccacacga aactatctat tacaatacac ttttctcgaa
16020

cgagatgaac cccccacagc gacatttcgg accaactcca acacagtttc tgaattcggt
16080

tgtttttagg aatttacagg cggaagtacc atgcaaggat ggatttgtcc aggagttccg
16140

tccgttatgg agagagaata cagaagaaag cgatctgacc tcagataaag cagtgggata
16200

tatcacatct gtagtgccgt acaggtctgt atcattgctg cattgtgaca ttgaaatccc
16260

tccaggatct aatcaaagct tactagatca gctggctacc aatctgtctc tgattgccat
16320

gcattctgtg aaggagggcg gggtcgtgat tatcaaagta ctgtatgcaa tgggatatta
16380

cttccatcta ctcatgaact tgtttactcc atgttccacg aaaggatata ttctctctaa
16440

tggctatgcc tgtagagggg atatggagtg ttacctgata tttgtcatgg gctacctagg
16500

cgggcctaca tttgtacatg aggtggtgag gatggcaaaa acgctagtac agcggcatgg
16560

cacacttttg tctaagtcag atgagattac actgactagg ttatttactt cacagcaaca
16620

gcgtgtaaca gacatcctat ccagcccttt accgagacta atgaagtact tgagagagaa
16680

tattgatact gcactgattg aagccggggg acagcctgtc cgtccattct gtgcagagag
16740

tttagtgagc acactaacag acatgactca gacaacccag atcatcgcca gccacattga
16800

tacagtcatt cgatctgtga tctatatgga agcagagggt gatcttgctg acacagtatt
16860

cttatttacc ccttacaacc tctctatgga cgggaaaaag agaacatcac ttaaacagtg
16920

cacaagacag atcttagagg tcacaatact gggtctcaga gtcaaaaatc tcaataaagt
16980

aggtgatgta atcagcctag tactcagagg tatgatttct ctggaggacc ttctcccact
17040

gagaacctac ttgaagtgta gtacctgccc taagtatttg aaggctgtcc taggtattac
17100

caaactcaaa gaaatgttca cagacgcctc tttattatac ttgactcgtg ctcaacaaaa
17160

attctacatg aaaactatag gcaatgcagt caagggatac tatagtaact gtgactctta
17220

aaggcaacca catatcaata ggccctcttt ctagccgatc gtattcttgt tgacttcatt
17280

ataccatatt agaaaaaaat tgaattccga ccctttaaga ctcgtattcg gattcaaata
17340

attatctcag aaaaaaagtg cacgtagttg ttcttgatta tagtcccgtc attcaccaag
17400

tctttgtttg gt
17412

<210> 5
<211> 36
<212> DNA
<213> Newcastle disease virus

<220>
<223> cDNA fragment

<400> 5
acgactcact ataggaccaa acagagaatc cgtgag                                36

<210> 6
<211> 35
<212> DNA
<213> Newcastle disease virus

<220>
<223> cDNA fragment

<400> 6
ccgggaagat ccagggcact cttcttgcat gttac                                 35

<210> 7
<211> 35
<212> DNA
<213> Newcastle disease virus

<220>
<223> cDNA fragment

<400> 7
gggcctgcct cactatggtg gtaacatgca agaag                                 35

<210> 8
<211> 35
<212> DNA
<213> Newcastle disease virus

<220>
<223> cDNA fragment

<400> 8
tgcatgttac caccaatgtg tcattgtatc gcttg                                 35

<210> 9
<211> 37
<212> DNA
<213> Newcastle disease virus

<220>
<223> cDNA fragment

<400> 9
caagaaggga gatacgtaat atacaagcga tacaatg                               37

<210> 10
<211> 35
<212> DNA

<213> Newcastle disease virus

<220>
<223> cDNA fragment

<400> 10
tcgcttgtat attacttgtt gtagcaaaga gcacc                                35

<210> 11
<211> 38
<212> DNA
<213> Newcastle disease virus

<220>
<223> cDNA fragment

<400> 11
ggcctggatc ttcccattat gctgtctgta tacggtgc                             38

<210> 12
<211> 38
<212> DNA
<213> Newcastle disease virus

<220>
<223> cDNA fragment

<400> 12
atgccatgcc gacccaccaa acaaagactt ggtgaatg                             38

<210> 13
<211> 19
<212> DNA
<213> Newcastle disease virus

<220>
<223> cDNA fragment

<400> 13
accaaacaga gaatccgtg                                                  19

<210> 14
<211> 20
<212> DNA
<213> Newcastle disease virus

<220>
<223> cDNA fragment

<400> 14
agtcttcagt catggacagc                                                 20

<210> 15
<211> 35
<212> DNA
<213> Newcastle disease virus

<220>
<223> helper plasmid

<400> 15
ctctagagtc gacccttctg ccaacatgtc ttccg                                35

<210> 16
<211> 35
<212> DNA
<213> Newcastle disease virus

<220>
<223> helper plasmid

<400> 16
gggaagcggc cgcccgtcgg tcagtatccc cagtc                                35

<210> 17
<211> 37
<212> DNA
<213> Newcastle disease virus

<220>
<223> helper plasmid

<400> 17
ctctagagtc gaccccagag tgaagatggc caccttc                              37

<210> 18
<211> 35
<212> DNA
<213> Newcastle disease virus

<220>
<223> helper plasmid

<400> 18
gggaagcggc cgcccagtga tcagccattc agcgc                                35

<210> 19
<211> 35
<212> DNA
<213> Newcastle disease virus

<220>
<223> helper plasmid

<400> 19
ctctagagtc gacccgggta ggacatggcg ggctc                                35

<210> 20
<211> 37
<212> DNA
<213> Newcastle disease virus

<220>
<223> helper plasmid

<400> 20
gggaagcggc cgccctgcct ttaagagtca cagttac                              37

## Description

This invention relates to Newcastle Disease Virus (NDV), an avian paramyxovirus, which has been demonstrated to possess significant oncolytic activity against mammalian cancers, especially selected from the specific cancers as described herein and/or mentioned in the claims. The invention provides the elucidation of the mechanisms of NDV-mediated oncolysis as well as the development of novel and improved oncolytic agents through the use of genetic engineering.

### Background

Oncolytic viruses, which are well known to inherently replicate selectively within tumor cells and kill tumor cells while leaving non-tumor cells unharmed, provide an attractive new tool for cancer immunotherapy. Typically, an oncolytic virus is defined as a virus for use in oncological treatment, preferably in treatment of human subjects in need thereof. A number of RNA viruses, including NDV, reovirus, measles virus, and vesicular stomatitis virus (VSV), are members of this novel class of viruses being exploited as potential oncolytic agents.

NDV is an intrinsically tumor-specific virus, which is under investigation as a clinical oncolytic immunotherapy agent, and whose safety margin is a function of its inability to replicate and kill normal human cells. NDV derives its name from the site of the original outbreak in chickens at a farm near Newcastle-upon-Tyne in England in 1926. It is an economically important pathogen in multiple avian species and it is endemic in many countries. NDV is a member of the Avulavirus genus in the Paramyxoviridae family. Several clinical trials have reported NDV to be a safe and therapeutically useful agent for cancer therapy (Cassel et al., 1965, Cancer 1:863-888; Steiner et al., 2004, J. Clin. Oncol. 22:4272-4281; Schulze et al., 2009, Cancer Immunol. Immunother. 58:61-69; Csatary et al., 1999, Anticancer Res. 19:635-638; Pecora et al., 2002, J. Clin. Oncol. 20:2251-2266; Lorence et al., 2003, Curr. Opin. Mol. Ther. 5:618-624; Freeman et al., 2006, Mol. Ther. 13:221-228); however, there remains a clear need for improvement in therapeutic outcome, in particular to enhancing its killing effect without affecting its safety toward normal cells. NDV is a non-segmented, negative-strand RNA virus of the Paramyxoviridae family, whose natural host range is limited to avian species; however, it is known to enter cells by binding to sialic acid residues present on a wide range of human and rodent cancer cells. NDV has been shown to selectively replicate in and destroy tumor cells, while sparing normal cells, an oncolytic property believed to be based in part on defective antiviral responses in tumor cells. Normal cells, which are competent in launching an efficient antiviral response rapidly after infection, are able to inhibit viral replication before viral-mediated cell damage can be initiated. The sensitivity of NDV to interferon (IFN), coupled with defective IFN signaling pathways in tumor cells, provides one plausible mechanism whereby NDV can replicate exclusively within neoplastic tissue. The selective replication of NDV in tumor cells may also be caused by several other mechanisms, including defects in activation of anti-viral signaling pathways, defects in apoptotic pathways, and activation of Ras signaling and/or expression of Rac1 (Schirrmacher, 2015, Expert Opin. Biol. Ther. 15:17 57-71).

Typically, an oncolytic NDV strain is defined as an NDV for use in oncological immunotherapy, preferably in treatment of human subjects in need thereof. Multiple preclinical model studies have shown significant anti-tumor activity of natural and recombinant oncolytic NDV after varying treatment modalities. Promising results were noted in preclinical models of glioblastoma multiforme, anaplastic astrocytoma, leukemia, lymphoma, melanoma, neuroblastoma, osteosarcoma, rhabdomyosarcoma, Ewing's sarcoma, fibrosarcoma, pheochromocytoma, colon carcinoma, lung carcinoma, prostate carcinoma, breast carcinoma, ovary carcinoma, gastric carcinoma, mesothelioma, renal cell carcinoma, and head & neck carcinoma. One typically useful oncolytic NDV that has been extensively explored for use of treatment in humans, and for which an advantageous safety and efficacy profile has been established in human trials, is the strain named MTH-68/H (Csatary LK, et al., J Neurooncol. 2004 Mar-Apr;67(1-2):83-93). MTH-68/H therapy has been employed in a range of different tumors with success. MTH-68/H has been developed into a highly purified, lyophilized product, containing live, replication competent viral particles, grown to standardized titers. A Phase II clinical trial in humans was completed where the inhalatory mode of administration was used on patients suffering from a variety of advanced malignancies no longer responding to conventional cancer therapies. The study demonstrated relative efficacy, an overall improved quality of life, and a relatively benign side effect profile (Csatary LK, et al., Cancer detection and Prevention, 1993, 17(6):619-627). Success of treatment with MTH-68/H has also been shown in glioblastomas and anaplastic astrocytomas. Glioblastoma multiforme (GBM) is the most common primary brain tumor and by far the most aggressive form of glial tumors. This neoplasm has a poor prognosis, averaging six months to a year without therapy or about one-and-one-half years with conventional therapy. Four cases of advanced GBM and anaplastic astrocytoma were treated with the NDV viral product MTH-68/H after the conventional modalities of cancer therapy had failed. Results included survival rates of twelve and half to six years post-viral treatment for the surviving patients, and six years for one patient who has since died, after having discontinued treatment. Against all odds, these surviving patients returned to good quality of life and to a lifestyle that resemble their pre-morbid daily routines including giving birth to a healthy child and full professional activity. They have been able to enjoy good clinical health. These patients regularly received the MTH-68/H viral therapy for a number of years without interruption as a form of monotherapy once the classical modalities failed. The MRI and CT results have revealed an objective decrease in size of the tumors, in some cases the near total disappearance of the mass.

As reviewed by Zamarin and Palese (Future Microbiol. 2012 Mar; 7(3): 347-367), when compared to other oncolytic agents in development such as poxvirus, HSV-1, adenovirus, measles, and reovirus vectors, NDV as an oncolytic virus exhibits several advantages over other viruses for use in oncological treatment.

First of all, NDV is an avian pathogen, which avoids the problems of preexisting immunity that can neutralize virus infectivity and of pathogenicity of the virus in humans, which are potential problems with vaccinia, HSV-1, adenovirus, and measles vectors. Serologic studies indicated that ∼96% of the human population is seronegative for NDV, which avoids the problem of pre-existing immunity seen with many human candidate oncolytic viruses. The actual level of seropositivity in today's population may be even lower due to low human exposure to NDV, the outbreaks of which are primarily limited to farm settings. To attest for viral safety, administration of virulent strains to humans has been shown to result in only mild to moderate adverse effects, with mild conjunctivitis, laryngitis, and flu-like symptoms as the only reported infections. Natural human infections with highly virulent (avian) strains have been reported in the literature in farmers and laboratory workers and have been limited to mild symptoms such as conjunctivitis and laryngitis.

Secondly, similarly to other oncolytic viruses, NDV possesses strong immunostimulatory properties that are the basis for oncolytic therapy being considered an immunotherapy. These properties include the induction of type I IFN and chemokines, upregulation of MHC and cell adhesion molecules, and facilitation of adhesion of lymphocytes and antigen-presenting cells (APCs) through expression of viral glycoproteins on the surface of infected cells. These properties have been shown to generate effective anti-tumor immune responses, which may persist long after clearance of the primary viral infection.

Third, the NDV genome has the plasticity to enable the incorporation and stable expression of foreign genes of relatively large size. Since these viruses replicate in the cytoplasm of the cell and not the nucleus, unintended integration of the foreign gene into the host genome is avoided, which could be a safety problem with some DNA oncolytic vectors. Moreover, the absence of homologous RNA recombination ensures that foreign genes expressed from the NDV genome are stable for many serial passages in cell culture and in tumor cells.

Lastly, the ubiquitous nature of the NDV receptor allows for utilization of the virus against a wide variety of tumor types. The specificity of NDV for cancer cells due to their defects in antiviral and apoptotic pathways ensures viral safety and may obviate the need for specific tumor targeting.

Nevertheless, the full view of all clinical data generated to date in different trials of different NDV strains shows that there is much need for improvement in terms of percentage responders and magnitude of therapeutic effect. This need for improvement has spurred efforts to design improved NDV strains, which is the subject of the current application.

Similar to other paramyxoviruses in its family, the 15186, 15192 or 15198 nucleotide(nt)-long negative single-strand RNA genome of NDV encodes six genes including the nucleocapsid protein (NP), phosphoprotein (P), matrix protein (M), fusion protein (F), hemagglutinin-neuraminidase (HN), and RNA-dependent RNA polymerase (L) (Lamb R, Paramyxoviridae: the viruses and their replication. In: Knipe D, editor. Fields Virology. Lippincott Williams & Wilkins; 2007. pp. 1449-1496). The genes are separated by junction sequences that consist of three elements, known as gene start (GS), intergenic (IG), and gene-end (GE) motifs, which regulate mRNA transcription. In the P gene, a unique RNA editing mechanism adds non-templated G residues, resulting in the expression of V and W proteins that are colinear to the P protein in the amino-terminal end. The genomic RNA is bound in a ribonucleotide protein complex (RNP) consisting of NP, P, and L proteins and is surrounded by a lipid envelope containing three virus glycoprotein spikes, HN, M and F. An important factor that influences the extent of virulence of NDV in birds is the cleavage site of the F protein.

Pathogenic classification and virulence of NDV strains in birds generally correlates with their oncolytic properties in human cancer cells. Velogenic (high virulence) strains produce severe respiratory and nervous system signs, spread rapidly through chicken flocks, and can cause up to 90% mortality. Mesogenic (intermediate virulence) strains cause coughing, affect egg production and quality, and can result in up to 10% mortality. Lentogenic (low virulence) strains produce only mild symptoms with little if any mortality. Correspondingly, velogenic strains can efficiently carry out multicycle replication in multiple human cancer cells with effective and efficient cell lysis, lentogenic strains are more attenuated due to lack of activation of the F0 protein, and mesogenic strains convey intermediate effects. On the basis of these observations in human cancers, NDV strains have been classified as either lytic or non-lytic, with velogenic and mesogenic viruses being lytic (high and low respectively), and lentogenic viruses in general being non-lytic. Early studies demonstrated that the lytic abilities of lentogenic NDV strains could be enhanced by the introduction of a polybasic cleavage site into their F proteins (Peeters et al, J. Virol. 1999; 73:5001-5009). Other NDV proteins including NP, P, V, HN, and L have also been shown to be implicated in virulence in birds. A deletion (18nt) introduced in the NP gene (Mebatsion T et al., J Virol. 2002 Oct;76(20):10138-46) resulted in the deletion of NP residues 443-460. The resulting mutant NDV propagated in embryonated specific-pathogen-free (SPF) chicken eggs to a level fully comparable to that of the parent virus.
In addition, a B-cell epitope of the S2 glycoprotein of murine hepatitis virus (MHV) was inserted inframe. Recombinant NDV viruses properly expressing the introduced MHV epitope were successfully generated, demonstrating that the NP can be used as the insertion point in the NDV genome to insert foreign or transgenic sequences to be co-expressed with NDV during virus infection.

Several studies showed that type I IFN antagonist activity of NDV V protein is associated with decreased pathogenicity in recombinant viruses possessing attenuating mutations or deletions in the V protein or in viruses expressing no or lower levels of V protein (Huang et al., 2003, J Virol. 77:8676-8685; Mebatsion et al., 2001, J Virol. 75:420-428; Qiu et al., 2016, PLoS ONE 11(2): e0148560. doi: 10.1371/journal.pone.0148560; Elankumaran et al., 2010, J Virol. 84:3835-3844; Jang et al., 2010, Appl Microbiol Biotechnol. 85:1509-1520; Alamares et al., 2010, Virus Res. 147:153-157; Park et al., 2003, J Virol.77:9522-9532.). It was found to be sufficient to introduce a small (6 nt) deletion in the P-gene to abolish expression of the V-protein (Mebatsion et al., 2001). Recently, two studies showed the role of the polymerase complex in viral pathogenesis, with L protein contributing to pathogenicity of the mesogenic Beaudette C strain, and NP, P, and L proteins contributing to pathogenicity of the velogenic Herts strain (Rout & Samal, 2008, J Virol. 82:7828-7836; Dortmans et al., 2010, J Virol. 84:10113-10120). In addition, several studies noted the contribution of the HN protein to virulence in birds (Römer-Oberdörfer et al., 2006, Avian Dis. 50:259-263; Paldurai et al., 2014, J Virol. 88: 8579-8596.) The HN (hemagglutinin-neuraminidase) protein of NDV is a multifunctional protein with receptor-recognition, hemagglutinin (HA) and receptor-destroying neuraminidase (NA) activities associated with the virus. HN is thought to possess both the receptor recognition of sialic acid at the termini of host glycoconjugates and the neuraminidase activity to hydrolyze sialic acid from progeny virion particles in order to prevent viral self-aggregation. It also recognizes sialic acid-containing receptors on cell surfaces and promotes the fusion activity of the F protein, thereby allowing the virus to penetrate the cell surface. Thus, the HN protein plays a critical role in viral infection of birds.
Reverse genetics (rg) systems to provide recombinant NDV vector virus have been known since 1999 (Peeters et al., 1999, J. Virol. 73:5001-5009), allowing such desired modification and engineering of NDV genomes. Indeed, various reports have shown the successful use of recombinant NDV vectors engineered to express various transgenes (foreign genes incorporated in the genome of the virus) with the goal improving viral oncolytic efficacy (Vigil et al. Cancer Res. 2007, 67:8285-8292; Janke et al., 2007, Gene Ther. 14:1639-1649). However, it should be noted that the replication capability of a recombinant NDV expressing a transgene may be hampered in comparison with its parental NDV strain not expressing the transgene. As discussed above, NDV F protein is responsible for viral fusion with the cell membrane and for viral spread from cell to cell via formation of syncytia. The presence of the multi-basic amino acid cleavage site in the F protein enables protein cleavage and activation by a broad range or proteases and is known to be a determinant of virulence in velogenic NDV strains. In order to increase the oncolytic potency of a highly attenuated lentogenic Hitchner B1 NDV strain, a polybasic cleavage site was introduced into the F protein to generate rNDV/F3aa with mutated F protein. Altomonte et al. (Mol Ther. 2010 18:275-84) reported an oncolytic NDV vector virus with the mutated F-protein rNDV/F(L289A), harboring an L289A mutation within the F protein, which is required for virus entry and cell-cell fusion. This rNDV/F3aa(L289A) virus with mutated F protein showed further enhanced fusion and cytotoxicity of hepatocellular carcinoma (HCC) cells in vitro, as compared with a rNDV/F3aa control virus. In vivo administration of rNDV/F3aa(L289A), via hepatic arterial infusion in immune-competent Buffalo rats bearing multifocal, orthotopic liver tumors resulted in tumor-specific syncytia formation and necrosis, with no evidence of toxicity to the neighboring hepatic parenchyma, which translated to a 20% prolongation of survival time relative to treatment with the original rNDV/F3aa virus.

Due to compelling preclinical data implicating oncolytic NDV as an ideal candidate for cancer therapy, phase I and II clinical trials have been initiated. Several trials have shown that NDV is a safe and potentially therapeutically useful therapeutic agent, with no reports of significant adverse effects in patients beyond conjunctivitis or mild flu-like symptoms (Csatary et al., 1999, Anticancer Res. 19:635-638; Pecora et al., 2002, J. Clin. Oncol. 20:2251-2266; Lorence et al., 2003, Curr. Opin. Mol. Ther. 5, 618-624; Freeman et al., 2006, Mol. Ther. 13:221-228). Although the results of these clinical trials have been encouraging, the extent of clinical responses has not been strong and robust enough to consider bringing one of these initial NDV strains into advanced clinical development. Thus, strategies for improving therapeutic effectiveness while maintaining an acceptable safety profile of oncolytic NDV strains are warranted, and the established system for generating modified NDV vectors through reverse-genetics (rg) has provided a unique opportunity to achieve this aim.

### The invention

In a first embodiment, the invention provides an oncolytic NDV for use in oncological treatment (this term where used herein is also replaceable with "for use as a medicament, especially in a method of treatment of cancer"; or the use of said oncolytic NDV in the preparation of a pharmaceutical formulation for use in said method of treatment) in humans (or more generically animals, such as mammalian animals), having a mutation in the HN gene, said mutation providing it with an unexpected beneficially increased replication capability in human cancer cells, preferably allowing replication of said oncolytic NDV in a human cancer cell at least 2-fold higher, preferable to ∼2- to 10-fold higher levels, preferably to 5- to 10-fold higher levels than an NDV parent strain not having said mutation. Preferably, said parent strain comprises or is an oncolytic NDV already having an advantageous safety and efficacy profile in humans, such as strain MTH-68/H. In particular, it is used for the treatment of one or more indications selected from the group of brain tumors, like glioblastoma multiforme, astrocytoma bone tumors, like osteosarcoma and/or Ewing's sarcoma, leukemia, lymphoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose & throat (ENT) tumors, tongue tumors, skin tumors, like melanoma, neuroblastoma, mesothelioma, renal cell carcinoma, fibrosarcoma, pheochromocytoma, head and/or neck carcinoma. Preferably the oncolytic NDV may be used for the treatment of adults and/or children.

In one embodiment, the invention encodes an oncolytic NDV having a leucine (L) at position 277 of the HN gene. It is preferred that said mutation in the NA gene encodes the change of a phenylalanine (F) at position 277 of the protein sequence of the parent HN gene to a leucine L in an oncolytic NDV according to the invention. An oncolytic NDV having such a mutation is herein identified as NDV^{F277L} to discern it from a parent NDV having a phenylalanine at position 277 of the HN gene. Also, the invention provides a method to obtain oncolytic NDV having a beneficially increased replication capability in comparison to its NDV parent strain, comprising serially passaging said parent strain virus in a human cancer cell culture, preferably a HeLa cell culture, and harvesting a progeny oncolytic NDV strain having increased replication capability. Preferably the invention provides a method for selecting said NDV^{F277L}. In a preferred embodiment, the invention provides an oncolytic NDV derived from NDV strain MTH-68/H, said oncolytic NDV according to the invention having an unexpected beneficially increased replication capability allowing replication of said oncolytic NDV in a human cancer cell up to ∼10-fold higher levels than oncolytic NDV parent strain MTH-68/H, thereby maintaining the useful oncological safety and efficacy specifications of the parent virus and advantageously supplementing it with the increased replication capability of an oncolytic NDV as provided by the invention herein. Also, the invention provides a method to obtain oncolytic NDV having a beneficially increased replication capability from NDV parent strain MTH-68/H, comprising serially passaging said MTH-68/H parent virus in a human cancer cell culture such as a HeLa cell culture and selecting a progeny oncolytic NDV strain having increased replication capability in comparison to its NDV parent strain. In a much-preferred embodiment, the invention provides oncolytic strain NDV-MutHu, said strain NDV-MutHu derived from NDV strain MTH-68/H, said oncolytic NDV-Muthu having an unexpected beneficially increased replication capability allowing replication of said oncolytic NDV in a human cancer cell culture, preferably a HeLa cell culture to at ∼10-fold higher levels than oncolytic NDV parent strain MTH-68/H. The invention also provides a nucleic acid comprising the nucleic acid sequence of strain NDV-MutHu, having mutations in the nucleic acids encoding viral proteins HN and M (M^{G165W} and HN^{F277L}) in comparison to strain NDV-MTH-68/H. The skilled artisan knows how to use the disclosed sequence to produce synthetic DNAs (e.g. vectors, in particular in rg-NDV vectors), and use the synthetic DNAs to reconstruct and express the viral genome. Thus, the skilled artisan is enabled to produce virus particles by using the information disclosed herein. In a further embodiment, the invention provides a method (herein also called 'reverse genetics') for providing cloned full-length NDV-MutHu cDNA, and, in a further embodiment, the invention also provides Infectious virus from said full-length NDV-MutHu cDNA, said infectious virus herein for example designated rgMutHu. Typically, NDV-MutHu and rgMutHu as provided herein have similar unexpected beneficial replication capability in human cancer cells. The invention also provides infectious virus (rgNDV) derived from said full-length NDV-MutHu cDNA, wherein either one or both mutations in viral protein M or HN have been restored, said rgNDV herein designated rgMutHu in which the HN and M mutations have been restored. In one embodiment of the invention, the mutation in the M gene has been restored resulting in infectious copy virus [rgMutHu(M^{W165G})]. In another embodiment of the invention, the mutation in the HN gene has been restored resulting in infectious copy virus [rgMutHu(HN^{L277F})]. In yet another embodiment, of the invention, both the mutation in the M gene as well as that in the HN gene have been restored, resulting in infectious copy virus [rgMutHu(M^{W165G}/HN^{L277F})] as herein provided, which is identical to rgMTH68, which is an infectious copy virus of oncolytic strain MTH-68/H.
Unexpected, and surprisingly, it was then established that only the HN mutation is to be held responsible for the improved growth kinetics of NDV-MutHu. NDV-MutHU infectious copy virus, when compared to its parent strain MTH68, grows to surprisingly higher titers than its parent strain. Even though in HeLa cells MTH68 seems to induce CPE earlier than NDV-MutHu and rgMutHu, unexpectedly NDV-MutHu, rgMutHu and restored rgMutHu(M^{W165G}) each reach ∼5- to 10-fold higher virus titers than parent strain MTH-68/H and its infectious copy virus rgMTH68, together with restored infectious copy virus [rgMutHu(HN^{L277F})] from which the HN-mutation was restored to the HN sequence of the parent strain. The invention thus provides a facile reverse genetics system for NDV wherein genetic modification (including the insertion of therapeutic transgenes or useful deletions) of oncolytic NDV with improved replication capability such as strains NDV-MutHu and restored rgMutHu(M^{W165G}) is possible.
Thus, having unexpectedly established the mutation in the HN gene as necessary and sufficient for beneficially improving replication capability, in a further embodiment, the invention provides a NDV strain mutation in the HN gene that confers an unexpected beneficially increased replication capability, said mutation allowing replication of said NDV in a human cancer cell to higher levels than replication of an otherwise identical NDV not having said beneficial mutation in the HN gene. In a further embodiment, the invention also provides an isolated, synthetic or recombinant nucleic acid encoding rgNDV having or provided with a mutation in the HN gene, said mutation allowing replication of said rgNDV in a human cancer cell to higher levels than replication of an otherwise identical rgNDV not having or not been provided with said mutation in the HN gene. Although the functions of the HN protein in NDV infection in birds have been well studied, its roles in viral replication in human cancer cells and in NDV-mediated oncolysis are not known. The inventors have now unexpectedly established that modifying HN beneficially affects replication of rgNDV in cancer cells. Herewith, the inventors identified a novel mutation in the HN protein of NDV with unexpected and promising utility in oncology. Changing the amino acid at position 277 of the HN protein from F to L increases replication in human cancer cells and increases viral yield of the rgNDV grown in such cells; changing L at position 277 back to F reduces replication and yield of rgNDV.
This finding shows that modifying the HN gene in oncolytic rgNDV strains improves replication, and this allows for several advantageous and novel developments. Firstly, the rgNDV virus provided herein grows to higher than expected titers (yields) in cell cultures, eggs or animals that are used to propagate the virus for pharmaceutical purposes, giving production advantages to such improved rgNDV strains. Secondly, higher titers are very useful biologically to obtain the desired oncolytic effects in vivo in that more virus will be produced for subsequent rounds of infection of cancer cells that were missed in the first round of infection. Thirdly, higher levels (yields) of transgenic proteins will be achieved for providing the desired enhanced oncolytic effects in vivo. Thus, by modifying the HN protein within a transgene-expressing NDV, the invention surprisingly provides a transgene-expressing NDV with increased replication capacity in comparison to that of the patent virus and higher yields of the transgenic construct protein, which is very useful in anti-tumor immunotherapy. The invention also provides a novel method for preparing an rgNDV having improved replication in a cancer cell over a parent NDV, said method comprising providing a nucleic acid construct encoding at least a part of a HN gene with a mutation, incorporating said nucleic acid construct with said mutation into a nucleic acid derived from said parent NDV to produce a nucleic acid encoding an rgNDV, using said nucleic acid encoding a rgNDV to produce infectious rgNDV, comparing the replication characteristics in cancer cells of said infectious rgNDV with the replication characteristics of said parent NDV, and selecting said rgNDV for further use when it shows improved replication characteristics over said parent NDV.

The invention also provides an isolated, synthetic or recombinant nucleic acid encoding a rgNDV obtainable by a method for preparing an rgNDV having improved replication in a cancer cell in comparison to that of a parent NDV, said method comprising providing a nucleic acid construct encoding at least a part of a HN gene with a mutation such as a mutation resulting in a change of the amino acid phenylalanine (F) at amino acid position 277 of the hemagglutinin-neuraminidase, incorporating said nucleic acid construct with said mutation into a nucleic acid derived from said parent NDV to produce a nucleic acid encoding an rgNDV, using said nucleic acid encoding a rgNDV to produce infectious rgNDV, comparing the replication characteristics in cancer cells of said infectious rgNDV with the replication characteristics of said parent NDV, selecting said rgNDV for further use when it shows improved replication characteristics over said parent NDV. In a further embodiment, the invention provides a nucleic acid obtainable with such a method as provided herein, said nucleic acid being an isolated, synthetic or recombinant nucleic acid encoding a rgNDV provided with a mutation in the HN gene resulting in a change of the amino acid phenylalanine (F) at amino acid position 277 of the hemagglutinin-neuraminidase into a leucine (L), said mutation (an F277L mutation) allowing replication of said rgNDV in a human cancer cell to a higher level than replication of an otherwise identical rgNDV having the amino acid phenylalanine (F) at position 277 in the HN gene.
The invention also provides an isolated, synthetic or recombinant nucleic acid encoding a rgNDV having or provided with a mutation in the HN gene, said mutation allowing replication of said rgNDV in a human cancer cell to higher levels than replication of an otherwise identical rgNDV not having or not been provided with said mutation in the HN gene, said nucleic acid additionally having been provided with a transgenic construct. In one embodiment, it is preferred that said transgenic construct provides increased lysis of a cancer cell. In a yet further preferred embodiment, said transgenic construct is selected from the group of nucleic acid constructs encoding a protein that reduces or inhibits IFN expression to improve oncolysis, encoding a protein with cytokine activity to improve oncolysis, encoding a protein with antibody activity to improve oncolysis, encoding a protein with apoptotic activity to improve oncolysis, encoding a protein capable of blocking checkpoint inhibition to improve oncolysis, encoding a green fluorescent protein (GFP) to improve visual detection of infected cells, and encoding a protein capable of enhanced binding (targeting) to a cancer cell to improve oncolysis.
In one much preferred embodiment, the invention provides a nucleic acid obtainable with such a method as provided herein, said nucleic acid being an isolated, synthetic or recombinant nucleic acid encoding a rgNDV provided with a mutation in the HN gene resulting in a change of the amino acid phenylalanine (F) at amino acid position 277 of the hemagglutinin-neuraminidase into a leucine (L), said mutation (an F277L mutation) allowing replication of said rgNDV in a human cancer cell to a higher level than replication of an otherwise identical rgNDV having the amino acid phenylalanine (F) at position 277 in the HN gene, said transgenic construct encodes a protein that reduces or inhibits IFN expression such as an IFN-beta receptor, preferably viral protein B18R from vaccinia virus. B18R is a homologue of the human IFN-β receptor. When B18R is secreted from cells infected with an rNDV expresser strain it acts as a decoy for IFN-β, thereby inhibiting activation by cell signaling of the antiviral host response in naive cells, resulting in increased lytic activity in cancer cells.
In one other much preferred embodiment, the invention provides a nucleic acid obtainable with such a method as provided herein, said nucleic acid being an isolated, synthetic or recombinant nucleic acid encoding a rgNDV provided with a mutation in the HN gene resulting in a change of the amino acid phenylalanine (F) at amino acid position 277 of the hemagglutinin-neuraminidase into a leucine (L), said mutation (an F277L mutation) allowing replication of said rgNDV in a human cancer cell to a higher level than replication of an otherwise identical rgNDV having the amino acid phenylalanine (F) at position 277 in the HN gene, said transgenic construct encodes a protein with apoptotic activity such as viral protein 3 from chicken anemia virus, apoptin. Apoptin (VP3 from chicken anemia virus) has been shown to selectively induce apoptosis when expressed by an rNDV expresser strain in human tumor cells, but not in normal human cells, hence an improved therapeutic index for killing cancer cells and not normal cells.
In one other much preferred embodiment, the invention provides a nucleic acid obtainable with such a method as provided herein, said nucleic acid being an isolated, synthetic or recombinant nucleic acid encoding a rgNDV provided with a mutation in the HN gene resulting in a change of the amino acid phenylalanine (F) at amino acid position 277 of the hemagglutinin-neuraminidase into a leucine (L), said mutation (an F277L mutation) allowing replication of said rgNDV in a human cancer cell to a higher level than replication of an otherwise identical rgNDV having the amino acid phenylalanine (F) at position 277 in the HN gene, said transgenic construct encodes an antibody capable of blocking checkpoint inhibition, such as an antibody directed against checkpoint inhibition protein PD-1 (programmed cell death protein 1, also known as CD279). It is preferred that such an antibody is Nivolumab or a functional analog thereof that works as a checkpoint inhibitor, blocking a signal that prevents activated T cells from attacking the cancer upon expression in rNDV-expresser cells, thus allowing the immune system to clear the cancer better than would the parent NDV. Binding of Nivolumab analogue to PD-1 on T-cells results in the elimination of downregulation of T-cell effector responses by cancer cells.
In a further embodiment, the invention provides a nucleic acid, obtainable with such a method as provided herein, said nucleic acid being an isolated, synthetic or recombinant nucleic acid encoding a rgNDV provided with a mutation in the HN gene wherein said transgenic construct encodes a green fluorescent protein, such as enhanced GFP (EGFP) to allow for tracing in-vitro or in-vivo replication, if desired. In another embodiment, the invention provides a nucleic acid, obtainable with such a method as provided herein, said nucleic acid being an isolated, synthetic or recombinant nucleic acid encoding a rgNDV provided with a mutation in the HN gene wherein said transgenic construct encodes a protein capable of providing specific binding (targeting) of rgNDV to a cancer cell, such as a modified HN protein that is fused to a single-chain antibody against a tumor-associated cell surface protein, a modified HN protein that is fused to a ligand that specifically binds to a tumor-associated cell-surface protein, or a bi-specific protein consisting of a single-chain antibody against the rgNDV HN protein fused to a single-chain antibody or ligand that specifically binds to a tumor-associated cell-surface protein thereby increasing the therapeutic window for treatment of cancer with this recombinant expresser strain.
In another embodiment, the invention provides a nucleic acid, obtainable with such a method as provided herein, said nucleic acid being an isolated, synthetic or recombinant nucleic acid encoding a rgNDV provided with a mutation in the HN gene, said nucleic acid additionally having been provided with a mutation in the F gene, said mutation capable of improving oncolytic potential of said rgNDV. In a further preferred embodiment, the invention provides a nucleic acid, obtainable with such a method as provided herein, said nucleic acid being an isolated, synthetic or recombinant nucleic acid encoding a rgNDV provided with a mutation in the HN gene, said nucleic acid additionally having been provided with a mutation in the F gene, said mutation capable of improving oncolytic potential of said rgNDV, wherein said mutation in the F gene comprises or is an L289A mutation.
In yet another embodiment, the invention provides a nucleic acid, obtainable with such a method as provided herein, said nucleic acid being an isolated, synthetic or recombinant nucleic acid encoding a rgNDV provided with a mutation in the HN gene, said nucleic acid additionally having been provided with a mutation in the RNA-editing sequence of the P gene that abolishes or decreases the expression of the V protein.
In yet another embodiment, the invention provides a nucleic acid, obtainable with such a method as provided herein, said nucleic acid being an isolated, synthetic or recombinant nucleic acid encoding a rgNDV provided with a mutation in the HN gene, said nucleic acid additionally having been provided with a mutation in the RNA-editing sequence of the P gene that abolishes or decreases the expression of the V protein.
The invention also provides an rgNDV comprising a nucleic acid according to the invention. Also, the invention provides a method for preparing a rgNDV having improved replication in a cancer cell over a parent NDV, said method comprising the steps: providing a nucleic acid construct encoding a HN gene with a mutation, incorporating said nucleic acid construct with said mutation in a nucleic acid encoding a rgNDV, using said nucleic acid encoding a rgNDV to produce infectious rgNDV, comparing the replication characteristics in cancer cells of said rgNDV with the replication characteristics of said parent NDV, selecting said rgNDV for further use when it shows improved replication characteristics over said parent NDV.
The invention also provides an rgNDV comprising a nucleic acid according to the invention additionally having been provided with a transgenic construct. Also, the invention provides a method for preparing a rgNDV having improved replication in a cancer cell over a parent NDV, said method comprising the steps: providing a nucleic acid construct encoding a HN gene with a mutation, incorporating said nucleic acid constrict with said mutation in a nucleic acid encoding a rgNDV, using said nucleic acid encoding a rgNDV to produce infectious rgNDV, comparing the replication characteristics in cancer cells of said rgNDV with the replication characteristics of said parent NDV, selecting said rgNDV for further use when it shows improved replication characteristics over said parent NDV, and further comprising providing said nucleic acid encoding a rgNDV of step b with a transgenic construct, preferably wherein said transgenic construct provides improved lysis of a cancer cell. In one embodiment, it is preferred that said transgenic construct is selected from the group of nucleic acid constructs encoding a protein that reduces or inhibits IFN expression, encoding a protein with cytokine activity, encoding a protein with antibody activity, encoding a protein with apoptotic activity, encoding a protein capable of blocking checkpoint inhibition, encoding GFP, and encoding a protein capable of enhanced binding (targeting) to a cancer cell. In another embodiment, said nucleic acid encoding a rgNDV of step b is also provided with a mutation in the F gene, said mutation capable of improving oncolytic potential of said rgNDV, in particular wherein said mutation in the F gene comprises or is an L289A mutation. In a further embodiment, said nucleic acid encoding a rgNDV of step b is also provided with a mutation in the RNA-editing sequence of the P gene that abolishes or decreases the expression of the V protein.
The invention also provides a method for preparing a rgNDV having improved replication in a cancer cell over a parent NDV, said method comprising the steps: providing a nucleic acid construct encoding a HN gene with a mutation, incorporating said nucleic acid constrict with said mutation in a nucleic acid encoding a rgNDV or transgene-expressing NDV with increased replication capacity as provided herein, using said nucleic acid encoding a rgNDV to produce infectious rgNDV, comparing the replication characteristics in cancer cells of said rgNDV with the replication characteristics of said parent NDV, selecting said rgNDV for further use when it shows improved replication characteristics over said parent NDV, and further comprising providing said nucleic acid encoding a rgNDV of step b with a transgenic construct, preferably wherein said transgenic construct provides improved lysis of a cancer cell, the method further comprising providing said nucleic acid encoding a rgNDV of step b with a decreased or abolished expression of the V protein. The invention also provides an rgNDV having improved replication in a cancer cell obtainable by a method for preparing a rgNDV having improved replication in a cancer cell over a parent NDV as provided, said method comprising the steps: providing a nucleic acid construct encoding a HN gene with a mutation, incorporating said nucleic acid constrict with said mutation in a nucleic acid encoding a rgNDV, using said nucleic acid encoding a rgNDV to produce infectious rgNDV, comparing the replication characteristics in cancer cells of said rgNDV with the replication characteristics of said parent NDV, selecting said rgNDV for further use when it shows improved replication characteristics over said parent NDV, and further comprising providing said nucleic acid encoding a rgNDV of step b with a transgenic construct, preferably wherein said transgenic construct provides improved lysis of a cancer cell. The invention also provides a pharmaceutical formulation comprising an rgNDV having improved replication in a cancer cell as provided herein without or preferably with at least one pharmaceutically acceptable carrier material.
For the indications mentioned herein, the appropriate dosage will, of course, vary depending upon, for example, the particular molecule of the invention to be employed, the mode of administration and the nature and severity of the condition being treated. In general, the dosage, in one embodiment of the invention, can preferably be in the range of 10⁷ to 10⁹ pfu per dose.
In the above cases of the new NDV strains according to the current invention, improved replication capacity in cancer cells would be associated with increased cancer cell lysis and increased anti-cancer activity relative to activity in normal cells would be associated with an increased therapeutic window or safety margin, as is commonly determined for cancer therapeutics.
In another embodiment, the invention provides a method for producing a pharmaceutical formulation, the method comprising: propagating an rgNDV having improved replication in a cancer cell as provided herein in at least one cell or embryonated egg that is susceptible to a NDV infection; collecting the progeny virus, wherein the virus is grown to sufficient quantities and under sufficient conditions that the virus is free from exogenous contamination, such that the progeny virus is suitable for formulation into a pharmaceutical formulation; and preferably manufacturing a pharmaceutical formulation comprising said progeny virus in the absence of or after addition of at least one pharmaceutically acceptable carrier material.

Pharmaceutical formulations (= pharmaceutical compositions) of the invention may be manufactured in conventional manner. E.g. a composition according to the invention comprising the virus of the invention is provided in lyophilized form and can be complemented with an aqueous carrier for injection when used for administration. For immediate administration it is directly dissolved in a suitable aqueous carrier, for example sterile water for injection or sterile buffered physiological saline.

The invention in a further embodiment also provides a cell or cell line or an embryonated egg comprising a rgNDV having improved replication in a cancer cell as provided herein and in another embodiment a method for treating (= method of treatment of) cancer, comprising administering to a subject in need thereof a rgNDV having improved replication in a cancer cell as provided herein.

In describing nucleic acid formulation, structure and function herein, reference is made to any of a group of polymeric nucleotides such as DNA or RNA. Nucleic acid is composed of either one or two chains of repeating units called nucleotides, which consist of a nitrogen base (a purine or pyrimidine) attached to a sugar phosphate. In the present specification, nucleotide residues are identified by using the following abbreviations. Adenine residue: A; guanine residue: G; thymine residue: T; cytosine residue: C; uracil residue: U. Also, unless explicitly otherwise indicated, the nucleotide sequences of nucleic acid are identified from 5'-terminal to 3'-terminal (left to right terminal), the 5'-terminal being identified as a first residue.

In describing protein or peptide formulation, structure and function herein, reference is made to amino acids. In the present specification, amino acid residues are expressed by using the following abbreviations. Also, unless explicitly otherwise indicated, the amino acid sequences of peptides and proteins are identified from N-terminal to C-terminal (left terminal to right terminal), the N-terminal being identified as a first residue. Amino acids are designated by their 3-letter abbreviation, 1-latter abbreviation, or full name, as follows. Ala : A : alanine; Asp : D : aspartic acid; Glu : E : glutamic acid ; Phe : F : phenylalanine; Gly : G : glycine; His : H : histidine; Ile : I : isoleucine; Lys : K : lysine; Leu : L : leucine; Met : M : methionine; Asn : N : asparagine; Pro : P: proline; Gln : Q : glutamine; Arg : R : arginine; Ser : S : serine; Thr : T : threonine; Val: V : valine; Trp : W : tryptophan; Tyr : Y : tyrosine; Cys : C : cysteine.

In another aspect, presented herein are methods for propagating the NDVs described herein (e.g., rgNDVs) in any cell, subject, tissue or organ susceptible to a NDV infection. In one embodiment, the NDVs described herein may be propagated in a cell line. In another embodiment, the NDVs described herein may be propagated in cancer cells. In another embodiment, the NDVs described herein may be propagated in an embryonated egg. In certain embodiments, presented herein are isolated cells, tissues or organs infected with an NDV described herein. In specific embodiments, presented herein are isolated cancer cells infected with an NDV described herein. In certain embodiments, presented herein are cell lines infected with an NDV described herein. In other embodiments, presented herein are embryonated eggs infected with an NDV described herein.

In another aspect, presented herein are formulations comprising an NDV described herein. In a specific embodiment, presented herein are pharmaceutical formulations comprising an NDV described herein and a pharmaceutically acceptable carrier for injection and/or stabilization of the rgNDV and/or improving its delivery to cancer cells. In particular it may be intended for being used for the treatment of one or more indications selected from the group of brain tumors, like glioblastoma and/or astrocytoma, leukemia, lymphoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose throat tumors, tongue tumors, skin tumors, like melanoma, neuroblastoma, mesothelioma, renal cell carcinoma, fibrosarcoma, pheochromocytoma, head and/or neck carcinoma in adults and/or children. In another embodiment, presented herein are pharmaceutical formulations comprising cancer cells infected with an NDV described herein (e.g., a rgNDV or transgene-expressing NDV with increased replication capacity as described herein), and a pharmaceutically acceptable carrier. In specific embodiments, the cancer cells have been treated with gamma radiation prior to incorporation into the pharmaceutical formulation. In specific embodiments, the cancer cells have been treated with gamma radiation before infection with the NDV (e.g., rgNDV). In other specific embodiments, the cancer cells have been treated with gamma radiation after infection with the NDV (e.g., rgNDV). In another embodiment, presented herein are pharmaceutical formulations comprising protein concentrate from lysed NDV-infected cancer cells (e.g., rgNDV infected cancer cells), and a pharmaceutically acceptable carrier.

In another aspect, presented herein are methods for producing a pharmaceutical formulation comprising an NDV described herein (e.g., a rgNDV or transgene-expressing NDV with increased replication capacity as described herein). In one embodiment, a method for producing a pharmaceutical formulation comprises or consists of: (a) propagating an NDV described herein (e.g., a rgNDV or transgene-expressing NDV with increased replication capacity as described herein) in a cell line that is susceptible to an NDV infection; and (b) collecting the progeny virus and processing the collected virus-containing material to enrich virus and/or eliminate host cell DNA, wherein the virus is grown to sufficient quantities and under sufficient conditions that the virus is free from contamination, such that the progeny virus is suitable for formulation into a pharmaceutical formulation. In another embodiment, a method for producing a pharmaceutical formulation comprises or consists of: (a) propagating an NDV described herein in an embryonated egg; and (b) collecting the progeny virus, wherein the virus is grown to sufficient quantities and under sufficient conditions that the virus is free from contamination, such that the progeny virus is suitable for formulation into a pharmaceutical formulation.

In another aspect, presented herein are methods for treating cancer, especially selected from the specific cancers as described and/or mentioned in the claims herein, utilizing a rgNDV described herein or a pharmaceutical formulation comprising such a rgNDV, especially in a therapeutically effective amount to a patient in need thereof. In a specific embodiment, a method for treating cancer comprises or consists of infecting a cancer cell in a subject with a rgNDV described herein or a formulation thereof. In another embodiment, a method for treating cancer comprises or consists of administering to a subject in need thereof a rgNDV described herein or a pharmaceutical formulation thereof by intravenous, intra-arterial, intratumoral, intramuscular, intradermal, subcutaneous, or any other medically relevant route of administration. In specific embodiments, an effective amount of a rgNDV described herein or a formulation comprising a therapeutically (including preventive) effective amount of a rgNDV described herein is administered to a subject to treat cancer.

In another embodiment, a method for treating cancer, especially selected from the specific cancers as described and/or mentioned in the claims herein, comprises or consists of administering to a subject in need thereof cancer cells infected with a rgNDV or a or transgene-expressing NDV with increased replication capacity as described herein, especially in a therapeutically effective amount, or pharmaceutical formulation thereof by intravenous, intra-arterial, intratumoral, intramuscular, intradermal, subcutaneous, or any other medically relevant route of administration. In specific embodiments, the cancer cells have been treated with gamma radiation prior to administration to the subject or incorporation into the pharmaceutical formulation. In another embodiment, a method for treating cancer comprises or consists of administering to a subject in need thereof protein concentrates or plasma membrane fragments from cancer cells infected with a rgNDV or a pharmaceutical formulation thereof.

In another aspect, presented herein are methods for treating cancer, especially selected from the specific cancers as described and/or mentioned in the claims herein, utilizing an NDV described herein or a pharmaceutical formulation comprising such NDV in combination with one or more other therapies. In one embodiment, presented herein are methods for treating cancer, especially selected from the specific cancers as described and/or mentioned in the claims herein, comprising administering to a subject an rgNDV or a transgene-expressing NDV with increased replication capacity as described herein and one or more other therapies (this term wherever used means therapies and/or therapeutics). In another embodiment, presented herein are methods for treating cancer comprising administering to a subject (especially therapeutically) an effective amount of an rgNDV described herein or a pharmaceutical formulation comprising an effective amount of an rgNDV described herein, and one or more other therapies. The rgNDV and one or more other therapies can be administered concurrently or sequentially to the subject (meaning they are jointly therapeutically active). In certain embodiments, the rgNDV and one or more other therapies are administered in the same ("fixed") pharmaceutical formulation. In other embodiments, the rgNDV and one or more other therapies are administered in different formulations. The rgNDV or transgene-expressing NDV with increased replication capacity as provided herein and one or more other therapies can be administered by the same or different routes of administration to the subject.

### Figure legends

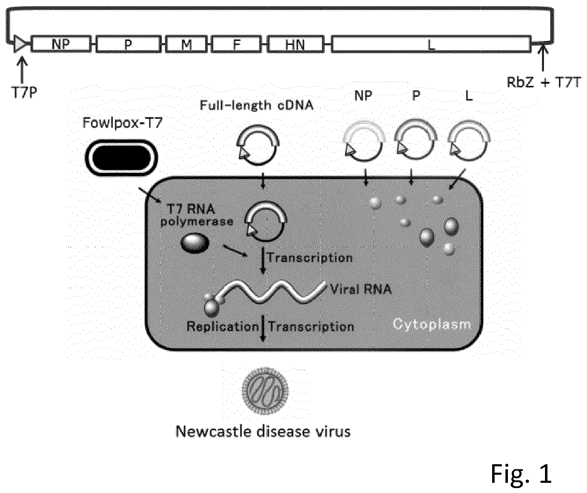

**Fig. 1****.** NDV reverse genetics
   Schematic presentation of the NDV reverse genetics system. The upper part shows the composition of the Full-length cDNA plasmid which contains the full-length NDV cDNA (encoding the NP, P, M, F, HN, and L proteins) cloned behind the bacteriophage T7 RNA Polymerase promoter (T7P; yellow triangle) and followed by a ribozyme sequence (Rz) and T7 transcription termination signal (T7T). A suitable host cell (shaded round-cornered box) is infected with a recombinant Fowlpox virus that expresses T7 DNA-dependent RNA Polymerase (Fowlpox-T7) and subsequently co-transfected with the full-length cDNA plasmid and three helper plasmids containing the genes encoding the NDV NP, P and L proteins, respectively. Transcription of the full-length cDNA results in the generation of the NDV antigenome RNA which is encapsidated by NP protein then transcribed and replicated by the RNA-dependent RNA Polymerase complex consisting of the L and P proteins, ultimately leading to the generation of infectious NDV.

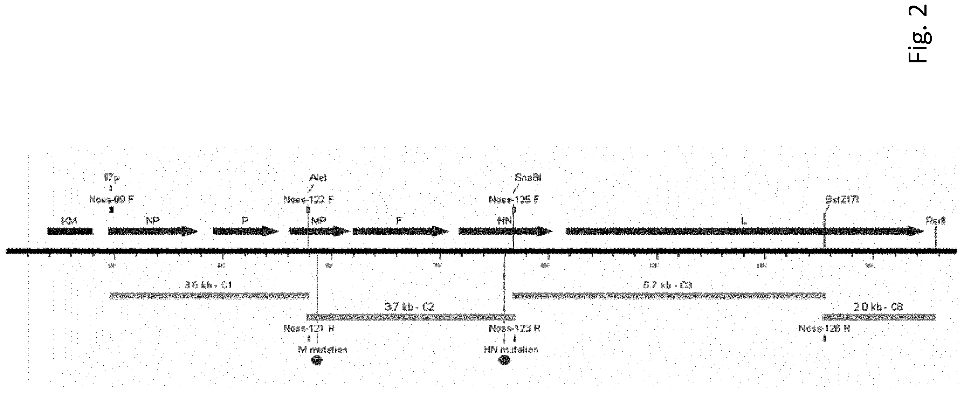

**Fig. 2****.** Assembly of full-length NDV cDNA
   Schematic presentation showing the positions and sizes of the subgenomic cDNA fragments that were used to assemble the complete NDV cDNA in the transcription vector pOLTV5 (Peeters et al., 1999, J. Virol. 73:5001-5009). The dots indicate the positions of the G165W mutations in the M gene and the F277L mutation in the HN gene, respectively.

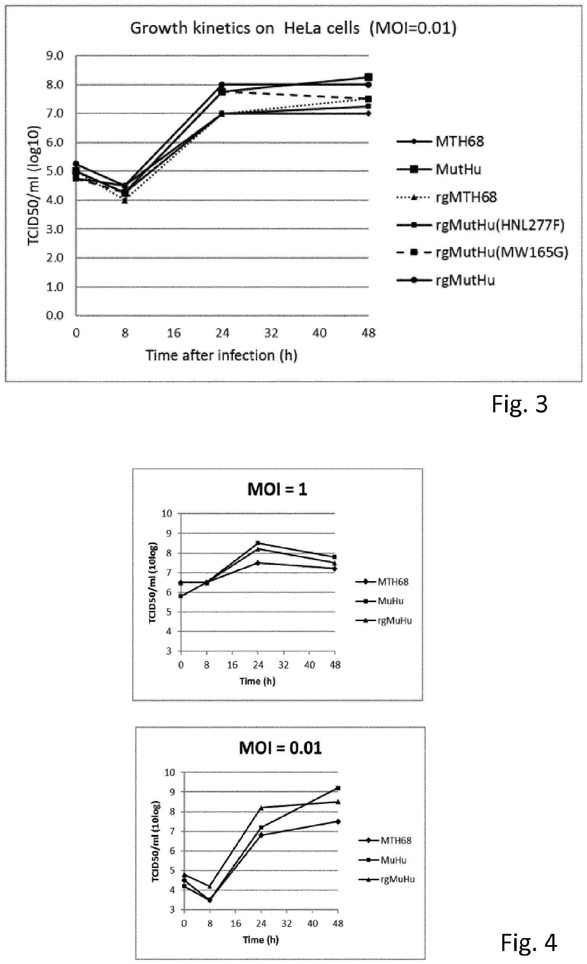

**Fig. 3****.** Growth kinetics in HeLa cells
   HeLa cells were infected at a multiplicity of infection (MOI) of 0.01 with the indicated viruses. At 0h, 8h, 24h and 48h after infection, the amount of infectious virus in the supernatant was determined by end-point titration on QM5 cells. MTH68: NDV strain MTH-68/H; MutHu: NDV strain MutHu; rgMTH68: NDV strain MTH-68/H derived by reverse genetics from cloned full-length cDNA; rgMutHu; NDV strain MutHu derived by reverse genetics from cloned full-length cDNA; rgMutHu(HNL277F); rgMutHu in which the amino acid mutation at position 277 in the HN gene was converted from L back to F; rgMutHu(MW165G): rgMutHu in which the amino acid mutation at position 165 in the M gene was converted from W back to G.
**Fig 4****.** Growth kinetics in HeLa cells
   HeLa cells were infected at a MOI of 0.01 (left panel) or 1.0 (right panel) with the indicated viruses. At 0h, 8h, 24h and 48h after infection, the amount of infectious virus in the supernatant was determined by end-point titration on QM5 cells. MTH68: NDV strain MTH-68/H; MutHu: NDV strain MutHu; rgMutHu; NDV strain MutHu derived by reverse genetics from cloned full-length cDNA.

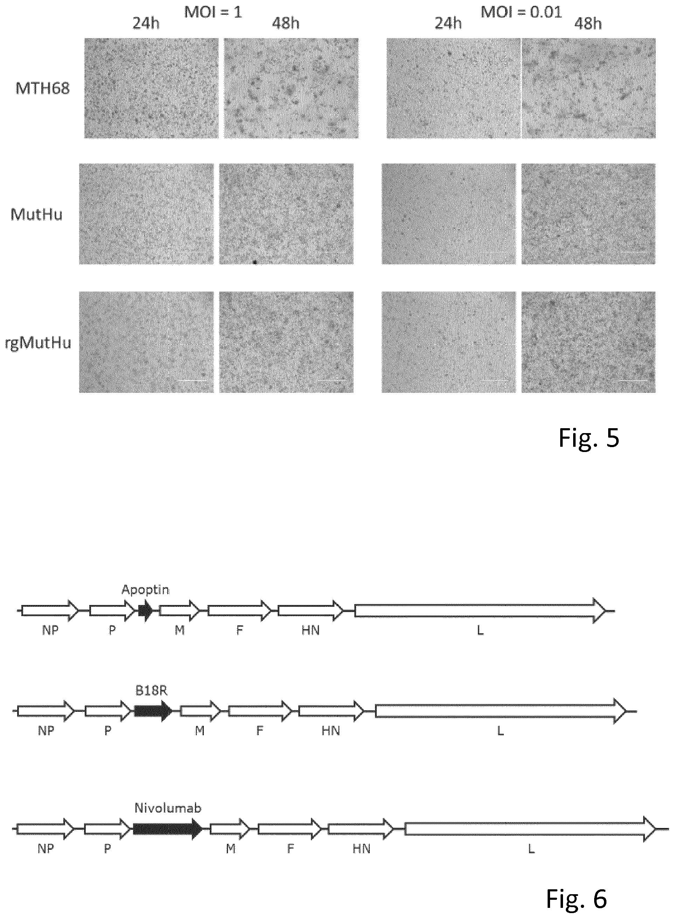

**Fig.5****.** Onset of cytopathogenic effect in NDV-infected HeLa cells
   HeLa cells were infected at a MOI of 0.01 (left) or 1.0 (right) with the indicated viruses. At 24h and 48h after infection photographs were taken using a light microscope in order to examine the extent of the cytopathogenic effect caused by the different viruses. MTH68: NDV strain MTH-68/H; MutHu: NDV strain MutHu rgMutHu, NDV strain MutHu derived by reverse genetics from cloned full-length cDNA.
**Fig. 6****.** Schematic presentation of the genomes of the rgMutHu strains.
   The figure shows the position of the genes encoding Apoptin, B18R and Nivolumab which were inserted as extra transcription units into the NDV rgMutHu genome between the P and M genes.
**Fig. 7****.** Expression of Apoptin by rgMutHu-Apoptin
   Expression of Apoptin was verified by means of immunological staining using monoclonal antibody (MAb) CVI-CAV-111.3 against VP3 of chicken anemia virus (Danen-Van Oorschot, 1997, Proc Natl Acad Sci USA. 94: 5843-5847). Briefly, rgMutHu-Apoptin infected cell monolayers were fixed and incubated with horse-radish peroxidase (HRPO)-conjugated MAb CVI-CAV-111.3 for 1h, and after washing the monolayers, binding of the MAb to Apoptin was detected by HPRO-assay using 3-amino-9-ethylcarbazole (AEC) as a substrate. (A) Non-infected QM5 cells, (B) QM5 cells infected with rgMutHu-Apoptin.

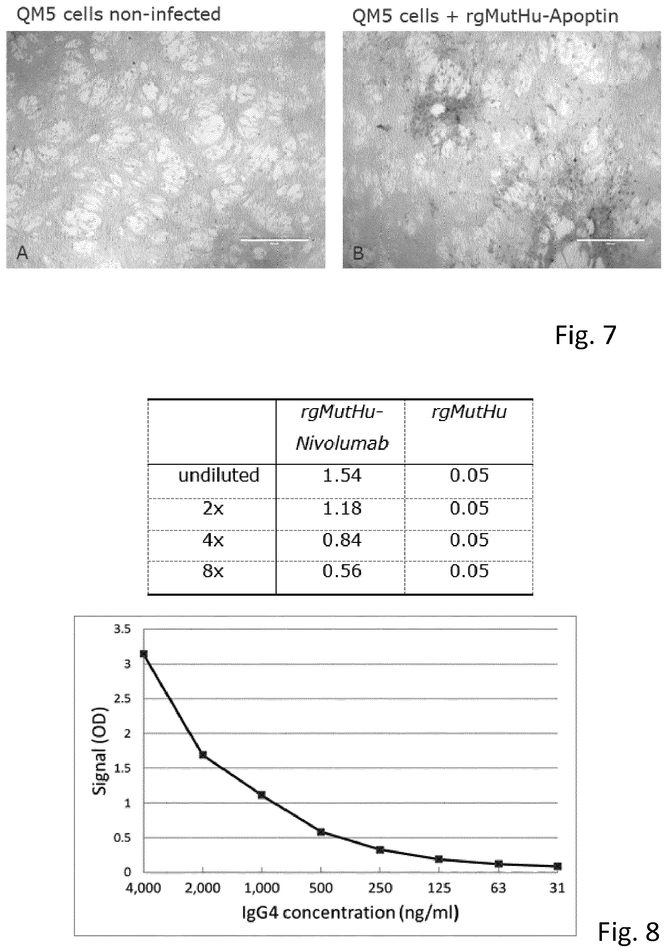

**Fig 8****.** Expression of Nivolumab by rgNDV-Nivolumab
   Undiluted and diluted samples from the supernatant harvested after 48h from rgMutHu-Nivolumab or rgMutHu infected HeLa cells were used to quantify the amount of Nivolumab using a commercial human IgG4 ELISA kit (ThermoFischer Scientific, catalog number: 88-50590-22). The values in the table represent the OD values and the corresponding standard curve is shown in the right panel. The expression level of Nivolumab amounted to approx. 2000 ng/mL.

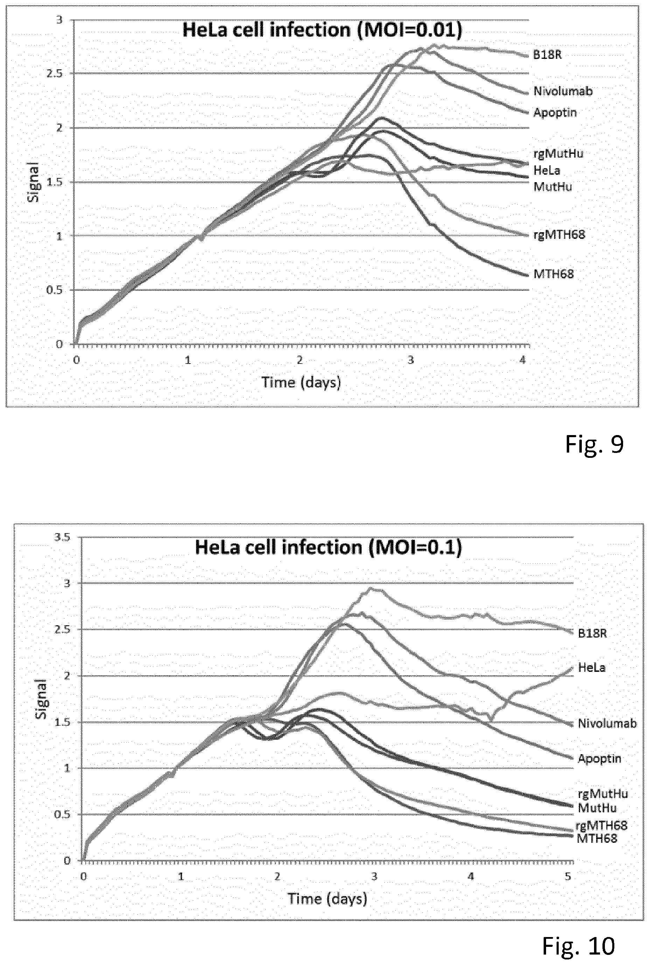

**Fig 9****.** Real-time cell analysis (RTCA) profiles from HeLa cells infected with different NDV strains at an MOI of 0.01
   The growth of uninfected or NDV-infected HeLa cells was examined by using a RTCA device (iCELLigence™; ACEA Biosciences Inc.) which measures differences in cellular impedance. The functional unit of a cellular impedance assay is a set of gold microelectrodes fused to the bottom surface of a cell culture plate well. When submersed in a current-conductive solution (such as buffer or standard tissue culture medium), the application of an electric potential across these electrodes causes electrons to exit the negative terminal, pass through bulk solution, and then deposit onto the positive terminal to complete the circuit. Because this phenomenon is dependent upon the electrodes interacting with bulk solution, the presence of adherent cells at the electrode-solution interface impedes electron flow. The magnitude of this impedance is dependent on the number of cells, the size and shape of the cells, and the cell-substrate attachment quality. Importantly, neither the gold microelectrode surfaces nor the applied electric potential has an effect on cell health or behavior (https://www.aceabio.com/products/icelligence/).
   Cells were seeded in the wells of the analysis plate and 24hr later, cells were infected with the indicated viruses at a MOI of 0.01 and further analyzed for 72h. MTH68: NDV strain MTH-68/H; MutHu: NDV strain MutHu; rgMTH68: NDV strain MTH-68/H derived by reverse genetics from cloned full-length cDNA; rgMutHu; NDV strain MutHu derived by reverse genetics from cloned full-length cDNA; Apoptin: rgMutHu containing the Apoptin gene; Nivolumab; rgMutHu containing the Nivolumab gene; B18R: rgMutHu containing the B18R gene; HeLa: uninfected HeLa cells.
**Fig 10****.** RTCA profiles from HeLa cells infected with different NDV strains at an MOI of 0.01
   The growth of uninfected or NDV-infected HeLa cells was examined by using a RTCA device. Cells were seeded in the wells of the analysis plate and 24hr later the cells were infected with the indicated viruses at a MOI of 0.1 and further analyzed for 72h. MTH68: NDV strain MTH-68/H; MutHu: NDV strain MutHu; rgMTH68: NDV strain MTH-68/H derived by reverse genetics from cloned full-length cDNA; rgMutHu; NDV strain MutHu derived by reverse genetics from cloned full-length cDNA; Apoptin: rgMutHu containing the Apoptin gene; Nivolumab; rgMutHu containing the Nivolumab gene; B18R: rgMutHu containing the B18R gene; HeLa: uninfected HeLa cells.

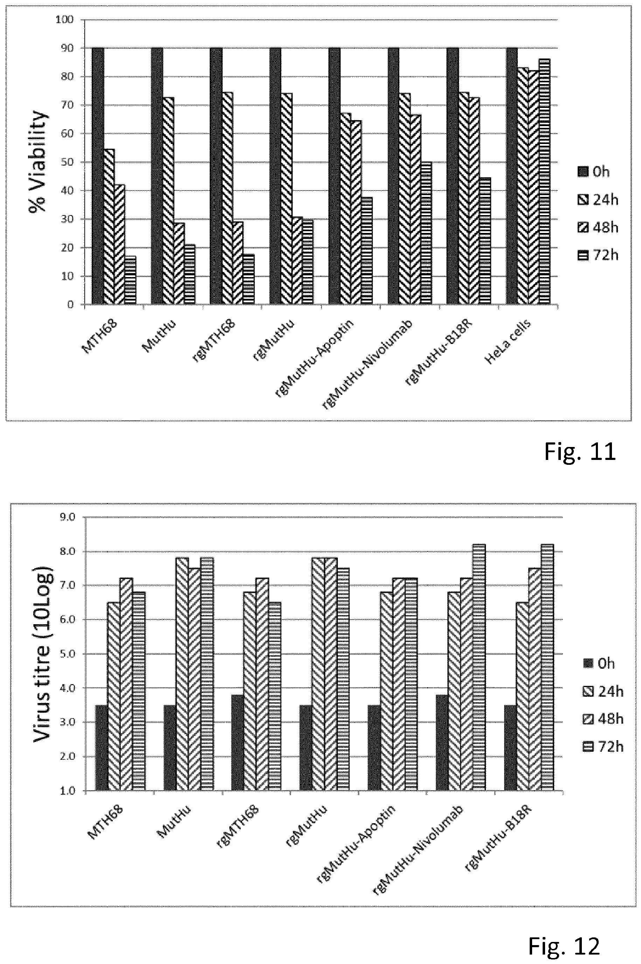

**Fig. 11****.** Cell viability at different time points after infection (MOI = 0.1)
   The percentage of viable cells was examined at different time points after infection of HeLa cells with the indicated viruses at a MOI of 0.1. Cell viability was determined by means of the trypan-exclusion assay using a Countess II FL Automated Cell Counter (ThermoFischer).
**Fig. 12****.** Virus titers at different time points after infection (MOI = 0.1)
   HeLa cells were infected with the indicated viruses at a MOI of 0.1 and infectious virus titers in the supernatant were determined at 0h, 24h, 48h and 72h after infection by end-point titration on QM5 cells. MTH68: NDV strain MTH-68/H; MutHu: NDV strain MutHu; rgMTH68: NDV strain MTH-68/H derived by reverse genetics from cloned full-length cDNA; rgMutHu; NDV strain MutHu derived by reverse genetics from cloned full-length cDNA; rgMutHu-Apoptin: rgMutHu containing the Apoptin gene; rgMutHu-Nivolumab; rgMutHu containing the Nivolumab gene; rgMutHu-B18R: rgMutHu containing the B18R gene.

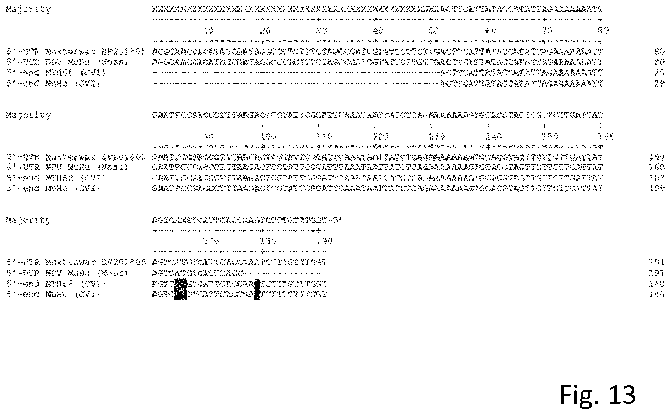

**Fig. 13****.** Appendix 2: **alignment of 5'-terminal sequences**

### Detailed description

### Example 1. Nucleotide sequence analysis of mutant NDV-MutHu.

Newcastle Disease Virus (NDV) is a non-segmented, negative-sense, single-stranded RNA virus belonging to the paramyxovirus family. The natural hosts of NDV are avian species, in particular water- and shorebirds. Some strains of NDV can cause severe and lethal disease in terrestrial poultry such as chickens and turkeys. Infection of humans is rare and is either asymptomatic or limited to a mild and transient conjunctivitis. NDV strains can be categorized into three different groups (pathotypes) based on their pathogenicity for day-old chickens: lentogenic (avirulent), mesogenic (intermediate virulent) and velogenic (virulent). These differences in pathogenicity can largely be explained by differences in the cleavage site in the fusion (F) protein. Lentogenic viruses have a monobasic cleavage site, which can only be cleaved by extra-cellular trypsin-like proteases found in the respiratory and digestive tracts of birds. Mesogenic and velogenic strains have a polybasic cleavage site, which can be cleaved by intra-cellular furin-like proteases, found more abundantly in most cell and organs, explaining why virulent strains can replicate systemically in birds. Several naturally occurring NDV strains have been shown to possess oncolytic anti-tumor properties. In humans, NDV selectively replicates in tumor cells and kills these cells while sparing normal cells. The ratio of killing of cancer cells to normal cells is the therapeutic index for a drug, and the higher it is, the better the efficacy of the drug while minimizing safety-related side effects. The selective replication of NDV in tumor cells is based on several mechanisms, including defects in activation of anti-viral signaling pathways, defects in type-I IFN-signaling pathways, defects in apoptotic pathways, and activation of Ras signaling and expression of Rac1 protein (for recent reviews, see: Schirrmacher, 2015, Expert Opin. Biol. Ther. 15:17 57-71; Zamarin & Palese, 2012, Future Microbiol. 7:347-367).
We identified a spontaneous mutant of an oncolytic NDV strain MTH-68/H (Csatary et al., 1999, Anticancer Res. 19:635-638.; further called MTH68). The replication capacity of the mutant strain (designated NDV-MutHu) in a variety of human neoplastic cell lines, as well as autologous primary tumors, is greatly enhanced as compared to the original MTH68 strain. We analyzed its nucleotide sequence and found that, compared to MTH68, NDV-MutHu has two nucleotide mutations, one leading to an amino acid substitution in the M protein (G165W) and the other in the HN protein (F277L).

### Example 2. A reverse genetics system that allows genetic modification of NDV-MutHu.

### 2.1 Reverse genetics

In order to be able to genetically modify the genome of an RNA virus such as NDV, a manipulatable genetic system must be developed that uses a copy of the full viral RNA (vRNA) genome in the form of DNA. This full-length cDNA is amenable to genetic modification by using recombinant DNA techniques. The authentic or modified cDNA can be converted back into vRNA in cells, which in the presence of the viral replication proteins results in the production of a new modified infectious virus. Such 'reverse genetics systems' have been developed in the last few decades for different classes of RNA viruses. This system enables the rapid and facile introduction of mutations and deletions and the insertion of a transgene transcriptional unit, thereby enabling the changing of the biological properties of the virus.
Reverse genetics systems for several NDV strains, including lentogenic as well as velogenic strains, were developed by the Central Veterinary Institute (CVI), part of Wageningen University and Research, currently Wageningen Bioveterinary Research (WBVR) under the supervision of Dr. Ben Peeters (Peeters et al., 1999, J. Virol. 73:5001-9; de Leeuw et al., 2005, J. Gen. Virol. 86:1759-69; Dortmans et al., 2009, J. Gen. Virol. 90:2746-50). In order to generate a reverse genetics system for NDV-MutHu, a similar approach was used. Details of the procedure can be found in the above cited papers and in the paragraphs below. Briefly, the system consists of 4 components, i.e., a transcription plasmid containing the full-length (either authentic or genetically modified) cDNA of the virus, which is used to generate the vRNA, and 3 expression plasmids ('helper plasmids') containing the NP, P and L genes of NDV respectively, which are used to generate the vRNA-replication complex (consisting of NP, P and L proteins). Transcription of the cDNA (i.e. conversion of the cDNA into vRNA) and expression of the NP, P and L genes by the helper plasmids is driven by a T7 promoter. The corresponding T7 DNA-dependent RNA polymerase (T7-RNAPol) is provided by a helper-virus (Fowlpox-T7).

In order to rescue virus, the 4 plasmids are co-transfected into Fowlpox-T7 infected cells (Fig. 1). Three to five days after transfection, the supernatant is inoculated into specific-pathogen-free embryonated chicken eggs (ECE) and incubated for 3 days. Infectious virus that is produced by transfected cells will replicate in the ECE and progeny virus can be harvested from the allantois fluid.

In order to develop a reverse genetics system for NDV-MutHu the following steps were followed:
- Generation of sub-genomic NDV-MutHu cDNA's by RT-PCR
- Assembly of full-length cDNA in a transcription vector
- Cloning of each of the NP, P and L genes into an expression vector
- Verify nucleotide sequence of full-length cDNA and helper-plasmids
- Repair nucleotide differences resulting from the cloning procedure, if necessary
- Rescue of infectious virus from cDNA using co-transfection (Fig. 1)

### 2.2 Construction of full-length NDV-MutHu cDNA and helper plasmids

NDV-MutHu (passage 28 HeLa cells) was used for the isolation of vRNA using standard procedures. The vRNA was used to generate first-strand cDNA by means of Reverse Transcriptase followed by PCR to generate 4 sub-genomic cDNA fragments (designated C1, C2, C3 and C8). The full-length cDNA of NDV-MutHu was assembled from these fragments and cloned in the transcription vector pOLTV5 (Peeters et al., 1999, J. Virol. 73:5001-5009) by a combination of In-Fusion® cloning and classical cloning using restriction enzymes. An overview of the procedure is shown in Fig. 2, and further details can be found in Appendix 1 of this reference. The resulting plasmid was designated pFL-NDV_MutHu. The NP, P and L-genes of NDV-MutHu were obtained by RT-PCR (Appendix 1) and cloned in the expression plasmid pCVI which was derived by deletion of a Clal restriction fragment from pCl-neo (Promega). The resulting helper plasmids were designated pCVI-NP^{MutHu}, pCVI-P^{MutHu} and pCVI-L^{MutHu}.

### 2.3 Nucleotide sequence analysis

Nucleotide sequence analysis was used to verify that the sequence of pFL-NDV MutHu was correct. A few nucleotides which differed from the Reference sequence were repaired (Table 1). These mutations may represent a minority species in the original virus stock or may be the result of the technical approach such as misincorporation of nucleotides during reverse-transcription and/or PCR-amplification. Two silent mutations (i.e., not leading to an amino acid change) were left unchanged (nt 1318 and 2339; see Table 1).
We noted a deletion of 1 nt in the Reference sequence compared to the rgMutHu sequence at position 11749. Since this deletion would result in a frameshift in the L-gene (and as a consequence a prematurely terminated L protein), we verified the nucleotide sequence of this region in the NDV-MutHu virus stock. The deletion was not present in the virus sequence and thus probably represents a sequencing error in the Reference sequence. A difference was also noted at position 13529 in the L gene sequence (T in Reference sequence; C in rgMutHu). This region was also verified several times using cDNA from 3 independent RT reactions. Each time the nucleotide at this position proved to be a C. Therefore, we assume that also this difference is due to a sequencing error in the Reference sequence. The change from T to C at this position results in an amino acid substitution from Tryptophan (Y) to Cysteine (C)."

**Table 1. Differences in nucleotide sequence between rgMutHu and the MutHu reference sequence**

| **Position** | **Gene** | **MutHu Ref** | **rgMutHu** | **remark** |
|---|---|---|---|---|
| **1318** | NP | G | A | silent |
| **2339** | P | A | G | silent; also, G in pCVI-P^{MutHu} |
| **5123** | F | G | T > G | repaired |
| **8721-8727** | L | | 5xA insert | repaired |
| **10383** | L | C | A > C | repaired |
| **10417** | L | G | A > G | repaired |
| **11749** | L | del 1 nt | A | error in Ref seq |
| **13529** | L | T | C | Y > C; also, C in pCVI-L^{MutHu} |

When comparing the nucleotide sequences of strains MutHu and MTH68 with those of other NDV strains, including strain Mukteswar (a mesogenic strain from which MTH68 is probably derived; Lancaster, 1964, Vet. Bull. 34:57-67), we noted a few differences in the last 20 nucleotides of the 5'-end of the viral genome (Appendix 2). These differences seem to be unique for MTH68/MutHu. Whether these differences are relevant for the oncolytic properties of these viruses is not clear.

### 2.4 Rescue of infectious virus from pFL-NDV MutHu

In order to generate infectious virus, we used the co-transfection system described above (and illustrated in Fig. 1) to transfect QM5 cells (derived from Quail) using plasmid pFL-NDV_MutHu and the helper plasmids pCVI-NP^{MutHu}, pCVI-P^{MutHu} and pCVI-L^{MutHu}. Rescue of infectious virus was successful as demonstrated by the presence of infectious virus in the allantoic fluid of ECE that were inoculated with the transfection supernatant (data not shown). The identity of the rescued virus was determined by RT-PCR followed by sequencing. The rescued virus was designated rgMutHu.

### 2.5 Restoration of amino acid substitutions in the HN and M proteins and rescue of corresponding viruses

The observation that NDV-MutHu replicates in HeLa cells to virus titers that are at least 10-fold higher than those of strain MTH68 suggests that either one or both of the amino acid differences between these two strains is responsible for this phenotype. In order to address this issue, plasmid pFL-NDV_MutHu was used to restore the two amino acid mutations either individually or simultaneously to the MTH68 sequence. Using In-Fusion® or classical restriction-enzyme based cloning procedures, three different plasmids were generated containing the desired alterations. These plasmids were designated pFL-NDV-MutHu(M^{W165G}), pFL-NDV_MutHu(HN^{L277F}) and pFL-NDV_MutHu(M^{W165G}/HN^{L277F}), respectively. Subsequently, virus was rescued from these plasmids using the above described procedure. All three viruses were rescued successfully (Table 3). It should be noted that the DNA sequence of plasmid pFL-NDV_MutHu(M^{W165G}/HN^{L277F}) is identical to that of strain MTH68 and therefore the rescued virus rgMutHu(M^{W165G}/HN^{L277F}) can be regarded as rgMTH68.

**Table 2. rescued viruses**

| **Virus** | **Remark** |
|---|---|
| **rgMutHu** | 2 amino acid substitutions compared to MTH68 |
| **rgMutHu(M^{W165G})** | Amino acid 165 in M restored from W to G |
| **rgMutHu(HN^{L277F})** | Amino acid 277 in HN restored from L to F |
| **rgMutHu(M^{W165G}/HN^{L277F})** = **rgMTH68** | Both amino acids restored to MTH68 sequence |

| | |
|---|---|
| W=Tryptophan; G=Glycine; L=Leucine; F=Phenylalanine | |

### Example 3. Identify whether one or both of the amino acid substitutions in NDV-MutHu are responsible for the difference in growth kinetics between NDV-MutHu and the parent strain MTH68.

### 3.1 Growth kinetics in HeLa cells

The rescued rg-viruses (Table 2) as well as the original MutHu and MTH68 viruses were used to determine their growth-kinetics in HeLa cells. Briefly, 4x10⁶ HeLa cells were seeded in 25cm² cell culture flasks and grown overnight. The cells were infected using a MOI of 0.01 (i.e., 1 infectious virus particle per 100 cells), and at 8, 24 and 48 hours after infection the virus titer in the supernatant was determined by end-point titration on QM5 cells.
The data (Fig. 3) indicate that strains MutHu and rgMutHu yield at least 10-fold higher virus titers than MTH68. Furthermore, the data indicate that the mutation at amino acid position 277 in the HN gene is responsible for this effect. The M mutation does not seem to have an effect. This can be best seen when looking at the virus titers 24h after infection, or even better when comparing the increase in virus titer between 8h and 24h (the exponential growth phase). The virus titer shows an increase of 3.5 (log10) for MutHu, rgMutHu and rgMutHu(M^{W165G}), whereas this is 2.5 for MTH68, 2.7 for rgMutHu(HN^{L277F}) and 3.0 for rgMTH68 (Table 3).

**Table 3. Virus titers (log10 TCID50/ml)**

| Virus | Time after infection (h) | | | |
|---|---|---|---|---|
| | 0 | 8 | 24 | 48 |
| MTH68 | 4.8 | 4.5 | 7.0 | 7.0 |
| MutHu | 5.0 | 4.3 | 7.8 | 8.3 |
| rgMTH68 | 5.0 | 4.0 | 7.0 | 7.5 |
| rgMutHu(HN^{L277F}) | 5.0 | 4.3 | 7.0 | 7.3 |
| rgMutHu(M^{W165G}) | 4.8 | 4.3 | 7.8 | 7.5 |
| rgMutHu | 5.3 | 4.5 | 8.0 | 8.0 |

### 3.2 Time of induction of cytopathogenic effect (CPE)

In an independent earlier experiment, we compared the growth-kinetics of strains MTH68, MutHu, and rgMutHu in HeLa cells using the same conditions as described above, except that also an infection with an MOI=1 was used. Also in this case we noted that strains MutHu and rgMutHu yield at least 10-fold higher virus titers compared to MTH68 (Fig. 4).
During the course of this experiment we noted differences in the onset of cytopathogenic effect (CPE) caused by the different viruses. Notably, MTH68 induced CPE earlier than either MutHu or rgMutHu (Fig. 5).

### 3.3 Conclusions

▪ A reverse genetics system was established for the oncolytic NDV strain NDV-MutHu
▪ Infectious virus, designated rgMutHu, was successfully rescued from cloned full-length NDV-MutHu cDNA
▪ NDV-MutHu and rgMutHu have similar growth kinetics in HeLa cells
▪ The HN²⁷⁷ mutation appears to be responsible for the improved growth kinetics of NDV-MutHu compared to MTH68
▪ rgMutHu in which the HN and M mutations have been restored [rgMutHu(M^{W165G}/HN^{L277F})] is identical to rgMTH68
▪ In HeLa cells MTH68 appears to induce CPE earlier than NDV-MutHu and rgMutHu, but NDV-MutHu and rgMutHu reach ∼10-fold higher virus titers
▪ Genetic modification (including the insertion of therapeutic transgenes) of oncolytic NDV strains NDV-MutHu and MTH68 is now possible

### Example 4. Generation of recombinant NDV-MutHu strains with enhanced oncolytic and immune stimulating properties due to the expression of different therapeutic proteins.

To this end, three different rgMutHu strains were generated, expressing the genes for:
1) Apoptin
2) B18R
3) Nivolumab

Apoptin (VP3 from chicken anemia virus) has been shown to selectively induce apoptosis in human tumor cells, but not in normal human cells.
B18R (from Vaccinia virus) is a homolog of the human IFN-β receptor. The secreted form of B18R acts as a decoy for IFN-β, thereby inhibiting IFN-mediated activation by cell signaling of the antiviral host response in naive cells.
Nivolumab is a human IgG4 anti-PD-1 monoclonal antibody (MAb) that is a checkpoint inhibitor, blocking a signal that prevents activated T cells from attacking the cancer, thus allowing the immune system to clear the cancer. Binding of Nivolumab to PD-1 on T-cells results in the elimination of downregulation of T-cell effector responses by cancer cells.

The properties of the recombinant viruses in comparison to the parent strain rgNDV-MutHu and strain MTH68 were examined by performing growth kinetics experiments and cytotoxicity assays in HeLa cells. In addition, we followed the fate of infected cells by using a RTCA that determines changes in attachment, size and morphology.

### 4.1 Generation of recombinant viruses

Recombinant NDV-MutHu viruses (rgNDV-MutHu) expressing Apoptin, B18R or Nivolumab were generated by means of the previously established reverse genetics system described above. Synthetic genes containing the open reading frames encoding the different proteins were obtained from GenScript Inc. and cloned between the P and M genes of NDV-MutHu (Fig 6). The genes were provided with the necessary NDV gene-start and gene-end sequences in order to allow transcription by the vRNA polymerase.
Nivulomab as a MAb consists of two proteins, i.e., IgG heavy (H) and light (L) chains. In order to allow expression of both proteins from one gene, we generated a fusion-construct that encodes H and L chains as a fusion protein separated by a furin cleavage site and the T2A peptide (Chng et al., 2015, mAbs 7:403-412). Due to a ribosomal stop-start event at the T2A peptide during translation, this results in two separate proteins (H chain and L chain) in equimolar amounts. Furthermore, we used the H7 and L2 signal sequences for the H and L chain, respectively, to allow secretion of the proteins (Haryadi et al., 2015, PLoS ONE 10(2): e0116878. doi: 10.1371/journal.pone.0116878). The secreted H and L chains assemble with each other to form the final IgG molecule.

Infectious virus was rescued for all three constructs, and virus stocks were prepared by two passages in HeLa cells. The nucleotide sequences of the inserted genes in the different recombinant viruses were verified by means of nucleotide sequence analysis and found to be correct.

Faithfull expression of Apoptin by rgMutHu-apoptin was verified by means of immunological staining of rgMutHu-apoptin infected monolayers using a MAb against Apoptin (Fig 7).
Expression of Nivolumab was verified and quantified by means of a commercial human IgG4 ELISA. Expression levels of Nivolumab reached approximately 2 µg/mL (Figure 8).

### 4.2 Cytotoxicity assays and growth kinetics

In order to examine the cytotoxic properties of the recombinant viruses and compare them to those of the parent virus rgMutHu as well as rgMTH68, we performed RTCA using a iCELLigence device which measures differences in cellular impedance. The functional unit of a cellular impedance assay is a set of gold microelectrodes fused to the bottom surface of a cell culture plate well. When submersed in an electrically conductive solution (such as buffer or standard tissue culture medium), the application of an electric potential across these electrodes causes electrons to exit the negative terminal, pass through bulk solution, and then deposit onto the positive terminal to complete the circuit. Because this phenomenon is dependent upon the electrodes interacting with bulk solution, the presence of adherent cells at the electrode-solution interface impedes electron flow. The magnitude of this impedance is dependent on the number of cells, the size and shape of the cells, and the cell-substrate attachment quality. Importantly, neither the gold microelectrode surfaces nor the applied electric potential has an effect on cell health or behavior (https://www.aceabio.com/product/icelligence/).
HeLa cell were grown in the wells of the device and after approx. 24h the cells were infected with the different NDV strains (Table 4). The effect on cell proliferation, morphology change, and attachment was then monitored over a period of 4 or 5 days.

**Table 4. NDV strains used.**

| *Strain* | *Remark* |
|---|---|
| MTH68 | Original MTH68 strain |
| MutHu | Original MutHu strain |
| rgMTH68 | MTH68 generated by reverse genetics |
| rgMutHu | MutHu generated by reverse genetics |
| rgMutHu-Apoptin | rgMutHu containing the Apoptin gene |
| rgMutHu-Nivolumab | rgMutHu containing the Nivolumab gene |
| rgMutHu-B18R | rgMutHu containing the B18R gene |

Figs 9 and 10 show the results of the RTCA using HeLa cells infected with the different NDV strains at 24h after seeding using a MOI of 0.01 and 0.1, respectively. The first noticeable observation is that the signal generated by all (MOI=0.01) or some (MOI=0.1) of the infected cultures increases above the value for non-infected HeLa cells. This increase does not correspond to cell proliferation (cell division) but is due to a change in morphology and size in the virus-infected cell cultures compared to non-infected cultures. This effect is reversed later in the infection cycle when cells start to die due to the cytotoxic effect of the viral infection. Thus, the rise and fall of the signal after virus infection is a combined effect of morphological changes and cell killing. The infection of HeLa cells induces cellular defense mechanisms ("danger signals") resulting in the release of cytokines that stimulate the proliferation and/or morphology change of infected as well as non-infected cells. This seems to be in agreement with the observation that the effect is larger and longer-lasting at MOI of 0.01.

The difference in curves for the different viruses (best seen in Fig 10; MOI=0.1) shows that the MTH68 strains are more cytotoxic than the MutHu strains. The cytotoxicity of the rgMutHu strains that carry an extra gene (Apoptin, B18R or Nivolumab) seems to be even further delayed.

We next studied the correlation between the RTCA results on the one hand and cell-killing & virus replication on the other. To this end, we determined the cell viability (percentage of living cells) and the virus titer at different time points after infection (MOI=0.1). Cell viability was determined by using the trypan-blue dye-exclusion assay, whereas infectious virus titers in the supernatant were determined by end point dilution on QM5 cells.

Fig 11 shows that MTH68 and MutHu strains are more cytotoxic than the rgMutHu strains that carry an extra gene (Apoptin, Nivolumab or B18R). This appears to be in agreement with the RTCA results although the cytotoxicity seems to be underestimated in the RTCA compared to the viability assay, apparently due the above noted effect of virus infection on the morphology and size of the cells.

Analysis of the virus replication kinetics (Fig 12) showed that final virus titers for MTH68 and rgMTH68 were approx. one log lower than those of MutHu and rgMutHu, confirming previous observations. Notably, strains rgMutHu-B18R and rgMutHu-Nivolumab reached virus titers at 72h post infection that even exceeded those of the parent strain rgMutHu. This was not the case for rgMutHu-Apoptin. At 24h post infection, however, the virus titers for rgMutHu-B18R and rgMutHu-Nivolumab (as well as rgNutHu-Apoptin) were significantly lower than those of rgMutHu, showing that replication was delayed, probably as a result of the larger genome size.
The insert-size of rgMutHu-Nivolumab is twice as large as that of rgMutHu-B18R and more than 4 times as large as that of rgMutHu-Apoptin. This difference in size seems to correlate with the cytotoxicity values at 72h post infection. However, the RTCA signal for the rgMutHu-B18R is much lower compared to that of the other two strains (which are almost similar). This shows that other effects than the genome size are responsible for the observed difference in RTCA signal. These effects are most probably related to the biological functions of the therapeutic proteins.

In this example 4 we have generated three recombinant NDV-MutHu strains, each expressing a different therapeutic protein, i.e., Apoptin, B18R and Nivolumab. These proteins were chosen because expression of these proteins is expected to result in an enhancement of the oncolytic and/or immune-stimulating properties of NDV-MutHu. Our cytotoxicity assays suggest that the insertion of an extra gene results in a delay in the onset of cell killing. However, this does not necessarily result in lower virus titers. In fact, rgMutHu-B18R and rgMutHu-Nivolumab reached final virus titers which were higher than those of the parent strain rgMutHu (Fig 12). It seems, therefore, that there is a balance between the onset and duration of cell-killing and the amount of progeny virus that is produced. In this respect, it is noteworthy that rgMutHu-Apoptin did not reach the same virus titers as the other two recombinant viruses. This suggests that expression of Apoptin by rgMutHu-Apoptin results in earlier cell-killing as would be expected from a pro-apoptotic protein. This seems to be confirmed by the cytotoxicity assays (Fig 11).
Our data show that the results of the RTCA should be interpreted with some caution since the signal generated by the iCELLigence device underestimates the actual cytotoxic capacity of the recombinant MutHu strains. This is primarily caused by the effect on the RTCA signal of changes in cell morphology and cell size after infection. Thus, the RTCA signal shows a combined effect of morphological changes and cell killing.
The observation that the recombinant rgMutHu strains have a slower in vitro cytotoxicity (direct cell killing effect) in HeLa cells compared to the parent strain does not necessarily mean that they are inferior as oncolytic viruses for tumor treatment. It has been shown that the anti-cancer effects of replication-competent oncolytic viruses such as NDV are for a large part due to their immune-stimulating properties. Indeed, it has been shown that a non-pathogenic NDV is a potent inducer of type-I IFN and Dendritic Cell maturation, and that intra-tumoral injection of NDV results in distant tumor immune infiltration in the absence of distant virus spread (Zamarin et al., 2014, Sci. Transl. Med. 6(226 Whereas the presence of a transgene seems to have a negative effect on the rate of virus replication and cytotoxicity when compared to the parent strain, the ultimate virus titers of the transgene-expressing rgMutHu strains are comparable or even higher than those of the parent strain (Fig. 12). A slower replication rate in association with the production of a therapeutic protein will benefit the induction of immunological anti-tumor responses perhaps at a modest cost of cytotoxicity. However, compared to rgNDV strains that do not carry the F277L mutation, the intrinsic higher replication rate of rgMutHu strain will compensate for the loss of in vivo replication experienced by rgNDV strains. Therefore, the F277L mutation will enhance both the immune-stimulating as well as the cytotoxic effect.
An in-vitro effect was anticipated for rgMutHu-B18R since B18R is able to capture IFN-β thereby interfering with IFN-signaling. However, published data suggest that IFN-β expression by HeLa cells is poorly inducible, and that HeLa cells do not produce IFN-β after infection with NDV (Enoch et al., 1986, PLoS ONE 10(2): e0116878 Blach-Olszewska et al., 1977, Arch Immunol Ther Exp (Warsz) 25:683-91). This may explain why we did not observe a clear effect of B18R in rgMutHu-B18R infected HeLa cells. A biological effect is expected for rgMutHu-Nivolumab only in the context of in vivo application since the antibody exerts its function by binding to T-cells expressing PD-1.

### Example 5: A method for preparing an rgNDV having improved replication in a cancer cell over a parent NDV and the resulting rgNDV.

The observation that the F to L substitution at amino acid position 277 in the HN protein of NDV results in improved replication in cancer cells indicates that the HN protein fulfills an as-yet not understood but important function in determining the extent of virus replication in these cells. The substitution of F at position 277 by an amino acid other than L might increase replication to even higher levels. Furthermore, amino acid substitutions at positions other than 277 in the HN protein may have similar or improved effects on the level of virus replication in cancer cells. Also, the combination of two or more amino acid substitutions at different positions in HN, or the deletion of one or more amino acids at specific positions in HN, may result in increased replication levels of rgNDV in cancer cells. Therefore, a systematic survey of the effect of amino acid substitutions and/or deletions in HN of rgNDV on replication in cancer cells is provided herein.
An rgNDV having improved replication in cancer cells over its parent NDV is obtained by generating rgNDV containing a genetically modified HN gene. Having identified L277 as an important mutation that affects virus replication, it is now provided by such techniques to perform studies to evaluate the effect of substitutions with other amino acids at this position or at other positions in the neighborhood of 277 or at positions elsewhere in HN. Additional studies may also focus on the identification of potential differences in the cellular interaction partners of wt and mutant HN. Using methods known in the art, specific mutations (i.e., substitution of a specific nucleotide by a different nucleotide) or deletions are introduced in a nucleic acid construct containing (part of) the nucleotide sequence encoding the HN protein of NDV. Based on the position(s) and type(s) of the nucleotide substitution(s) or deletions this will result in (a) specific amino acid substitution(s) or deletions in the HN protein. The genetically modified nucleic acid is used to replace the corresponding nucleotides in the full-length NDV genome, which can subsequently be used to generate infectious virus. This collection of mutant rgNDV can be used to examine replication in cancer cells, thus allowing the identification rgNDV with improved replication properties over the parent NDV.

### References.

Blach-Olszewska et al., (1977) Why HeLa cells do not produce interferon? Arch Immunol Ther Exp (Warsz) 25:683-91.
Chng et al., (2015) Cleavage efficient 2A peptides for high level monoclonal antibody expression in CHO cells, mAbs, 7:2, 403-412; http://dx.doi.org/10.1080/19420862.2015.1008351.
Enoch et al., (1986) Activation of the Human beta-Interferon Gene Requires an Interferon-Inducible Factor, Mol. Cell. Biol. 6:801-10.
Haryadi et al., (2015) Optimization of Heavy Chain and Light Chain Signal Peptides for High Level Expression of Therapeutic Antibodies in CHO Cells. PLoS ONE 10(2): e0116878. doi:10.1371/journal.pone.0116878.
Schirrmacher, (2015) Oncolytic Newcastle disease virus as a prospective anti-cancer therapy. A biologic agent with potential to break therapy resistance. Expert Opin. Biol. Ther. 15:17 57-71
Zamarin et al., (2014) Localized oncolytic virotherapy overcomes systemic tumor resistance to immune checkpoint blockade immunotherapy. Sci. Transl. Med. 6(226).
Zamarin & Palese, (2017) Oncolytic Newcastle Disease Virus for cancer therapy: old challenges and new directions. Future Microbiol. 7: 347-67.

**Appendix 1: primers used for the generation of cDNA fragments and helper-plasmids cDNA fragments**

| Fragment | Size | Primer | Sequence (5'-3') |
|---|---|---|---|
| C1 | 3.6 kb | Noss-9F (SEQ ID No.5) | ACGACTCACTATAGGACCAAACAGAGAATCCGTGAG |
| | | Noss-121R (SEQ ID No.6) | CCGGGAAGATCCAGGGCACTCTTCTTGCATGTTAC |
| C2 | 3.7 kb | Noss-122F (SEQ ID No.7) | GGGCCTGCCTCACTATGGTGGTAACATGCAAGAAG |
| | | Noss-123R (SEQ ID No.8) | TGCATGTTACCACCAATGTGTCATTGTATCGCTTG |
| C3 | 5.7 kb | Noss-125F (SEQ ID No.9) | CAAGAAGGGAGATACGTAATATACAAGCGATACAATG |
| | | Noss-126R (SEQ ID No.10) | TCGCTTGTATATTACTTGTTGTAGCAAAGAGCACC |
| C8 | 2.0 | Noss-133 (SEQ ID No.11) | GGCCTGGATCTTCCCATTATGCTGTCTGTATACGGTGC |
| | | Noss-10R (SEQ ID No.12) | ATGCCATGCCGACCCACCAAACAAAGACTTGGTGAATG |
| RT-primer (C1, C2) | | RT-21 (SEQ ID No.13) | ACCAAACAGAGAATCCGTG |
| RT-primer (C3, C8) | | RT-45 (SEQ ID No.14) | AGTCTTCAGTCATGGACAGC |

**Helper-plasmids (generated by In-Fusion® cloning in pCVI)**

| Gene | primer | sequence |
|---|---|---|
| NP | Noss-22F (SEQ ID No.15) | CTCTAGAGTCGACCCTTCTGCCAACATGTCTTCCG |
| | Noss-23R (SEQ ID No.16) | GGGAAGCGGCCGCCCGTCGGTCAGTATCCCCAGTC |
| P | Noss-24F (SEQ ID No.17) | CTCTAGAGTCGACCCCAGAGTGAAGATGGCCACCTTC |
| | Noss-25R(SEQ ID No.18) | GGGAAGCGGCCGCCCAGTGATCAGCCATTCAGCGC |
| L | Noss-26F (SEQ ID No.19) | CTCTAGAGTCGACCCGGGTAGGACATGGCGGGCTC |
| | Noss-27R (SEQ ID No.20) | GGGAAGCGGCCGCCCTGCCTTTAAGAGTCACAGTTAC |

### Sequences:

- SEQ ID No. 1:: Nucleic acid (cDNA) sequence of Newcastle disease virus strain (NDV) MutHu genome;
- SEQ ID No. 2:: Nucleic acid (cDNA) sequence of Newcastle disease virus strain rgMutHu-Apoptin genome created by reverse genetics as disclosed herein, wherein the nucleic acid additionally encodes the pro-apoptotic protein apoptin;
- SEQ ID No. 3:: Nucleic acid (cDNA) sequence of Newcastle disease virus strain rgMutHu-B18 genome created by reverse genetics as disclosed herein, wherein the nucleic acid additionally encodes a homolog of the human IFN-β receptor;
- SEQ ID No. 4:: Nucleic acid (cDNA) sequence of Newcastle disease virus strain rgMutHu-Nivolumab genome created by reverse genetics as disclosed herein, wherein the nucleic acid additionally encodes human IgG4 anti-PD-1 monoclonal antibody;

## Claims

1. A Newcastle Disease Virus (NDV) for use in oncological treatment of a subject considered in need thereof, in particular for the treatment of one or more indications selected from the group of brain tumors, like glioblastoma and/or astrocytoma, leukemia, lymphoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose & throat (ENT) tumors, tongue tumors, skin tumors, like melanoma, neuroblastoma, mesothelioma, renal cell carcinoma, fibrosarcoma, pheochromocytoma, head and/or neck carcinoma, wherein said NDV having a mutation in the HN gene providing it with an increased replication capability in a human cancer cell, preferably allowing replication of said oncolytic NDV in a human cancer cell in at least 2-fold higher levels than the parent strain of said NDV not having said mutation, particularly which is encoded by and/or which comprises at least one of the nucleic acids according to SEQ ID No. 1 to 4 or parts thereof, preferably having a sequence identity of at least 75 %, particularly at least 90%, more particularly 95 %.

2. An NDV according to claim 1, wherein said parent strain comprises or is strain MTH-68/H and/or wherein the mutation in the HN gene leads to a change in amino acid sequence of hemagglutinin-neuraminidase, preferably wherein an amino acid in position 277, particularly phenylalanine (F), is substituted, more preferably wherein the amino acid in position 277 is substituted to an amino acid with a hydrophobic side chain except phenylalanine, more particularly wherein the amino acid in position 277 is substituted to leucine (L) at position 277 of the HN gene.

3. An NDV according to any one of the preceding claims, wherein said subject is a mammal, particularly a mammalian animal or a human, subject.

4. A nucleic acid encoding a NDV, particularly a rgNDV, having a mutation in the HN gene, said mutation allowing replication of said NDV in a cancer cell to a higher level than replication of an otherwise identical NDV not having said mutation in the HN gene, preferably wherein the nucleic acid comprises or consists of at least one of SEQ ID No. 1 to No. 4 or parts thereof, preferably having a sequence identity of at least 75%, particularly at least 90%, more particularly 95 %, preferably wherein the nucleic acid is derived from a NDV according to any one of the preceding claims.

5. A nucleic acid according to claim 4 wherein said mutated HN gene encodes hemagglutinin-neuraminidase with an amino acid substitution at position 277 to an amino acid with a hydrophobic side chain other than phenylalanine, particularly a leucine (L), more particularly wherein the mutated HN gene encodes HN^{F277L}.

6. A nucleic acid according to any one of the preceding claims, additionally comprising a transgenic construct, wherein said transgenic construct provides for improved lysis of a cancer cell and/or increased replication capability in a human cancer cell, particularly improved lysis of a neoplastic tissue cell, preferably wherein said transgenic construct is selected from the group of nucleic acid constructs encoding one protein or a combination of two or more proteins and/or at least a part thereof selected from the group consisting of a protein that reduces or inhibits interferon expression, a protein with cytokine activity, a protein with antibody activity, a protein with apoptotic activity, a protein capable of blocking checkpoint inhibition, a green fluorescent protein (GFP), and/or a protein capable of enhanced binding (targeting) to a cancer cell.

7. A nucleic acid according to any one of the preceding claims, comprising a or said transgenic construct, wherein the transgenic construct encodes a protein and/or a part thereof that reduces and/or inhibits interferon expression, such as an IFN-beta receptor, preferably viral protein B18R from vaccinia virus, particularly wherein the nucleic acid comprises or consists of the SEQ ID No. 3 or parts thereof, preferably having a sequence identity of at least 75 %, particularly at least 90%, more particularly 95 %.

8. A nucleic acid according to any one of the preceding claims, comprising a or said transgenic construct, wherein the transgenic construct encodes a protein and/or a part thereof with apoptotic activity, such as viral protein-3 from chicken anemia virus, apoptin, particularly wherein the nucleic acid comprises or consists of the SEQ ID No. 2 or parts thereof, preferably having a sequence identity of at least 75 %, particularly at least 90%, more particularly 95 %.

9. A nucleic acid according to any one of the preceding claims, comprising a or said transgenic construct, wherein the transgenic construct encodes an antibody and/or a part thereof capable of blocking checkpoint inhibition, such as an antibody directed against checkpoint inhibition protein PD-1, particularly wherein the nucleic acid comprises or consists of the SEQ ID No. 4 or parts thereof, preferably having a sequence identity of at least 75 %, particularly at least 90%, more particularly 95 %.

10. A nucleic acid according to any one of the preceding claims, comprising a or said transgenic construct, wherein the transgenic construct encodes a protein and/or a part thereof capable of providing enhanced binding (targeting) of NDV to a cancer cell, particularly like one protein or a combination of two or more proteins and/or at least a part thereof selected from the group consisting of a modified HN protein that is fused to a single-chain antibody against a tumor-associated cell surface protein, a modified HN protein that is fused to a ligand that specifically binds to a tumor-associated cell-surface protein, and/or a bi-specific protein consisting of a single-chain antibody against the rgNDV HN protein fused to a single-chain antibody and/or a ligand that specifically binds to a tumor-associated cell-surface protein.

11. A nucleic acid, encoding a NDV, particularly a rgNDV, more particularly the nucleic acid according to anyone of claims 4 to 10, and/or having a mutation in the F gene, wherein said mutation is capable of improving oncolytic potential of said NDV, preferably wherein said mutated F gene encodes a fusion protein with an amino acid substitution at position 289 to an amino acid with a hydrophobic side chain other than leucine, particularly an alanine, more particularly wherein the mutated F gene encodes F^{L289A}.

12. A nucleic acid according to any one of claims 4 to 11 additionally having a mutation in the RNA-editing sequence of the P gene that abolishes and/or decreases the expression of the V protein and/or additionally having a deletion in the NP-gene and/or in the V-gene and/or in the HN-gene.

13. A method for preparing a rgNDV, having improved replication in a cancer cell over a parent NDV, said method comprising the steps:
a. providing a nucleic acid construct encoding a HN gene with a mutation, particularly wherein the mutation in the HN gene leads to a change in the expression of hemagglutinin-neuraminidase, wherein the amino acid, particularly phenylalanine (F), in position 277 is substituted, particularly wherein the amino acid in position 277 is substituted to an amino acid with a hydrophobic side chain, more particularly wherein the amino acid in position 277 is substituted to having a leucine (L) at position 277 of the HN gene,
b. incorporating said nucleic acid construct with said mutation in a nucleic acid encoding a rgNDV, particularly to achieve a nucleic acid according to anyone of claims 4 to 12,
c. using said nucleic acid encoding a rgNDV to produce infectious rgNDV,
d. comparing the replication characteristics in cancer cells of said rgNDV with the replication characteristics of said parent NDV,
e. selecting said rgNDV for further use when is shows improved replication characteristics over said parent NDV.

14. A method according to claim 13, wherein the nucleic acid encoding a rgNDV of step b further comprises a transgenic construct, preferably wherein said transgenic construct encodes a protein which leads to an improved lysis of a cancer cell, particularly improved lysis of a neoplastic tissue cell, more preferably wherein said transgenic construct is one or a combination of two or more selected from the group of nucleic acid constructs encoding a protein that reduces or inhibits interferon expression, encoding a protein with cytokine activity, encoding a protein with antibody activity, encoding a protein with apoptotic activity, encoding a protein capable of blocking checkpoint inhibition, encoding a green fluorescent protein (GFP), and/or encoding a protein capable of enhanced binding (targeting) to a cancer cell.

15. A method according to any one of claims 13 to 14, wherein the nucleic acid encoding a rgNDV of step b carries a mutation in the F gene, particularly wherein said mutation is capable of improving oncolytic potential of said rgNDV, preferably wherein said mutation in the F gene comprises or is a mutation which encodes a fusion protein having an amino acid substitution in position 289, particularly a L289A substitution.

16. A method according to any one of the preceding claims, wherein the nucleic acid encoding a rgNDV of step b further carries a mutation in the RNA-editing sequence of the P gene, particularly that abolishes and/or decreases the expression of the V protein.

17. A pharmaceutical formulation comprising particles of NDV according to any one of the claims 1 to 3 and/or a nucleic acid according to any one of the preceding claims, preferably for use in oncological treatment of a subject considered in need thereof, in particular for the treatment of one or more indications selected from the group of brain tumors, like glioblastoma and/or astrocytoma, leukemia, lymphoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose & throat (ENT) tumors, tongue tumors, skin tumors, like melanoma, neuroblastoma, mesothelioma, renal cell carcinoma, fibrosarcoma, pheochromocytoma, head and/or neck carcinoma.
